# EUROPEAN PATENT APPLICATION

(11) **EP 4 643 879 A1**
(43) Date of publication of application: **05.11.2025**
(21) Application number: 23912213.8
(22) Date of filing: 26.12.2023
(51) Int. Cl.: A61K 45/00, A61K 8/44, A61K 31/198, A61K 31/405, A61K 31/4172, A61P 17/00, A61P 17/16, A61P 43/00, A61Q 19/00

(54) **COMPOSITION FOR IMPROVING SKIN CONDITIONS**

(30) Priority: 26.12.2022 JP 2022209098
(71) Applicant: AJINOMOTO CO., INC., Chuo-ku Tokyo 104-8315 (JP)
(72) Inventor: TAKINO, Yoshinobu, Kawasaki-shi, Kanagawa 210-8681 (JP); OKURA, Fumie, Kawasaki-shi, Kanagawa 210-8681 (JP); IKEGAMI, Eri, Kawasaki-shi, Kanagawa 210-8681 (JP); SHIRASAWA, Ayaka, Kawasaki-shi, Kanagawa 210-8681 (JP); SHIMBO, Kazutaka, Kawasaki-shi, Kanagawa 210-8681 (JP)
(74) Representative: Mewburn Ellis LLP
(86) International application number: PCT/JP2023/046829
(87) International publication number: WO 2024/143435

(57) **Abstract**

The present invention provides a composition for improving skin conditions of decreased moisture content of the stratum corneum, spots, decreased barrier function, and abnormal pH value.

A composition for improving skin conditions, containing at least one member selected from the group consisting of a caspase 14 production promoter, a desmoglein-1 production promoter, and a protein-glutamine γ-glutamyltransferase E production promoter as an active ingredient.

## Description

### [Technical Field]

The present invention relates to a composition for improving at least one skin condition from decreased moisture content of the stratum corneum, spots, decreased barrier function, and abnormal pH value.

### [Background Art]

Many people desire to prevent or improve the deterioration of skin conditions, such as decreased moisture content of the stratum corneum, spots, decreased barrier function, abnormal pH value and the like, which are caused by ultraviolet rays, aging, and the like.

Skin cosmetics and skin external preparations contain moisturizing agents such as glycerin, propylene glycol, and sorbitol to suppress evaporation of moisture. However, suppressing evaporation of moisture alone is not sufficient, and the development of a composition for improving deteriorated skin conditions that can improve skin function itself has been desired.

It has been disclosed that a skin external preparation containing an extract of Akebiae Caulis as an active ingredient promotes the activity of arginase and provides moisture and luster to the skin (Patent Literature 1).

It has been disclosed that an extract from a natural special sesame seed containing catalase and the like is effective in improving rough skin and preventing skin aging (Patent Literature 2).

However, it was not known that these specific proteins can improve skin functions related to decreased moisture content of the stratum corneum, spots, decreased barrier function, and abnormal pH value.

### [Citation List]

### [Patent Literature]

[Patent Literature 1]
   JP H10-7581 A
[Patent Literature 2]
   JP 2010-1267 A

### [Summary of Invention]

### [Technical Problem]

The purpose of the present invention is to provide a composition that can improve at least one skin condition from decreased moisture content of the stratum corneum, spots, decreased barrier function, and abnormal pH value.

### [Solution to Problem]

The present inventors have found that there is a correlation between skin conditions (decreased moisture content of the stratum corneum, spots, decreased barrier function, abnormal pH value) and the amount of a specific protein in the stratum corneum, and further that there is a correlation between the amount of the protein correlated with the skin condition and the amount of a specific amino acid in the stratum corneum.

Based on such new findings, the present inventors have found that the production of the correlated specific protein in the epidermis can be promoted and skin conditions can be improved, by orally or parenterally applying a specific amino acid, and have completed the present invention.

Accordingly, the present invention provides the following.
[1] A composition for improving skin conditions, comprising at least one member selected from the group consisting of a desmoglein-1 production promoter, a caspase 14 production promoter, and a protein-glutamine γ-glutamyltransferase E production promoter as an active ingredient.
[2] The composition of the above-mentioned [1], comprising a desmoglein-1 production promoter which is one or more members selected from the group consisting of Phe, Trp, Met, and Leu.
[3] The composition of the above-mentioned [2], wherein the desmoglein-1 production promoter comprises Phe, Trp, Met, and Leu.
[4] The composition of the above-mentioned [3], wherein a weight ratio of Phe, Trp, Met, and Leu (Phe:Trp:Met:Leu) is 1:0.2 - 2.8:0.4 - 1.1:0.1 - 1.6.
[5] The composition of the above-mentioned [2], wherein the desmoglein-1 production promoter comprises Phe, Trp, and Leu.
[6] The composition of the above-mentioned [5], wherein a weight ratio of Phe, Trp, and Leu (Phe:Trp:Leu) is 1:0.05 - 1.3:0.05 - 0.8.
[7] The composition of any of the above-mentioned [2] to [6], which is for the improvement of decreased moisture contents of the stratum corneum, improvement of skin spots, or improvement of abnormal pH value of the skin surface.
[8] The composition of the above-mentioned [1], comprising a caspase-14 production promoter which is one or more members selected from the group consisting of Val, His, and Leu.
[9] The composition of the above-mentioned [8], wherein the caspase-14 production promoter comprises Val, His, and Leu.
[10] The composition of the above-mentioned [9], wherein a weight ratio of Val, His, and Leu (Val:His:Leu) is 1:0.1 - 2.1:0.1 - 1.8.
[11] The composition of any of the above-mentioned [8] to [10], which is for the improvement of decreased barrier function of the skin.
[12] The composition of the above-mentioned [1], comprising a protein-glutamine γ-glutamyltransferase E production promoter which is one or more members selected from the group consisting of Val and Trp.
[13] The composition of the above-mentioned [12], wherein the protein-glutamine γ-glutamyltransferase E production promoter comprises Val and Trp.
[14] The composition of the above-mentioned [13], wherein a weight ratio of Val and Trp (Val:Trp) is 1:0.1 - 5.9.
[15] The composition of any of the above-mentioned [12] to [14], which is for the improvement of decreased moisture contents of the stratum corneum, or improvement of skin spots.
[16] A composition for promoting production of desmoglein-1, comprising one or more members selected from the group consisting of Phe, Trp, Met, and Leu as an active ingredient.
[17] The composition of the above-mentioned [16], comprising Phe, Trp, Met, and Leu as an active ingredient.
[18] The composition of the above-mentioned [17], wherein a weight ratio of Phe, Trp, Met, and Leu (Phe:Trp:Met:Leu) is 1:0.2 - 2.8:0.4 - 1.1:0.1 - 1.6.
[19] The composition of the above-mentioned [16], comprising Phe, Trp, and Leu as an active ingredient.
[20] The composition of the above-mentioned [19], wherein a weight ratio of Phe, Trp, and Leu (Phe:Trp:Leu) is 1:0.05 - 1.3:0.05 - 0.8.
[21] A composition for promoting production of caspase-14, comprising one or more members selected from the group consisting of Val, His, and Leu as an active ingredient.
[22] The composition of the above-mentioned [21], comprising Val, His, and Leu as an active ingredient.
[23] The composition of the above-mentioned [22], wherein a weight ratio of Val, His, and Leu (Val:His:Leu) is 1:0.1 - 2.1:0.1 - 1.8.
[24] A composition for promoting production of protein-glutamine γ-glutamyltransferase E, comprising one or more members selected from the group consisting of Val and Trp as an active ingredient.
[25] The composition of the above-mentioned [24], comprising Val and Trp as an active ingredient.
[26] The composition of the above-mentioned [25], wherein a weight ratio of Val and Trp (Val:Trp) is 1:0.1 - 5.9.
[27] A method for improving skin conditions comprising administering at least one member selected from the group consisting of a desmoglein-1 production promoter, a caspase 14 production promoter, and a protein-glutamine γ-glutamyltransferase E production promoter to a subject in need thereof.
[28] The method of the above-mentioned [27], comprising administering a desmoglein-1 production promoter which is one or more members selected from the group consisting of Phe, Trp, Met, and Leu.
[29] The method of the above-mentioned [28], wherein the desmoglein-1 production promoter comprises Phe, Trp, Met, and Leu.
[30] The method of the above-mentioned [29], wherein a weight ratio of Phe, Trp, Met, and Leu (Phe:Trp:Met:Leu) is 1:0.2 - 2.8:0.4 - 1.1:0.1 - 1.6.
[31] The method of the above-mentioned [28], wherein the desmoglein-1 production promoter comprises Phe, Trp, and Leu.
[32] The method of the above-mentioned [31], wherein a weight ratio of Phe, Trp, and Leu (Phe:Trp:Leu) is 1:0.05 - 1.3:0.05 - 0.8.
[33] The method of any of [28] to [32], wherein the improvement of skin conditions is improvement of decreased moisture contents of the stratum corneum, improvement of skin spots, or improvement of abnormal pH value of the skin surface.
[34] The method of the above-mentioned [27], comprising administering a caspase-14 production promoter which is one or more members selected from the group consisting of Val, His, and Leu.
[35] The method of the above-mentioned [34], wherein the caspase-14 production promoter comprises Val, His, and Leu.
[36] The method of the above-mentioned [35], wherein a weight ratio of Val, His, and Leu (Val:His:Leu) is 1:0.1 - 2.1:0.1 - 1.8.
[37] The method of any of the above-mentioned [34] to [36], wherein the improvement of skin conditions is improvement of decreased barrier function of the skin.
[38] The method of the above-mentioned [27], comprising administering a protein-glutamine γ-glutamyltransferase E production promoter which is one or more members selected from the group consisting of Val and Trp.
[39] The method of the above-mentioned [38], wherein the protein-glutamine γ-glutamyltransferase E production promoter comprises Val and Trp.
[40] The method of the above-mentioned [39], wherein a weight ratio of Val and Trp (Val:Trp) is 1:0.1 - 5.9.
[41] The method of any of the above-mentioned [38] to [40], wherein the improvement of skin conditions is improvement of decreased moisture contents of the stratum corneum, or improvement of skin spots.
[42] A method for promoting production of desmoglein-1 in a subject in need thereof, comprising administering an effective amount of one or more members selected from the group consisting of Phe, Trp, Met, and Leu to the subject.
[43] The method of the above-mentioned [42], comprising administering Phe, Trp, Met, and Leu.
[44] The method of the above-mentioned [43], wherein a weight ratio of Phe, Trp, Met, and Leu (Phe:Trp:Met:Leu) is 1:0.2 - 2.8:0.4 - 1.1:0.1 - 1.6.
[45] The method of the above-mentioned [42], comprising administering Phe, Trp, and Leu.
[46] The method of the above-mentioned [45], wherein a weight ratio of Phe, Trp, and Leu (Phe:Trp:Leu) is 1:0.05 - 1.3:0.05 - 0.8.
[47] A method for promoting production of caspase-14 in a subject in need thereof, comprising administering an effective amount of one or more members selected from the group consisting of Val, His, and Leu to the subject.
[48] The method of the above-mentioned [47], comprising administering Val, His, and Leu.
[49] The method of the above-mentioned [48], wherein a weight ratio of Val, His, and Leu (Val:His:Leu) is 1:0.1 - 2.1:0.1 - 1.8.
[50] A method for promoting production of protein-glutamine γ-glutamyltransferase E in a subject in need thereof, comprising administering an effective amount of one or more members selected from the group consisting of Val and Trp to the subject.
[51] The method of the above-mentioned [50], comprising administering Val and Trp.
[52] The method of the above-mentioned [51], wherein a weight ratio of Val and Trp (Val:Trp) is 1:0.1 - 5.9.
[53] A desmoglein-1 production promoter, a caspase 14 production promoter, or a protein-glutamine γ-glutamyltransferase E production promoter, for use in improving skin conditions.
[54] The agent of the above-mentioned [53], wherein the desmoglein-1 production promoter is one or more members selected from the group consisting of Phe, Trp, Met, and Leu.
[55] The agent of the above-mentioned [54], wherein the desmoglein-1 production promoter comprises Phe, Trp, Met, and Leu.
[56] The agent of the above-mentioned [55], wherein a weight ratio of Phe, Trp, Met, and Leu (Phe:Trp:Met:Leu) is 1:0.2 - 2.8:0.4 - 1.1:0.1 - 1.6.
[57] The agent of the above-mentioned [54], wherein the desmoglein-1 production promoter comprises Phe, Trp, and Leu.
[58] The agent of the above-mentioned [57], wherein a weight ratio of Phe, Trp, and Leu (Phe:Trp:Leu) is 1:0.05 - 1.3:0.05 - 0.8.
[59] The agent of any of the above-mentioned [54] to [58], for use in the improvement of decreased moisture contents of the stratum corneum, improvement of skin spots, or improvement of abnormal pH value of the skin surface.
[60] The agent of the above-mentioned [53], wherein the caspase-14 production promoter is one or more members selected from the group consisting of Val, His, and Leu.
[61] The agent of the above-mentioned [60], wherein the caspase-14 production promoter comprises Val, His, and Leu.
[62] The agent of [61], wherein a weight ratio of Val, His, and Leu (Val:His:Leu) is 1:0.1 - 2.1:0.1 - 1.8.
[63] The agent of any of the above-mentioned [60] to [62], for use in the improvement of decreased barrier function of the skin.
[64] The agent of the above-mentioned [53], wherein the protein-glutamine γ-glutamyltransferase E production promoter is one or more members selected from the group consisting of Val and Trp.
[65] The agent of [64], wherein the protein-glutamine γ-glutamyltransferase E production promoter comprises Val and Trp.
[66] The agent of the above-mentioned [65], wherein a weight ratio of Val and Trp (Val:Trp) is 1:0.1 - 5.9.
[67] The agent of any of the above-mentioned [64] to [66], for use in the improvement of decreased moisture contents of the stratum corneum, or improvement of skin spots.
[68] One or more amino acids selected from the group consisting of Phe, Trp, Met, and Leu for use in promoting production of desmoglein-1.
[69] The amino acids of the above-mentioned [68], which are Phe, Trp, Met, and Leu.
[70] The amino acids of the above-mentioned [69], wherein a weight ratio of Phe, Trp, Met, and Leu (Phe:Trp:Met:Leu) is 1:0.2 - 2.8:0.4 - 1.1:0.1 - 1.6.
[71] The amino acids of the above-mentioned [68], which are Phe, Trp, and Leu.
[72] The amino acids of the above-mentioned [71], wherein a weight ratio of Phe, Trp, and Leu (Phe:Trp:Leu) is 1:0.05 - 1.3:0.05 - 0.8.
[73] One or more amino acids selected from the group consisting of Val, His, and Leu, for use in promoting production of caspase-14.
[74] The amino acids of the above-mentioned [73], which are Val, His, and Leu.
[75] The amino acids of the above-mentioned [74], wherein a weight ratio of Val, His, and Leu (Val:His:Leu) is 1:0.1 - 2.1:0.1 - 1.8.
[76] One or more amino acids selected from the group consisting of Val and Trp, for use in promoting production of protein-glutamine γ-glutamyltransferase E.
[77] The amino acids of the above-mentioned [76], which are Val and Trp.
[78] The amino acids of the above-mentioned [77], wherein a weight ratio of Val and Trp (Val:Trp) is 1:0.1 - 5.9.
[79] Use of a desmoglein-1 production promoter, a caspase 14 production promoter, or a protein-glutamine γ-glutamyltransferase E production promoter, for producing a composition for improving skin conditions.
[80] The use of the above-mentioned [79], wherein the desmoglein-1 production promoter is one or more members selected from the group consisting of Phe, Trp, Met, and Leu.
[81] The use of the above-mentioned [80], wherein the desmoglein-1 production promoter comprises Phe, Trp, Met, and Leu.
[82] The use of [81], wherein a weight ratio of Phe, Trp, Met, and Leu (Phe:Trp:Met:Leu) is 1:0.2 - 2.8:0.4 - 1.1:0.1 - 1.6.
[83] The use of the above-mentioned [80], wherein the desmoglein-1 production promoter comprises Phe, Trp, and Leu.
[84] The use of the above-mentioned [83], wherein a weight ratio of Phe, Trp, and Leu (Phe:Trp:Leu) is 1:0.05 - 1.3:0.05 - 0.8.
[85] The use of any of the above-mentioned [80] to [84], wherein the improvement of skin conditions is improvement of decreased moisture contents of the stratum corneum, improvement of skin spots, or improvement of abnormal pH value of the skin surface.
[86] The use of the above-mentioned [79], wherein the caspase-14 production promoter is one or more members selected from the group consisting of Val, His, and Leu.
[87] The use of the above-mentioned [86], wherein the caspase-14 production promoter comprises Val, His, and Leu.
[88] The use of the above-mentioned [87], wherein a weight ratio of Val, His, and Leu (Val:His:Leu) is 1:0.1 - 2.1:0.1 - 1.8.
[89] The use of any of the above-mentioned [86] to [88], wherein the improvement of skin conditions is improvement of decreased barrier function of the skin.
[90] The use of the above-mentioned [79], wherein the protein-glutamine γ-glutamyltransferase E production promoter is one or more members selected from the group consisting of Val and Trp.
[91] The use of the above-mentioned [90], wherein protein-glutamine γ-glutamyltransferase E production promoter comprises Val and Trp.
[92] The use of the above-mentioned [91], wherein a weight ratio of Val and Trp (Val:Trp) is 1:0.1 - 5.9.
[93] The use of any of the above-mentioned [90] to [92], wherein the improvement of skin conditions is improvement of decreased moisture contents of the stratum corneum, or improvement of skin spots.
[94] Use of one or more amino acids selected from the group consisting of Phe, Trp, Met, and Leu in producing a composition for promoting production of desmoglein-1.
[95] The use of the above-mentioned [94], wherein the amino acids are Phe, Trp, Met, and Leu.
[96] The use of the above-mentioned [95], wherein a weight ratio of Phe, Trp, Met, and Leu (Phe:Trp:Met:Leu) is 1:0.2 - 2.8:0.4 - 1.1:0.1 - 1.6.
[97] The use of [94], wherein the amino acids are Phe, Trp, and Leu.
[98] The use of the above-mentioned [97], wherein a weight ratio of Phe, Trp, and Leu (Phe:Trp:Leu) is 1:0.05 - 1.3:0.05 - 0.8.
[99] Use of one or more amino acids selected from the group consisting of Val, His, and Leu in producing a composition for promoting production of caspase-14.
[100] The use of the above-mentioned [99], wherein the amino acids are Val, His, and Leu.
[101] The use of the above-mentioned [100], wherein a weight ratio of Val, His, and Leu (Val:His:Leu) is 1:0.1 - 2.1:0.1 - 1.8.
[102] Use of one or more amino acids selected from the group consisting of Val and Trp in producing a composition for promoting production of protein-glutamine γ-glutamyltransferase E.
[103] The use of the above-mentioned [102], wherein the amino acids are Val and Trp.
[104] The use of the above-mentioned [103], wherein a weight ratio of Val and Trp (Val:Trp) is 1:0.1 - 5.9.

[A1] A composition for improving a decreased moisture content of the stratum corneum, comprising at least one member selected from the group consisting of (1) an arginase-1 production promoter, (2) a catalase production promoter, (3) a cystatin-M production promoter, (4) a desmoglein-1 production promoter, (5) a glutathione S-transferase P production promoter, (6) a keratin type II cytoskeleton 78 production promoter, (7) a myosin-14 production promoter, (8) a myosin-9 production promoter, (9) a protein-glutamine γ-glutamyltransferase E production promoter, (10) a γ-glutamyl cyclotransferase production promoter, and (11) a keratin type I cuticle Ha3-I production promoter, as an active ingredient.
[A2] The composition of the above-mentioned [A1], wherein the arginase-1 production promoter is one or more members selected from the group consisting of Val, Leu, Phe, Trp, PCA, and Orn.
[A3] The composition of the above-mentioned [A1], wherein the catalase production promoter is one or more members selected from the group consisting of Val, Ile, His, Asn, Arg, and Tyr.
[A4] The composition of the above-mentioned [A1], wherein the cystatin-M production promoter is one or more members selected from the group consisting of Thr, Leu, Phe, Trp, UCA, and PCA.
[A5] The composition of the above-mentioned [A1], wherein the desmoglein-1 production promoter is one or more members selected from the group consisting of Leu, Met, Phe, Trp, PCA, and Asn.
[A5-1] The composition of the above-mentioned [A1], wherein the desmoglein-1 production promoter comprises Leu, Met, Phe, and Trp.
[A6] The composition of the above-mentioned [A1], wherein the glutathione S-transferase P production promoter is one or more members selected from the group consisting of Ile, Lys, Met, His, Gly, Ser, Asn, and Tyr.
[A7] The composition of the above-mentioned [A1], wherein the keratin type II cytoskeleton 78 production promoter is one or more members selected from the group consisting of Met, His, Trp, PCA, and Orn.
[A8] The composition of the above-mentioned [A1], wherein the myosin-14 production promoter is one or more members selected from the group consisting of Trp, PCA, Orn, and Cit.
[A9] The composition of the above-mentioned [A1], wherein the myosin-9 production promoter is one or more members selected from the group consisting of UCA, Asp, and Cit.
[A10] The composition of the above-mentioned [A1], wherein the protein-glutamine γ-glutamyltransferase E production promoter is one or more members selected from the group consisting of Val, Trp, PCA, Tau, Orn, and Tyr.
[A10-1] The composition of the above-mentioned [A1], wherein the protein-glutamine γ-glutamyltransferase E production promoter comprises Val and Trp.
[A11] The composition of the above-mentioned [A1], wherein the γ-glutamyl cyclotransferase production promoter is one or more members selected from the group consisting of Thr, Asn, and Asp.
[A12] The composition of the above-mentioned [A1], wherein the keratin type I cuticle Ha3-I production promoter is one or more members selected from the group consisting of Met, Tau, and Orn.
[A13] A composition for improving a decreased moisture content of the stratum corneum, which is one or more members selected from the group consisting of Gly, Val, Leu, Ile, Met, Phe, Tyr, Trp, His, Lys, Arg, Ser, Thr, Asp, Asn, Orn, Cit, Tau, UCA, and PCA.
[A14] A composition for improving a decreased moisture content of the stratum corneum, which is one or more members selected from the group consisting of Val, Leu, Ile, Met, Phe, Trp, His, Lys, and Thr.
[A15] A composition for promoting production of arginase-1, comprising one or more members selected from the group consisting of Val, Leu, Phe, Trp, PCA, and Orn as an active ingredient.
[A16] A composition for promoting production of catalase, comprising one or more members selected from the group consisting of Val, Ile, His, Asn, Arg, and Tyr as an active ingredient.
[A17] A composition for promoting production of cystatin-M, comprising one or more members selected from the group consisting of Thr, Leu, Phe, Trp, UCA, and PCA as an active ingredient.
[A18] A composition for promoting production of desmoglein-1, comprising one or more members selected from the group consisting of Leu, Met, Phe, Trp, PCA, and Asn as an active ingredient.
[A18-1] A composition for promoting production of desmoglein-1, comprising Leu, Met, Phe, and Trp as an active ingredient.
[A19] A composition for promoting production of glutathione S-transferase P, comprising one or more members selected from the group consisting of Ile, Lys, Met, His, Gly, Ser, Asn, and Tyr as an active ingredient.
[A20] A composition for promoting production of keratin type II cytoskeleton 78, comprising one or more members selected from the group consisting of Met, His, Trp, PCA, and Orn as an active ingredient.
[A21] A composition for promoting production of myosin-14, comprising one or more members selected from the group consisting of Trp, PCA, Orn, and Cit as an active ingredient.
[A22] A composition for promoting production of myosin-9, comprising one or more members selected from the group consisting of UCA, Asp, and Cit as an active ingredient.
[A23] A composition for promoting production of protein-glutamine γ-glutamyltransferase E, comprising one or more members selected from the group consisting of Val, Trp, PCA, Tau, Orn, and Tyr as an active ingredient.
[A23-1] A composition for promoting production of protein-glutamine γ-glutamyltransferase E, comprising Val and Trp as an active ingredient.
[A24] A composition for promoting production of γ-glutamyl cyclotransferase, comprising one or more members selected from the group consisting of Thr, Asn, and Asp as an active ingredient.
[A25] A composition for promoting production of keratin type I cuticle Ha3-I, comprising one or more members selected from the group consisting of Met, Tau, and Orn as an active ingredient.
[B1] A composition for improving skin spots, comprising at least one member selected from the group consisting of (1) a desmoglein-1 production promoter, (2) a γ-glutamyl cyclotransferase production promoter, (3) a keratin type II cytoskeleton 5 production promoter, (4) a keratin type II cytoskeleton 78 production promoter, (5) a keratin type II cytoskeleton 80 production promoter, (6) a lysosome C production promoter, (7) a neuroblast differentiation-related protein AHNAK production promoter, (8) a protein-glutamine γ-glutamyltransferase E production promoter, (9) a thioredoxin production promoter, (10) a zinc-α-2-glycoprotein production promoter, (11) a cystatin-M production promoter, and (12) a protein S100-A8 production promoter, as an active ingredient.
[B2] The composition of the above-mentioned [B1], wherein the desmoglein-1 production promoter is one or more members selected from the group consisting of Leu, Met, Phe, Trp, PCA, and Asn.
[B2-1] The composition of the above-mentioned [B1], wherein the desmoglein-1 production promoter comprises Leu, Met, Phe, and Trp.
[B3] The composition of the above-mentioned [B1], wherein the γ-glutamyl cyclotransferase production promoter is one or more members selected from the group consisting of Val, Ile, Lys, Met, PCA, Ala, and Asn.
[B4] The composition of the above-mentioned [B1], wherein the keratin type II cytoskeleton 5 production promoter is one or more members selected from the group consisting of Val, Lys, and His.
[B5] The composition of the above-mentioned [B1], wherein the keratin type II cytoskeleton 78 production promoter is one or more members selected from the group consisting of Met, His, Trp, PCA, and Orn.
[B6] The composition of the above-mentioned [B1], wherein the keratin type II cytoskeleton 80 production promoter is one or more members selected from the group consisting of Thr, UCA, and PCA.
[B7] The composition of the above-mentioned [B1], wherein the lysosome C production promoter is one or more members selected from the group consisting of Val, Ile, Leu, Phe, Trp, Ala, Ser, and Tau.
[B8] The composition of the above-mentioned [B1], wherein the neuroblast differentiation-related protein AHNAK production promoter is one or more members selected from the group consisting of Val, Met, His, Trp, Asn, and Orn.
[B9] The composition of the above-mentioned [B1], wherein the protein-glutamine γ-glutamyltransferase E production promoter is one or more members selected from the group consisting of Val, Trp, and Orn.
[B9-1] The composition of the above-mentioned [B1], wherein the protein-glutamine γ-glutamyltransferase E production promoter comprises Val and Trp.
[B10] The composition of the above-mentioned [B1], wherein the thioredoxin production promoter is one or more members selected from the group consisting of PCA, Ser, and Asn.
[B11] The composition of the above-mentioned [B1], wherein the zinc-α-2-glycoprotein production promoter is Asn.
[B12] The composition of the above-mentioned [B1], wherein the cystatin-M production promoter is one or more members selected from the group consisting of Leu, Met, Phe, Trp, Asn, and Gln.
[B13] The composition of the above-mentioned [B1], wherein the protein S100-A8 production promoter is one or more members selected from the group consisting of Leu and Met.
[B14] A composition for improving skin spots, which is one or more members selected from the group consisting of Ala, Val, Leu, Ile, Met, Phe, Trp, His, Lys, Ser, Thr, Asn, Gln, Orn, Tau, UCA, and PCA.
[B15] A composition for improving skin spots, which is one or more members selected from the group consisting of Val, Leu, Ile, Met, Phe, Trp, His, Lys, and Thr.
[B16] A composition for promoting production of desmoglein-1, comprising one or more members selected from the group consisting of Leu, Met, Phe, Trp, PCA, and Asn as an active ingredient.
[B16-1] A composition for promoting production of desmoglein-1, comprising Leu, Met, Phe, and Trp as an active ingredient.
[B17] A composition for promoting production of γ-glutamyl cyclotransferase, comprising one or more members selected from the group consisting of Val, Ile, Lys, Met, PCA, Ala, and Asn as an active ingredient.
[B18] A composition for promoting production of keratin type II cytoskeleton 5, comprising one or more members selected from the group consisting of Val, Lys, and His as an active ingredient.
[B19] A composition for promoting production of keratin type II cytoskeleton 78, comprising one or more members selected from the group consisting of Met, His, Trp, PCA, and Orn as an active ingredient.
[B20] A composition for promoting production of keratin type II cytoskeleton 80, comprising one or more members selected from the group consisting of Thr, UCA, and PCA as an active ingredient.
[B21] A composition for promoting production of lysosome C, comprising one or more members selected from the group consisting of Val, Ile, Leu, Phe, Trp, Ala, Ser, and Tau as an active ingredient.
[B22] A composition for promoting production of neuroblast differentiation-related protein AHNAK, comprising one or more members selected from the group consisting of Val, Met, His, Trp, Asn, and Orn as an active ingredient.
[B23] A composition for promoting production of protein-glutamine γ-glutamyltransferase E, comprising one or more members selected from the group consisting of Val, Trp, and Orn as an active ingredient.
[B23-1] A composition for promoting production of protein-glutamine γ-glutamyltransferase E, comprising Val and Trp as an active ingredient.
[B24] A composition for promoting production of thioredoxin, comprising one or more members selected from the group consisting of PCA, Ser, and Asn as an active ingredient.
[B25] A composition for promoting production of zinc-α-2-glycoprotein, comprising Asn as an active ingredient.
[B26] A composition for promoting production of cystatin-M, comprising one or more members selected from the group consisting of Leu, Met, Phe, Trp, Asn, and Gln as an active ingredient.
[B27] A composition for promoting production of protein S100-A8, comprising one or more members selected from the group consisting of Leu and Met as an active ingredient.
[C1] A composition for improving decreased barrier function of the skin, comprising at least one member selected from the group consisting of (1) a caspase-14 production promoter, (2) a keratin type II cuticle Hb5 production promoter, (3) a keratin type II cytoskeleton 1b production promoter, (4) a phosphatidylethanolamine-binding protein 1 production promoter, (5) a hornerin production promoter, (6) a keratin type I cuticle Ha1 production promoter, (7) a keratin type I cuticle Ha3-I production promoter, (8) a keratin type II cuticle Hb3 production promoter, (9) a keratin type II cuticle Hb6 production promoter, and (10) a thioredoxin production promoter, as an active ingredient.
[C2] The composition of the above-mentioned [C1], wherein the caspase-14 production promoter is one or more members selected from the group consisting of Val, Leu, His, PCA, and Tau.
[C2-1] The composition of the above-mentioned [C1], wherein the caspase-14 production promoter comprises His.
[C2-2] The composition of the above-mentioned [C1], wherein the caspase-14 production promoter comprises Val, Leu, and His.
[C3] The composition of the above-mentioned [C1], wherein the keratin type II cuticle Hb5 production promoter is one or more members selected from the group consisting of Leu, Phe, Orn, and Arg.
[C4] The composition of the above-mentioned [C1], wherein the keratin type II cytoskeleton 1b production promoter is one or more members selected from the group consisting of His, Trp, and Pro.
[C5] The composition of the above-mentioned [C1], wherein the phosphatidylethanolamine-binding protein 1 production promoter is one or more members selected from the group consisting of Val, Lys, His, PCA, Tau, and Glu.
[C6] The composition of the above-mentioned [C1], wherein the hornerin production promoter is one or more members selected from the group consisting of Leu, His, Trp, Gly, Pro, and Cit.
[C7] The composition of the above-mentioned [C1], wherein the keratin type I cuticle Ha1 production promoter is one or more members selected from the group consisting of Met, Tau, Gln, and PCA.
[C8] The composition of the above-mentioned [C1], wherein the keratin type I cuticle Ha3-I production promoter is one or more members selected from the group consisting of Met, Tau, and Orn.
[C9] The composition of the above-mentioned [C1], wherein the keratin type II cuticle Hb3 production promoter is one or more members selected from the group consisting of Thr, Met, His, Phe, Tau, UCA, and PCA.
[C10] The composition of the above-mentioned [C1], wherein the keratin type II cuticle Hb6 production promoter is one or more members selected from the group consisting of Phe, Tau, and PCA.
[C11] The composition of the above-mentioned [C1], wherein the thioredoxin production promoter is one or more members selected from the group consisting of Leu, His, and Phe.
[C12] A composition for improving decreased barrier function of the skin, which is one or more members selected from the group consisting of Gly, Val, Leu, Met, Phe, Trp, His, Lys, Arg, Thr, Glu, Gln, Pro, Orn, Cit, Tau, UCA, and PCA.
[C13] A composition for improving decreased barrier function of the skin, which is one or more members selected from the group consisting of Val, Leu, Met, Phe, Trp, His, Lys, and Thr.
[C14] A composition for promoting production of caspase-14, comprising one or more members selected from the group consisting of Val, Leu, His, PCA, and Tau as an active ingredient.
[C14-1] A composition for promoting production of caspase-14, comprising His as an active ingredient.
[C14-2] A composition for promoting production of caspase-14, comprising Val, Leu, and His as an active ingredient.
[C15] A composition for promoting production of keratin type II cuticle Hb5, comprising one or more members selected from the group consisting of Leu, Phe, Orn, and Arg as an active ingredient.
[C16] A composition for promoting production of keratin type II cytoskeleton 1b, comprising one or more members selected from the group consisting of His, Trp, and Pro as an active ingredient.
[C17] A composition for promoting production of phosphatidylethanolamine-binding protein 1, comprising one or more members selected from the group consisting of Val, Lys, His, PCA, Tau, and Glu as an active ingredient.
[C18] A composition for promoting production of hornerin, comprising one or more members selected from the group consisting of Leu, His, Trp, Gly, Pro, and Cit as an active ingredient.
[C19] A composition for promoting production of keratin type I cuticle Ha1, comprising one or more members selected from the group consisting of Met, Tau, Gln, and PCA as an active ingredient.
[C20] A composition for promoting production of keratin type I cuticle Ha3-I, comprising one or more members selected from the group consisting of Met, Tau, and Orn as an active ingredient.
[C21] A composition for promoting production of keratin type II cuticle Hb3, comprising one or more members selected from the group consisting of Thr, Met, His, Phe, Tau, UCA, and PCA as an active ingredient.
[C22] A composition for promoting production of keratin type II cuticle Hb6, comprising one or more members selected from the group consisting of Phe, Tau, and PCA as an active ingredient.
[C23] A composition for promoting production of thioredoxin, comprising one or more members selected from the group consisting of Leu, His, and Phe as an active ingredient.
[D1] A composition for improving abnormal pH value of the skin surface, comprising at least one member selected from the group consisting of (1) a 78 kDa glucose regulated protein production promoter, (2) an actin cytoplasmic 2 production promoter, (3) an α-actin-4 production promoter, (4) an apolipoprotein D production promoter, (5) a desmoglein-1 production promoter, (6) an epiplakin production promoter, (7) a filaggrin production promoter, (8) a heat shock protein β-1 production promoter, (9) an interleukin-36γ production promoter, (10) a protein S100-P production promoter, (11) an α-enolase production promoter, (12) a keratin type II cuticle Hb3 production promoter, (13) a keratin type II cytoskeleton 75 production promoter, (14) a protein-glutamine γ-glutamyltransferase K production promoter, (15) a serpin B3 production promoter, and (16) a triose phosphoric acid isomerase production promoter as an active ingredient.
[D2] The composition of the above-mentioned [D1], wherein the 78 kDa glucose regulated protein production promoter is Orn.
[D3] The composition of the above-mentioned [D1], wherein the actin cytoplasmic 2 production promoter is one or more members selected from the group consisting of Asn and Orn.
[D4] The composition of the above-mentioned [D1], wherein the α-actin-4 production promoter is one or more members selected from the group consisting of Leu, Lys, Phe, UCA, and Glu.
[D5] The composition of the above-mentioned [D1], wherein the apolipoprotein D production promoter is one or more members selected from the group consisting of Ile, Lys, Phe, Asp, and Arg.
[D6] The composition of the above-mentioned [D1], wherein the desmoglein-1 production promoter is one or more members selected from the group consisting of Leu, Met, Phe, Trp, PCA, and Asn.
[D6-1] The composition of the above-mentioned [D1], wherein the desmoglein-1 production promoter comprises Leu, Met, Phe, and Trp.
[D7] The composition of the above-mentioned [D1], wherein the epiplakin production promoter is one or more members selected from the group consisting of Thr, Met, PCA, and Arg.
[D8] The composition of the above-mentioned [D1], wherein the filaggrin production promoter is one or more members selected from the group consisting of Val and Ile.
[D9] The composition of the above-mentioned [D1], wherein the heat shock protein β-1 production promoter is Orn.
[D10] The composition of the above-mentioned [D1], wherein the interleukin-36γ production promoter is one or more members selected from the group consisting of Ile, Leu, His, Phe, and Cit.
[D11] The composition of the above-mentioned [D1], wherein the protein S100-P production promoter is one or more members selected from the group consisting of Leu, Phe, Trp, Pro, and Glu.
[D12] The composition of the above-mentioned [D1], wherein the α-enolase production promoter is one or more members selected from the group consisting of Thr, Leu, Trp, Gln, and Glu.
[D13] The composition of the above-mentioned [D1], wherein the keratin type II cuticle Hb3 production promoter is one or more members selected from the group consisting of Thr, Met, His, Phe, Tau, UCA, and PCA.
[D14] The composition of the above-mentioned [D1], wherein the keratin type II cytoskeleton 75 production promoter is one or more members selected from the group consisting of Leu, His, Trp, and Tau.
[D15] The composition of the above-mentioned [D1], wherein the protein-glutamine γ-glutamyltransferase K production promoter is one or more members selected from the group consisting of Val, Thr, Ile, His, Ser, and Orn.
[D16] The composition of the above-mentioned [D1], wherein the serpin B3 production promoter is one or more members selected from the group consisting of Val, Leu, Asn, and Gln.
[D17] The composition of the above-mentioned [D1], wherein the triose phosphoric acid isomerase production promoter is one or more members selected from the group consisting of Thr, Leu, Asn, and Gln.
[D18] A composition for improving an abnormal pH value of the skin surface, which is one or more members selected from the group consisting of Val, Leu, Ile, Met, Phe, Trp, His, Lys, Arg, Ser, Thr, Asp, Glu, Asn, Gln, Pro, Orn, Cit, Tau, UCA, and PCA.
[D19] A composition for improving an abnormal pH value of the skin surface, which is one or more members selected from the group consisting of Val, Leu, Ile, Met, Phe, Trp, His, Lys, and Thr.
[D20] A composition for promoting production of 78 kDa glucose regulated protein, comprising Orn as an active ingredient.
[D21] A composition for promoting production of actin cytoplasmic 2, comprising one or more members selected from the group consisting of Asn and Orn as an active ingredient.
[D22] A composition for promoting production of α-actin-4, comprising one or more members selected from the group consisting of Leu, Lys, Phe, UCA, and Glu as an active ingredient.
[D23] A composition for promoting production of apolipoprotein D, comprising one or more members selected from the group consisting of Ile, Lys, Phe, Asp, and Arg as an active ingredient.
[D24] A composition for promoting production of desmoglein-1, comprising one or more members selected from the group consisting of Leu, Met, Phe, Trp, PCA, and Asn as an active ingredient.
[D24-1] A composition for promoting production of desmoglein-1, comprising Leu, Met, Phe, and Trp as an active ingredient.
[D25] A composition for promoting production of epiplakin, comprising one or more members selected from the group consisting of Thr, Met, PCA, and Arg as an active ingredient.
[D26] A composition for promoting production of filaggrin, comprising one or more members selected from the group consisting of Val and Ile as an active ingredient.
[D27] A composition for promoting production of heat shock protein β-1, comprising Orn as an active ingredient.
[D28] A composition for promoting production of interleukin-36γ, comprising one or more members selected from the group consisting of Ile, Leu, His, Phe, and Cit as an active ingredient.
[D29] A composition for promoting production of protein S100-P, comprising one or more members selected from the group consisting of Leu, Phe, Trp, Pro, and Glu as an active ingredient.
[D30] A composition for promoting production of α-enolase, comprising one or more members selected from the group consisting of Thr, Leu, Trp, Gln, and Glu as an active ingredient.
[D31] A composition for promoting production of keratin type II cuticle Hb3, comprising one or more members selected from the group consisting of Thr, Met, His, Phe, Tau, UCA, and PCA as an active ingredient.
[D32] A composition for promoting production of keratin type II cytoskeleton 75, comprising one or more members selected from the group consisting of Leu, His, Trp, and Tau as an active ingredient.
[D33] A composition for promoting production of protein-glutamine γ-glutamyltransferase K, comprising one or more members selected from the group consisting of Val, Thr, Ile, His, Ser, and Orn as an active ingredient.
[D34] A composition for promoting production of serpin B3, comprising one or more members selected from the group consisting of Val, Leu, Asn, and Gln as an active ingredient.
[D35] A composition for promoting production of triose phosphoric acid isomerase, comprising one or more members selected from the group consisting of Thr, Leu, Asn, and Gln as an active ingredient.

### [Advantageous Effects of Invention]

According to the present invention, a composition for improving a decreased moisture content of the stratum corneum, which can improve skin functions related to improving a decreased moisture content of the stratum corneum, can be provided.

According to the present invention, moreover, (1) a composition for promoting production of arginase-1, (2) a composition for promoting production of catalase, (3) a composition for promoting production of cystatin-M, (4) a composition for promoting production of desmoglein-1, (5) a composition for promoting production of glutathione S-transferase P, (6) a composition for promoting production of keratin type II cytoskeleton 78, (7) a composition for promoting production of myosin-14, (8) a composition for promoting production of myosin-9, (9) a composition for promoting production of protein-glutamine γ-glutamyltransferase E, (10) a composition for promoting production of γ-glutamyl cyclotransferase, and (11) a composition for promoting production of keratin type I cuticle Ha3-I, which are novel, can be provided.

According to the present invention, a composition for improving skin spots that can improve skin functions related to the improvement of skin spots can be provided.

According to the present invention, moreover, (1) a composition for promoting production of desmoglein-1, (2) a composition for promoting production of γ-glutamyl cyclotransferase, (3) a composition for promoting production of keratin type II cytoskeleton 5, (4) a composition for promoting production of keratin type II cytoskeleton 78, (5) a composition for promoting production of keratin type II cytoskeleton 80, (6) a composition for promoting production of lysosome C, (7) a composition for promoting production of neuroblast differentiation-related protein AHNAK, (8) a composition for promoting production of protein-glutamine γ-glutamyltransferase E, (9) a composition for promoting production of thioredoxin, (10) a composition for promoting production of zinc-α-2-glycoprotein, (11) a composition for promoting production of cystatin-M, and (12) a composition for promoting production of protein S100-A8, which are novel, can be provided.

According to the present invention, a composition for improving decreased barrier function of the skin, which can improve skin functions related to improving decreased barrier function of the skin, can be provided.

According to the present invention, moreover, (1) a composition for promoting production of caspase-14, (2) a composition for promoting production of keratin type II cuticle Hb5, (3) a composition for promoting production of keratin type II cytoskeleton 1b, (4) a composition for promoting production of phosphatidylethanolamine-binding protein 1, (5) a composition for promoting production of hornerin, (6) a composition for promoting production of keratin type I cuticle Ha1, (7) a composition for promoting production of keratin type I cuticle Ha3-I, (8) a composition for promoting production of keratin type II cuticle Hb3, (9) a composition for promoting production of keratin type II cuticle Hb6, and (10) a composition for promoting production of thioredoxin, which are novel, can be provided.

According to the present invention, a composition for improving an abnormal pH value of the skin surface, which can improve skin functions related to improving an abnormal pH values of the skin surface, can be provided.

According to the present invention, moreover, (1) a composition for promoting production of 78 kDa glucose regulated protein, (2) a composition for promoting production of actin cytoplasmic 2, (3) a composition for promoting production of α-actin-4, (4) a composition for promoting production of apolipoprotein D, (5) a composition for promoting production of desmoglein-1, (6) a composition for promoting production of epiplakin, (7) a composition for promoting production of filaggrin, (8) a composition for promoting production of heat shock protein β-1, (9) a composition for promoting production of interleukin-36γ, (10) a composition for promoting production of protein S100-P, (11) a composition for promoting production of α-enolase, (12) a composition for promoting production of keratin type II cuticle Hb3, (13) a composition for promoting production of keratin type II cytoskeleton 75, (14) a composition for promoting production of protein-glutamine γ-glutamyltransferase K, (15) a composition for promoting production of serpin B3, and (16) a composition for promoting production of triose phosphoric acid isomerase, which are novel, can be provided.

### [Brief Description of Drawings]

[Fig. 1]

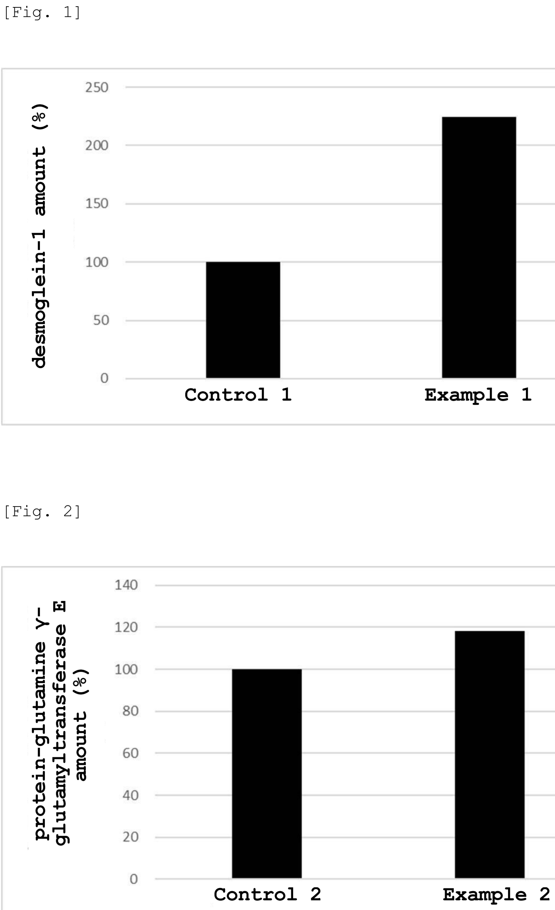

   Fig. 1 shows the results of Experimental Example 2-1.
[Fig. 2]
   Fig. 2 shows the results of Experimental Example 2-2.
[Fig. 3]

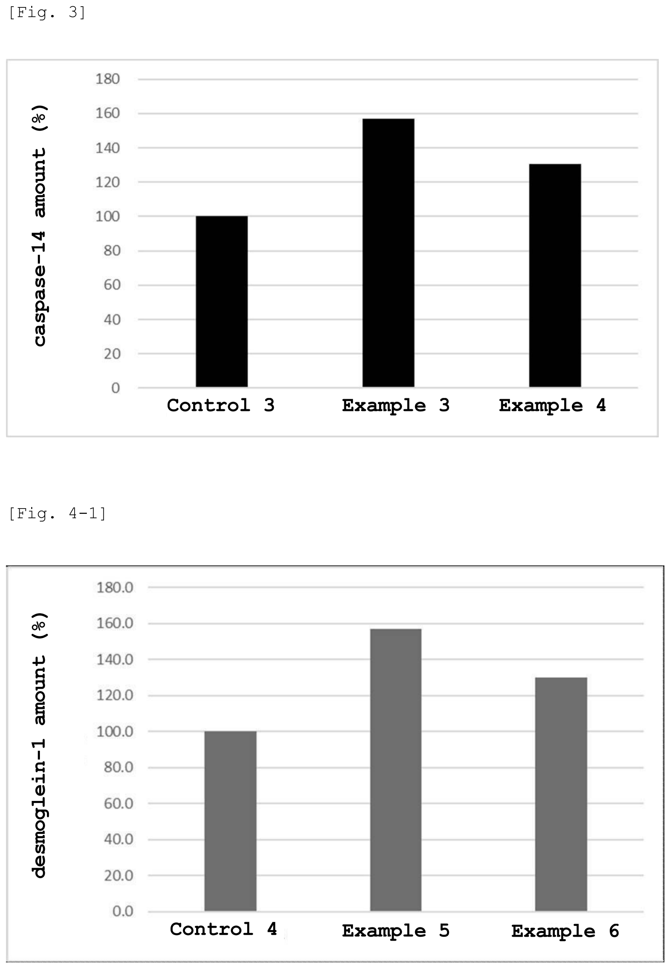

   Fig. 3 shows the results of Experimental Example 2-3.
[Fig. 4-1]
   Fig. 4-1 shows the results of Experimental Example 3-1-1.
[Fig. 4-2]

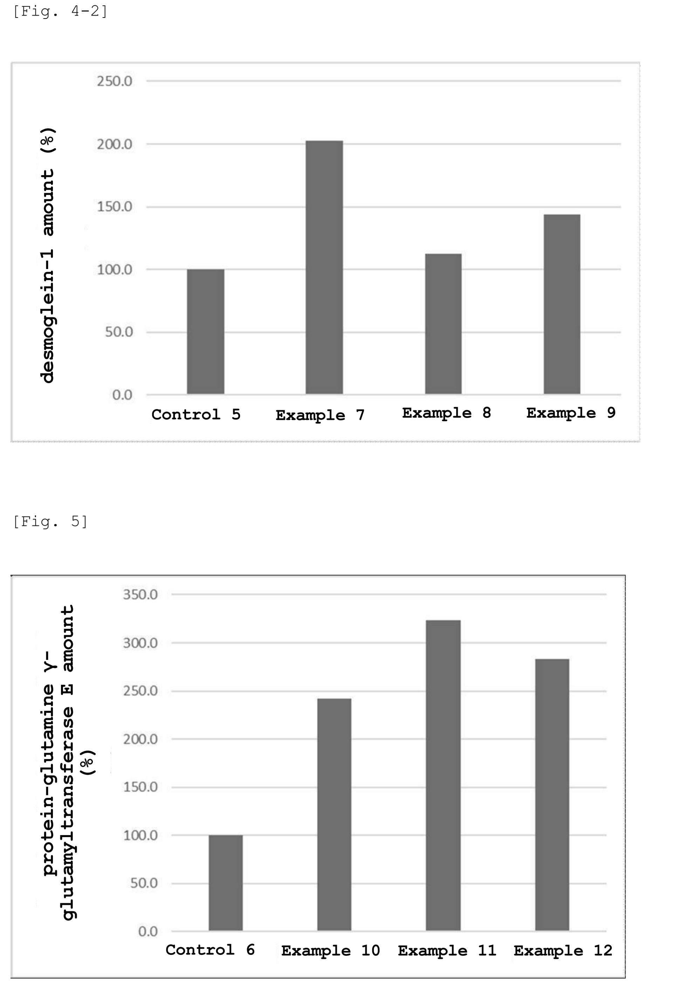

   Fig. 4-2 shows the results of Experimental Example 3-1-2.
[Fig. 5]
   Fig. 5 shows the results of Experimental Example 3-2.
[Fig. 6-1]

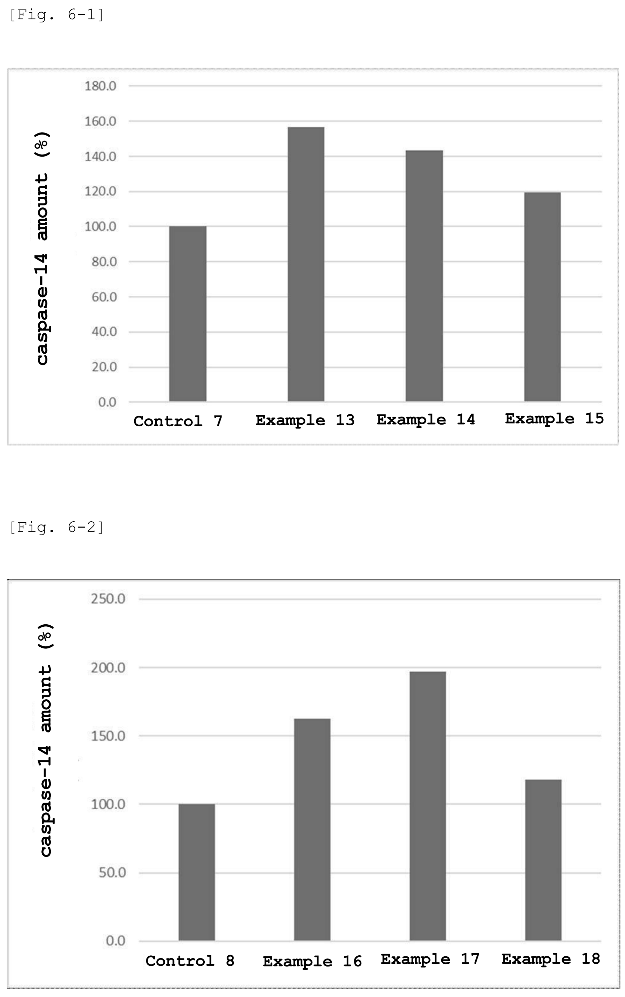

   Fig. 6-1 shows the results of Experimental Example 3-3-1.
[Fig. 6-2]
   Fig. 6-2 shows the results of Experimental Example 3-3-2.
[Fig. 6-3]

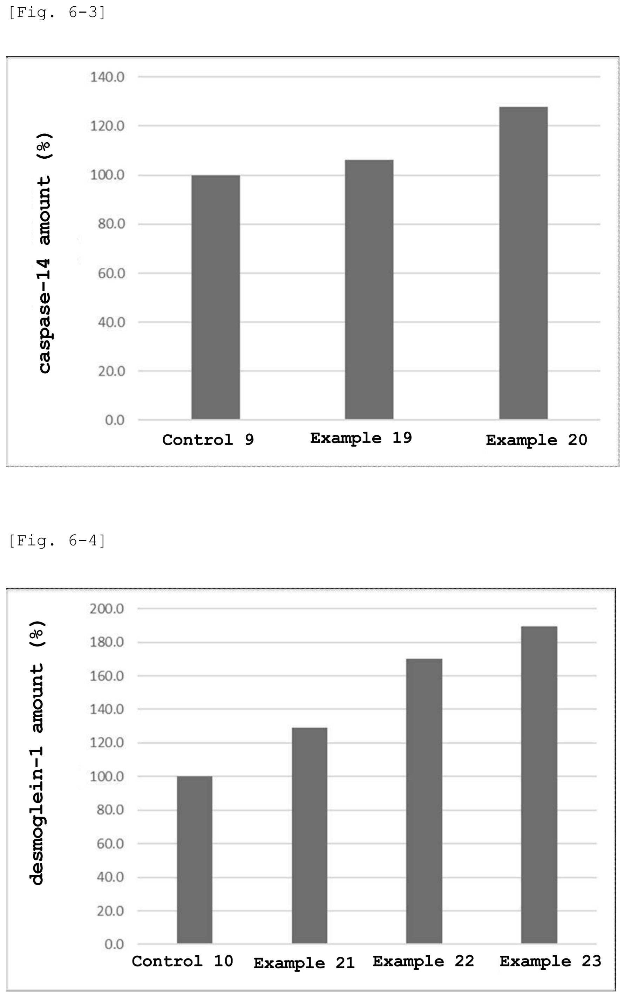

   Fig. 6-3 shows the results of Experimental Example 3-3-3.
[Fig. 6-4]
   Fig. 6-4 shows the results of Experimental Example 3-4.

### [Description of Embodiments]

The present invention is described in detail below.

The abbreviations used in the present specification mean the following:
(1) Gly: glycine
(2) Ala: alanine
(3) Val: valine
(4) Leu: leucine
(5) Ile: isoleucine
(6) Met: methionine
(7) Phe: phenylalanine
(8) Tyr: tyrosine
(9) Trp: tryptophan
(10) His: histidine
(11) Lys: lysine
(12) Arg: arginine
(13) Ser: serine
(14) Thr: threonine
(15) Asp: aspartic acid
(16) Glu: glutamic acid
(17) Asn: asparagine
(18) Gln: glutamine
(19) Pro: proline
(20) Orn: ornithine
(21) Cit: citrulline
(22) Tau: taurine
(23) UCA: urocanic acid
(24) PCA: pyrrolidone carboxylic acid

In the present specification, the "amino acid" is a concept including amino acids and amino acid metabolic intermediates (pyrrolidone carboxylic acid, ornithine, urocanic acid, taurine, and citrulline).

In the present invention, an amino acid in L-form or DL-form is preferred, and L-form is particularly preferred.

In the present invention, the amino acid may be in the form of a salt. Examples of the salt include salts that are acceptable as foods, pharmaceutical products, cosmetics or skin external preparations, and include alkali metal salts such as sodium salt, potassium salt, and the like; alkaline earth metal salts such as calcium salt, magnesium salt, barium salt, and the like; aluminum salt; salts with organic bases such as ethylenediamine, propylenediamine, ethanolamine, monoalkylethanolamine, dialkylethanolamine, diethanolamine, triethanolamine, and the like; salts with inorganic acids such as hydrochloric acid, hydrobromic acid, nitric acid, sulfuric acid, phosphoric acid, and the like; and salts with organic acids such as formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, and the like.

In the present invention, the composition for improving skin conditions includes (A) a composition for improving a decreased moisture content of the stratum corneum, (B) a composition for improving skin spots, (C) a composition for improving decreased barrier function of the skin, and (D) a composition for improving an abnormal pH value of the skin surface.

The (A) composition for improving a decreased moisture content of the stratum corneum, (B) composition for improving skin spots, (C) composition for improving decreased barrier function of the skin, and (D) composition for improving an abnormal pH value of the skin surface of the present invention are described in the following.

### (A) composition for improving a decreased moisture content of the stratum corneum

The composition for improving a decreased moisture content of the stratum corneum of the present invention contains at least one member selected from the group consisting of (1) an arginase-1 production promoter, (2) a catalase production promoter, (3) a cystatin-M production promoter, (4) a desmoglein-1 production promoter, (5) a glutathione S-transferase P production promoter, (6) a keratin type II cytoskeleton 78 production promoter, (7) a myosin-14 production promoter, (8) a myosin-9 production promoter, (9) a protein-glutamine γ-glutamyltransferase E production promoter, (10) a γ-glutamyl cyclotransferase production promoter, and (11) a keratin type I cuticle Ha3-I production promoter as an active ingredient.

The (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, (9) protein-glutamine γ-glutamyltransferase E production promoter, (10) γ-glutamyl cyclotransferase production promoter, and (11) keratin type I cuticle Ha3-I production promoter in the present invention are respectively particularly useful for promoting production, in epidermal keratinocytes, of arginase-1, catalase, cystatin-M, desmoglein-1, glutathione S-transferase P, keratin type II cytoskeleton 78, myosin-14, myosin-9, protein-glutamine γ-glutamyltransferase E, γ-glutamyl cyclotransferase, and keratin type I cuticle Ha3-I.

Among them, (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, and (9) protein-glutamine γ-glutamyltransferase E production promoter are preferably used to improve decreased moisture content of the stratum corneum in young people (e.g., 39 years old or younger), and (10) γ-glutamyl cyclotransferase production promoter and (11) keratin type I cuticle Ha3-I production promoter are preferably used to improve decreased moisture content of the stratum corneum in older people (e.g., 40 years old or older).

In the present invention, "improvement of decreased moisture content of the stratum corneum" refers to improvement of a state in which the moisture-retaining ability of the stratum corneum has decreased due to deterioration of skin condition.

In the present invention, the moisture content of the stratum corneum can be evaluated, for example, by measuring the conductance of the skin surface using Skicon (Yayoi Co., Ltd.), and can be measured according to the below-mentioned Examples.

In the present invention, the arginase-1 production promoter is preferably one or more members selected from the group consisting of Val, Leu, Phe, Trp, PCA, and Orn, more preferably one or more members selected from the group consisting of Val, Leu, Phe, and Trp.

In order to enhance the effect, it is preferable to use two or more of these amino acids in combination.

In the present invention, the catalase production promoter is preferably one or more members selected from the group consisting of Val, Ile, His, Asn, Arg, and Tyr, more preferably one or more members selected from the group consisting of Val, Ile, and His.

In order to enhance the effect, it is preferable to use two or more of these amino acids in combination.

In the present invention, the cystatin-M production promoter id preferably, one or more members selected from the group consisting of Thr, Leu, Phe, Trp, UCA, and PCA, more preferably one or more members selected from the group consisting of Thr, Leu, Phe, and Trp.

In order to enhance the effect, it is preferable to use two or more of these amino acids in combination.

In the present invention, the desmoglein-1 production promoter is preferably one or more members selected from the group consisting of Leu, Met, Phe, Trp, PCA, and Asn, more preferably one or more members selected from the group consisting of Leu, Met, Phe, and Trp.

In order to enhance the effect, it is preferable to use two or more of these amino acids in combination. In the present invention, as the desmoglein-1 production promoter, a combination of Leu, Met, Phe, and Trp is particularly preferred.

In a preferred embodiment, the composition for improving a decreased moisture content of the stratum corneum (e.g., skin lotion, milky lotion and cream) of the present invention contains Leu, Met, Phe, and Trp, and is used at concentrations of Leu 0.00043 - 8.55 wt%, Met 0.00022 - 4.46 wt%, Phe 0.00028 - 5.61 wt%, and Trp 0.00007 - 1.39 wt%, in the composition for improving.

In the present invention, when the desmoglein-1 production promoter is a combination of Phe, Trp, Met, and Leu, the weight ratio of Phe, Trp, Met, and Leu (Phe:Trp:Met:Leu) is preferably 1:0.2 - 2.8:0.4 - 1.1:0.1 - 1.6, more preferably 1:0.2 - 0.3:0.4 - 0.8:1.5 - 1.6, further preferably 1:0.2 - 0.3:0.4 - 0.5:1.5 - 1.6.

Also, in the present invention, as the desmoglein-1 production promoter, a combination of Leu, Phe, and Trp is particularly preferred.

In a preferred embodiment, the composition for improving a decreased moisture content of the stratum corneum (e.g., skin lotion, milky lotion and cream) of the present invention contains Leu, Phe, and Trp, and is used at concentrations of Leu 0.000029 - 0.575 wt%, Phe 0.00043 - 8.69 wt%, and Trp 0.00054 - 10.74 wt%, in the composition for improving.

In the present invention, when the desmoglein-1 production promoter is a combination of Phe, Trp, and Leu, the weight ratio of Phe, Trp, and Leu (Phe:Trp:Leu) is preferably 1:0.05 - 1.3:0.05 - 0.8, more preferably 1:1.2 - 1.3:0.05 - 0.8, further preferably 1:1.2 - 1.3:0.05 - 0.1.

In the present invention, the glutathione S-transferase P production promoter is preferably one or more members selected from the group consisting of Ile, Lys, Met, His, Gly, Ser, Asn, and Tyr, more preferably one or more members selected from the group consisting of Ile, Lys, Met, and His.

In order to enhance the effect, it is preferable to use two or more of these amino acids in combination.

In the present invention, the keratin type II cytoskeleton 78 production promoter is preferably one or more members selected from the group consisting of Met, His, Trp, PCA, and Orn, more preferably one or more members selected from the group consisting of Met, His, and Trp.

In order to enhance the effect, it is preferable to use two or more of these amino acids in combination.

In the present invention, the myosin-14 production promoter is preferably one or more members selected from the group consisting of Trp, PCA, Orn, and Cit, more preferably Trp.

In order to enhance the effect, it is preferable to use two or more of these amino acids in combination.

In the present invention, the myosin-9 production promoter is preferably one or more members selected from the group consisting of UCA, Asp, and Cit.

In order to enhance the effect, it is preferable to use two or more of these amino acids in combination.

In the present invention, the protein-glutamine γ-glutamyltransferase E production promoter is preferably one or more members selected from the group consisting of Val, Trp, PCA, Tau, Orn, and Tyr, more preferably one or more members selected from the group consisting of Val and Trp.

In order to enhance the effect, it is preferable to use two or more of these amino acids in combination. In the present invention, as the protein-glutamine γ-glutamyltransferase E production promoter, a combination of Val and Trp is particularly preferred.

In a preferred embodiment, the composition for improving a decreased moisture content of the stratum corneum (e.g., skin lotion, milky lotion and cream) of the present invention contains Val and Trp, and is used at concentrations of Val 0.00067 - 13.41 wt% and Trp 0.00033 - 6.59 wt%, in the composition for improving.

In the present invention, when the protein-glutamine γ-glutamyltransferase E production promoter is a combination of Val and Trp, the weight ratio of Val and Trp (Val:Trp) is preferably 1:0.1 - 5.9, more preferably 1:0.4 - 5.9, further preferably 1:1.7 - 5.9, particularly preferably 1:1.7 - 1.8.

In the present invention, the γ-glutamyl cyclotransferase production promoter is preferably one or more members selected from the group consisting of Thr, Asn, and Asp, more preferably Thr.

In order to enhance the effect, it is preferable to use two or more of these amino acids in combination.

In the present invention, the keratin type I cuticle Ha3-I production promoter is preferably one or more members selected from the group consisting of Met, Tau, and Orn, more preferably Met.

In order to enhance the effect, it is preferable to use two or more of these amino acids in combination.

The present invention also relates to a composition for improving a decreased moisture content of the stratum corneum, containing one or more members selected from the group consisting of Gly, Val, Leu, Ile, Met, Phe, Tyr, Trp, His, Lys, Arg, Ser, Thr, Asp, Asn, Orn, Cit, Tau, UCA, and PCA (preferably, one or more members selected from the group consisting of Val, Leu, Ile, Met, Phe, Trp, His, Lys, and Thr) as an active ingredient.

The The composition for improving a decreased moisture content of the stratum corneum can be applied orally or parenterally. Examples of the composition to be applied parenterally include cosmetics and skin external preparations. Examples of the composition to be applied orally include foods (e.g., supplement) and pharmaceutical products for oral administration.

When the composition for improving a decreased moisture content of the stratum corneum is a composition to be applied parenterally, the content of the active ingredient is, for example, 0.001 - 20 wt%, preferably 0.01 - 10 wt%, more preferably 0.05 - 5 wt%, further preferably 0.1 - 2 wt%.

When the composition for improving a decreased moisture content of the stratum corneum is a composition to be applied orally, the content of the active ingredient is, for example, 0.01 - 100 wt%, preferably 0.1 - 95 wt%, more preferably 0.5 - 90 wt%, further preferably 2 - 90 wt%.

Examples of the form, additives, method of use, dosage, and the like of the composition for improving a decreased moisture content of the stratum corneum are the same as the examples of the form, additives, method of use, dosage, and the like explained in the aforementioned "composition for improving a decreased moisture content of the stratum corneum containing at least one member selected from the group consisting of (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, (9) protein-glutamine γ-glutamyltransferase E production promoter, (10) γ-glutamyl cyclotransferase production promoter, and (11) keratin type I cuticle Ha3-I production promoter, as an active ingredient".

Also, the present invention relates to a composition for promoting production of arginase-1 (particularly, a composition for promoting production of arginase-1 in epidermal keratinocytes), containing one or more members selected from the group consisting of Val, Leu, Phe, Trp, PCA, and Orn (preferably, one or more members selected from the group consisting of Val, Leu, Phe, and Trp) (the aforementioned arginase-1 production promoter), as an active ingredient;
a composition for promoting production of catalase (particularly, a composition for promoting production of catalase in epidermal keratinocytes), containing one or more members selected from the group consisting of Val, Ile, His, Asn, Arg, and Tyr (preferably, one or more members selected from the group consisting of Val, Ile, and His) (the aforementioned catalase production promoter), as an active ingredient;
a composition for promoting production of cystatin-M (particularly, a composition for promoting production of cystatin-M in epidermal keratinocytes), containing one or more members selected from the group consisting of Thr, Leu, Phe, Trp, UCA, and PCA (preferably, one or more members selected from the group consisting of Thr, Leu, Phe, and Trp) (the aforementioned cystatin-M production promoter), as an active ingredient;
a composition for promoting production of desmoglein-1 (particularly, a composition for promoting production of desmoglein-1 in epidermal keratinocytes), containing one or more members selected from the group consisting of Leu, Met, Phe, Trp, PCA, and Asn (preferably, one or more members selected from the group consisting of Leu, Met, Phe, and Trp, more preferably, a combination of Leu, Met, Phe, and Trp, or a combination of Leu, Phe, and Trp) (the aforementioned desmoglein-1 production promoter), as an active ingredient;
a composition for promoting production of glutathione S-transferase P (particularly, a composition for promoting production of glutathione S-transferase P in epidermal keratinocytes), containing one or more members selected from the group consisting of Ile, Lys, Met, His, Gly, Ser, Asn, and Tyr (preferably, one or more members selected from the group consisting of Ile, Lys, Met, and His) (the aforementioned glutathione S-transferase P production promoter), as an active ingredient;
a composition for promoting production of keratin type II cytoskeleton 78 (particularly, a composition for promoting production of keratin type II cytoskeleton 78 in epidermal keratinocytes), containing one or more members selected from the group consisting of Met, His, Trp, PCA, and Orn (preferably, one or more members selected from the group consisting of Met, His, and Trp) (the aforementioned keratin type II cytoskeleton 78 production promoter), as an active ingredient;
a composition for promoting production of myosin-14 (particularly, a composition for promoting production of myosin-14 in epidermal keratinocytes), containing one or more members selected from the group consisting of Trp, PCA, Orn, and Cit (preferably, Trp) (the aforementioned myosin-14 production promoter), as an active ingredient;
a composition for promoting production of myosin-9 (particularly, a composition for promoting production of myosin-9 in epidermal keratinocytes), containing one or more members selected from the group consisting of UCA, Asp, and Cit (the aforementioned myosin-9 production promoter), as an active ingredient;
a composition for promoting production of protein-glutamine γ-glutamyltransferase E (particularly, a composition for promoting production of protein-glutamine γ-glutamyltransferase E in epidermal keratinocytes), containing one or more members selected from the group consisting of Val, Trp, PCA, Tau, Orn, and Tyr (preferably, one or more members selected from the group consisting of Val and Trp, more preferably, a combination of Val and Trp) (the aforementioned protein-glutamine γ-glutamyltransferase E production promoter), as an active ingredient;
a composition for promoting production of γ-glutamyl cyclotransferase (particularly, a composition for promoting production of γ-glutamyl cyclotransferase in epidermal keratinocytes), containing one or more members selected from the group consisting of Thr, Asn, and Asp (preferably, Thr) (the aforementioned γ-glutamyl cyclotransferase production promoter), as an active ingredient; and
a composition for promoting production of keratin type I cuticle Ha3-I (particularly, a composition for promoting production of keratin type I cuticle Ha3-I in epidermal keratinocytes), containing one or more members selected from the group consisting of Met, Tau, and Orn (preferably, Met) (the aforementioned keratin type I cuticle Ha3-I production promoter), as an active ingredient.

The composition for promoting production of arginase-1, the composition for promoting production of catalase, the composition for promoting production of cystatin-M, the composition for promoting production of desmoglein-1, the composition for promoting production of glutathione S-transferase P, the composition for promoting production of keratin type II cytoskeleton 78, the composition for promoting production of myosin-14, the composition for promoting production of myosin-9, the composition for promoting production of protein-glutamine γ-glutamyltransferase E, the composition for promoting production of γ-glutamyl cyclotransferase, and the composition for promoting production of keratin type I cuticle Ha3-I, of the present invention, can be used for the improvement of diseases and conditions (e.g., improvement of decreased moisture content of the stratum corneum, etc.) that require promoted production of arginase-1, catalase, cystatin-M, desmoglein-1, glutathione S-transferase P, keratin type II cytoskeleton 78, myosin-14, myosin-9, protein-glutamine γ-glutamyltransferase E, γ-glutamyl cyclotransferase, and keratin type I cuticle Ha3-I.

Examples of the active ingredient content, form, additives, method of use, dosage, ratio of amino acid as active ingredient, and the like of the composition for promoting production of arginase-1, the composition for promoting production of catalase, the composition for promoting production of cystatin-M, the composition for promoting production of desmoglein-1, the composition for promoting production of glutathione S-transferase P, the composition for promoting production of keratin type II cytoskeleton 78, the composition for promoting production of myosin-14, the composition for promoting production of myosin-9, the composition for promoting production of protein-glutamine γ-glutamyltransferase E, the composition for promoting production of γ-glutamyl cyclotransferase, and the composition for promoting production of keratin type I cuticle Ha3-I, of the present invention, are the same as the examples of the active ingredient content, form, additives, method of use, dosage, ratio of amino acid as active ingredient, and the like explained in the aforementioned "composition for improving a decreased moisture content of the stratum corneum, containing at least one member selected from the group consisting of (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, (9) protein-glutamine γ-glutamyltransferase E production promoter, (10) γ-glutamyl cyclotransferase production promoter, and (11) keratin type I cuticle Ha3-I production promoter, as an active ingredient".

The composition for improving a decreased moisture content of the stratum corneum of the present invention can be applied orally or parenterally. Examples of the composition to be applied parenterally include cosmetics and skin external preparations. Examples of the composition to be applied orally include foods (e.g., supplement) and pharmaceutical products for oral administration.

When the composition for improving a decreased moisture content of the stratum corneum of the present invention is a composition to be applied parenterally, the content of the active ingredient (at least one member selected from the group consisting of (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, (9) protein-glutamine γ-glutamyltransferase E production promoter, (10) γ-glutamyl cyclotransferase production promoter, and (11) keratin type I cuticle Ha3-I production promoter) in the composition for improving a decreased moisture content of the stratum corneum of the present invention is, for example, 0.001 - 20 wt%, preferably 0.01 - 10 wt%, more preferably 0.05 - 5 wt%, further preferably 0.1 - 2 wt%.

When the composition for improving a decreased moisture content of the stratum corneum of the present invention is a composition to be applied orally, the content of the active ingredient (at least one member selected from the group consisting of (1) arginase-1 production promoter, (2) catalase production promoter, (3) cystatin-M production promoter, (4) desmoglein-1 production promoter, (5) glutathione S-transferase P production promoter, (6) keratin type II cytoskeleton 78 production promoter, (7) myosin-14 production promoter, (8) myosin-9 production promoter, (9) protein-glutamine γ-glutamyltransferase E production promoter, (10) γ-glutamyl cyclotransferase production promoter, and (11) keratin type I cuticle Ha3-I production promoter) in the composition for improving a decreased moisture content of the stratum corneum of the present invention is, for example, 0.01 - 100 wt%, preferably 0.1 - 95 wt%, more preferably 0.5 - 90 wt%, further preferably 2 - 90 wt%.

### (B) Composition for improving skin spots

The composition for improving skin spots of the present invention contains at least one member selected from the group consisting of (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter, as an active ingredient.

The (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter in the present invention are respectively particularly useful for promoting production, in epidermal keratinocytes, of desmoglein-1, γ-glutamyl cyclotransferase, keratin type II cytoskeleton 5, keratin type II cytoskeleton 78, keratin type II cytoskeleton 80, lysosome C, neuroblast differentiation-related protein AHNAK, protein-glutamine γ-glutamyltransferase E, thioredoxin, zinc-α-2-glycoprotein, cystatin-M, and protein S100-A8.

Among them, (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, and (10) zinc-α-2-glycoprotein production promoter are preferably used to improve spots in young people (e.g., 39 years old or younger), and (10) zinc-α-2-glycoprotein production promoter, (11) cystatin-M production promoter, and (12) protein S100-A8 production promoter are preferably used to improve spots in older people (e.g., 40 years old or older).

In the present invention, the "improvement of skin spots" refers to improving the skin condition to one in which spots are less likely to occur, thereby reducing spots, wherein the spot is a condition in which brown pigmentation is deposited on the skin. Furthermore, yellowing, dullness, and lower transparency of the skin caused by melanin pigments are also expected to be improved.

In the present invention, the amount of melanin can be measured, for example, using a hue colorimeter or the like in accordance with the below-mentioned Example.

In the present invention, the desmoglein-1 production promoter is preferably one or more members selected from the group consisting of Leu, Met, Phe, Trp, PCA, and Asn, more preferably one or more members selected from the group consisting of Leu, Met, Phe, and Trp.

In order to enhance the effect, it is preferable to use two or more of these amino acids in combination. In the present invention, as the desmoglein-1 production promoter, a combination of Leu, Met, Phe, and Trp is particularly preferred.

In a preferred embodiment, the composition for improving skin spots of the present invention (e.g., skin lotion, milky lotion and cream) contains Leu, Met, Phe, and Trp, and is used at concentrations of Leu 0.00043 - 8.55 wt%, Met 0.00022 - 4.46 wt%, Phe 0.00028 - 5.61 wt%, and Trp 0.00007 - 1.39 wt%, in the composition for improving.

In the present invention, when the desmoglein-1 production promoter is a combination of Phe, Trp, Met, and Leu, the weight ratio of Phe, Trp, Met, and Leu (Phe:Trp:Met:Leu) is preferably 1:0.2 - 2.8:0.4 - 1.1:0.1 - 1.6, more preferably 1:0.2 - 0.3:0.4 - 0.8:1.5 - 1.6, further preferably 1:0.2 - 0.3:0.4 - 0.5:1.5 - 1.6.

Also, in the present invention, as the desmoglein-1 production promoter, a combination of Leu, Phe, and Trp is particularly preferred.

In a preferred embodiment, the composition for improving skin spots of the present invention (e.g., skin lotion, milky lotion, and cream) contains Leu, Phe, and Trp, and is used at concentrations of Leu 0.000029 - 0.575 wt%, Phe 0.00043 - 8.69 wt%, and Trp 0.00054 - 10.74 wt%, in the composition for improving.

In the present invention, when the desmoglein-1 production promoter is a combination of Phe, Trp, and Leu, the weight ratio of Phe, Trp, and Leu (Phe:Trp:Leu) is preferably 1:0.05 - 1.3:0.05 - 0.8, more preferably 1:1.2 - 1.3:0.05 - 0.8, further preferably 1:1.2 - 1.3:0.05 - 0.1.

In the present invention, the γ-glutamyl cyclotransferase production promoter is preferably one or more members selected from the group consisting of Val, Ile, Lys, Met, PCA, Ala, and Asn, more preferably one or more members selected from the group consisting of Val, Ile, Lys, and Met.

In order to enhance the effect, it is preferable to use two or more of these amino acids in combination.

In the present invention, the keratin type II cytoskeleton 5 production promoter is preferably one or more members selected from the group consisting of Val, Lys, and His.

In order to enhance the effect, it is preferable to use two or more of these amino acids in combination.

In the present invention, the keratin type II cytoskeleton 78 production promoter is preferably one or more members selected from the group consisting of Met, His, Trp, PCA, and Orn, more preferably one or more members selected from the group consisting of Met, His, and Trp.

In order to enhance the effect, it is preferable to use two or more of these amino acids in combination.

In the present invention, the keratin type II cytoskeleton 80 production promoter is preferably one or more members selected from the group consisting of Thr, UCA, and PCA, more preferably Thr.

In order to enhance the effect, it is preferable to use two or more of these amino acids in combination.

In the present invention, the lysosome C production promoter is preferably one or more members selected from the group consisting of Val, Ile, Leu, Phe, Trp, Ala, Ser, and Tau, more preferably one or more members selected from the group consisting of Val, Ile, Leu, Phe, and Trp.

In order to enhance the effect, it is preferable to use two or more of these amino acids in combination.

In the present invention, the neuroblast differentiation-related protein AHNAK production promoter is preferably one or more members selected from the group consisting of Val, Met, His, Trp, Asn, and Orn, more preferably one or more members selected from the group consisting of Val, Met, His, and Trp.

In order to enhance the effect, it is preferable to use two or more of these amino acids in combination.

In the present invention, the protein-glutamine γ-glutamyltransferase E production promoter is preferably one or more members selected from the group consisting of Val, Trp, and Orn, more preferably one or more members selected from the group consisting of Val and Trp.

In order to enhance the effect, it is preferable to use two or more of these amino acids in combination. In the present invention, as the protein-glutamine γ-glutamyltransferase E production promoter, a combination of Val and Trp is particularly preferred.

In a preferred embodiment, the composition for improving skin spots of the present invention (e.g., skin lotion, milky lotion and cream) contains Val and Trp, and is used at concentrations of Val 0.00067 - 13.41 wt% and Trp 0.00033 - 6.59 wt%, in the composition for improving.

In the present invention, when the protein-glutamine γ-glutamyltransferase E production promoter is a combination of Val and Trp, the weight ratio of Val and Trp (Val:Trp) is preferably 1:0.1 - 5.9, more preferably 1:0.4 - 5.9, further preferably 1:1.7 - 5.9, particularly preferably 1:1.7 - 1.8.

In the present invention, the thioredoxin production promoter is preferably one or more members selected from the group consisting of PCA, Ser, and Asn.

In order to enhance the effect, it is preferable to use two or more of these amino acids in combination.

In the present invention, the zinc-α-2-glycoprotein production promoter is preferably Asn.

In the present invention, the cystatin-M production promoter is preferably one or more members selected from the group consisting of Leu, Met, Phe, Trp, Asn, and Gln, more preferably one or more members selected from the group consisting of Leu, Met, Phe, and Trp.

In order to enhance the effect, it is preferable to use two or more of these amino acids in combination.

In the present invention, the protein S100-A8 production promoter is preferably one or more members selected from the group consisting of Leu and Met.

In order to enhance the effect, it is preferable to use two or more of these amino acids in combination.

The present invention also relates to a composition for improving skin spots, containing one or more members selected from the group consisting of Ala, Val, Leu, Ile, Met, Phe, Trp, His, Lys, Ser, Thr, Asn, Gln, Orn, Tau, UCA, and PCA (preferably, one or more members selected from the group consisting of Val, Leu, Ile, Met, Phe, Trp, His, Lys, and Thr), as an active ingredient.

The composition for improving skin spots can be applied orally or parenterally. Examples of the composition to be applied parenterally include cosmetics and skin external preparations. Examples of the composition to be applied orally include foods (e.g., supplement) and pharmaceutical products for oral administration.

When the composition for improving skin spots is a composition to be applied parenterally, the content of the active ingredient is, for example, 0.001 - 20 wt%, preferably 0.01 - 10 wt%, more preferably 0.05 - 5 wt%, further preferably 0.1 - 2 wt%.

When the composition for improving skin spots is a composition to be applied orally, the content of the active ingredient is, for example, 0.01 - 100 wt%, preferably 0.1 - 95 wt%, more preferably 0.5 - 90 wt%, further preferably 2 - 90 wt%.

Examples of the form, additives, method of use, dosage, and the like of the The composition for improving skin spots are the same as the examples of the form, additives, method of use, dosage, and the like explained in the aforementioned "composition for improving skin spots, containing at least one member selected from the group consisting of (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter, as an active ingredient".

In addition, the present invention relates to a composition for promoting production of desmoglein-1 (particularly, a composition for promoting production of desmoglein-1 in epidermal keratinocytes), containing one or more members selected from the group consisting of Leu, Met, Phe, Trp, PCA, and Asn (preferably, one or more members selected from the group consisting of Leu, Met, Phe, and Trp, more preferably, a combination of Leu, Met, Phe and Trp, or a combination of Leu, Phe and Trp) (the aforementioned desmoglein-1 production promoter), as an active ingredient;
a composition for promoting production of γ-glutamyl cyclotransferase (particularly, a composition for promoting production of γ-glutamyl cyclotransferase in epidermal keratinocytes), containing one or more members selected from the group consisting of Val, Ile, Lys, Met, PCA, Ala, and Asn (preferably, one or more members selected from the group consisting of Val, Ile, Lys, and Met) (the aforementioned γ-glutamyl cyclotransferase production promoter), as an active ingredient;
a composition for promoting production of keratin type II cytoskeleton 5 (particularly, a composition for promoting production of keratin type II cytoskeleton 5 in epidermal keratinocytes), containing one or more members selected from the group consisting of Val, Lys, and His (the aforementioned keratin type II cytoskeleton 5 production promoter), as an active ingredient;
a composition for promoting production of keratin type II cytoskeleton 78 (particularly, a composition for promoting production of keratin type II cytoskeleton 78 in epidermal keratinocytes), containing one or more members selected from the group consisting of Met, His, Trp, PCA, and Orn (preferably, one or more members selected from the group consisting of Met, His, and Trp) (the aforementioned keratin type II cytoskeleton 78 production promoter), as an active ingredient;
a composition for promoting production of keratin type II cytoskeleton 80 (particularly, a composition for promoting production of keratin type II cytoskeleton 80 in epidermal keratinocytes), containing one or more members selected from the group consisting of Thr, UCA, and PCA (preferably, Thr) (the aforementioned keratin type II cytoskeleton 80 production promoter), as an active ingredient;
a composition for promoting production of lysosome C (particularly, a composition for promoting production of lysosome C in epidermal keratinocytes), containing one or more members selected from the group consisting of Val, Ile, Leu, Phe, Trp, Ala, Ser, and Tau (preferably, one or more members selected from the group consisting of Val, Ile, Leu, Phe, and Trp) (the aforementioned lysosome C production promoter), as an active ingredient;
a composition for promoting production of neuroblast differentiation-related protein AHNAK (particularly, a composition for promoting production of neuroblast differentiation-related protein AHNAK in epidermal keratinocytes), containing one or more members selected from the group consisting of Val, Met, His, Trp, Asn, and Orn (preferably, one or more members selected from the group consisting of Val, Met, His, and Trp) (the aforementioned neuroblast differentiation-related protein AHNAK production promoter), as an active ingredient;
a composition for promoting production of protein-glutamine γ-glutamyltransferase E (particularly, a composition for promoting production of protein-glutamine γ-glutamyltransferase E in epidermal keratinocytes), containing one or more members selected from the group consisting of Val, Trp, and Orn (preferably, one or more members selected from the group consisting of Val and Trp, more preferably, a combination of Val and Trp) (the aforementioned protein-glutamine γ-glutamyltransferase E production promoter), as an active ingredient;
a composition for promoting production of thioredoxin (particularly, a composition for promoting production of thioredoxin in epidermal keratinocytes), containing one or more members selected from the group consisting of PCA, Ser, and Asn (the aforementioned thioredoxin production promoter), as an active ingredient;
a composition for promoting production of zinc-α-2-glycoprotein (particularly, a composition for promoting production of zinc-α-2-glycoprotein in epidermal keratinocytes), containing Asn (the aforementioned zinc-α-2-glycoprotein production promoter), as an active ingredient;
a composition for promoting production of cystatin-M (particularly, a composition for promoting production of cystatin-M in epidermal keratinocytes), containing one or more members selected from the group consisting of Leu, Met, Phe, Trp, Asn, and Gln (preferably, one or more members selected from the group consisting of Leu, Met, Phe, and Trp) (the aforementioned cystatin-M production promoter), as an active ingredient; and
a composition for promoting production of protein S100-A8 (particularly, a composition for promoting production of protein S100-A8 in epidermal keratinocytes), containing one or more members selected from the group consisting of Leu and Met (the aforementioned protein S100-A8 production promoter), as an active ingredient.

The composition for promoting production of desmoglein-1, the composition for promoting production of γ-glutamyl cyclotransferase, the composition for promoting production of keratin type II cytoskeleton 5, the composition for promoting production of keratin type II cytoskeleton 78, the composition for promoting production of keratin type II cytoskeleton 80, the composition for promoting production of lysosome C, the composition for promoting production of neuroblast differentiation-related protein AHNAK, the composition for promoting production of protein-glutamine γ-glutamyltransferase E, the composition for promoting production of thioredoxin, the composition for promoting production of zinc-α-2-glycoprotein, the composition for promoting production of cystatin-M, and the composition for promoting production of protein S100-A8, of the present invention, can be used for the improvement of diseases and conditions (e.g., improvement of skin spots, etc.) that require promoted production of desmoglein-1, γ-glutamyl cyclotransferase, keratin type II cytoskeleton 5, keratin type II cytoskeleton 78, keratin type II cytoskeleton 80, lysosome C, neuroblast differentiation-related protein AHNAK, protein-glutamine γ-glutamyltransferase E, thioredoxin, zinc-α-2-glycoprotein, cystatin-M, and protein S100-A8.

Examples of the active ingredient content, form, additives, method of use, dosage, ratio of amino acid as active ingredient, and the like of the composition for promoting production of desmoglein-1, the composition for promoting production of γ-glutamyl cyclotransferase, the composition for promoting production of keratin type II cytoskeleton 5, the composition for promoting production of keratin type II cytoskeleton 78, the composition for promoting production of keratin type II cytoskeleton 80, the composition for promoting production of lysosome C, the composition for promoting production of neuroblast differentiation-related protein AHNAK, the composition for promoting production of protein-glutamine γ-glutamyltransferase E, the composition for promoting production of thioredoxin, the composition for promoting production of zinc-α-2-glycoprotein, the composition for promoting production of cystatin-M, and the composition for promoting production of protein S100-A8, of the present invention, are the same as the examples of the active ingredient content, form, additives, method of use, dosage, ratio of amino acid as active ingredient, and the like explained in the aforementioned "composition for improving skin spots, containing at least one member selected from the group consisting of (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter, as an active ingredient".

The composition for improving skin spots of the present invention can be applied orally or parenterally. Examples of the composition to be applied parenterally include cosmetics and skin external preparations. Examples of the composition to be applied orally include foods (e.g., supplement) and pharmaceutical products for oral administration.

When the composition for improving skin spots of the present invention is a composition to be applied parenterally, the content of the active ingredient (at least one member selected from the group consisting of (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter) in the composition for improving skin spots of the present invention is, for example, 0.001 - 20 wt%, preferably 0.01 - 10 wt%, more preferably 0.05 - 5 wt%, further preferably 0.1 - 2 wt%.

When the composition for improving skin spots of the present invention is a composition to be applied orally, the content of the active ingredient (at least one member selected from the group consisting of (1) desmoglein-1 production promoter, (2) γ-glutamyl cyclotransferase production promoter, (3) keratin type II cytoskeleton 5 production promoter, (4) keratin type II cytoskeleton 78 production promoter, (5) keratin type II cytoskeleton 80 production promoter, (6) lysosome C production promoter, (7) neuroblast differentiation-related protein AHNAK production promoter, (8) protein-glutamine γ-glutamyltransferase E production promoter, (9) thioredoxin production promoter, (10) zinc-α-2-glycoprotein production promoter, (11) cystatin-M production promoter, and (12) protein S100-A8 production promoter) in the composition for improving skin spots of the present invention is, for example, 0.01 - 100 wt%, preferably 0.1 - 95 wt%, more preferably 0.5 - 90 wt%, further preferably 2 - 90 wt%.

### (C) Composition for improving decreased barrier function of the skin

The composition for improving decreased barrier function of the skin of the present invention contains at least one member selected from the group consisting of (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter, as an active ingredient.

The (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter in the present invention are respectively particularly useful for promoting production, in epidermal keratinocytes, of caspase-14, keratin type II cuticle Hb5, keratin type II cytoskeleton 1b, phosphatidylethanolamine-binding protein 1, hornerin, keratin type I cuticle Ha1, keratin type I cuticle Ha3-I, keratin type II cuticle Hb3, keratin type II cuticle Hb6, and thioredoxin.

Among them, (1) caspase-14 production promoter, (2) keratin type II cuticle Hb5 production promoter, (3) keratin type II cytoskeleton 1b production promoter, and (4) phosphatidylethanolamine-binding protein 1 production promoter are preferably used to improve decreased barrier function in young people (e.g., 39 years old or younger), and (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter are preferably used to improve barrier function in older people (e.g., 40 years old or older).

In the present invention, the "improvement of decreased barrier function of the skin" refers to improvement of the function of the skin in controlling the loss of internal components (for example, water and natural moisturizing factors) and preventing the intrusion of foreign substances (chemical substances, microorganisms, etc.) into the body.

In the present invention, the transepidermal water transpiration amount can be measured, for example, in accordance with the below-mentioned Examples and using a combination of Cutometer DUAL MPA580 and Tewameter TM300 (both from Courage+Khazaka).

In the present invention, the caspase-14 production promoter is preferably one or more members selected from the group consisting of Val, Leu, His, PCA, and Tau, more preferably one or more members selected from the group consisting of Val, Leu, and His, further preferably His.

In order to enhance the effect, it is preferable to use two or more of these amino acids in combination. In the present invention, caspase-14 production promoter is particularly preferably a combination of Val, Leu, and His.

In a preferred embodiment, the composition for improving decreased barrier function of the skin of the present invention (e.g., skin lotion, milky lotion and cream) contains His, and is used at a concentration of His 0.001 - 20 wt% in the composition for improving.

In a preferred embodiment, moreover, the composition for improving decreased barrier function of the skin of the present invention (e.g., skin lotion, milky lotion and cream) contains Val, Leu, and His, and is used at concentrations of Val 0.00032 - 6.35 wt%, Leu 0.00054 - 10.77 wt%, and His 0.00014 - 2.88 wt%, in the composition for improving.

In the present invention, when the caspase-14 production promoter is a combination of Val, His, and Leu, the weight ratio of Val, His, and Leu (Val:His:Leu) is preferably 1:0.1 - 2.1:0.1 - 1.8, more preferably 1:0.1 - 2.1:0.3 - 1.8, further preferably 1:0.1 - 1.4:1.1 - 1.8, particularly preferably 1:0.3 - 1.4:1.1 - 1.8, 1:0.4 - 1.4:1.1 - 1.8, 1:1.3 - 1.4:1.1 - 1.2.

In the present invention, the keratin type II cuticle Hb5 production promoter is preferably one or more members selected from the group consisting of Leu, Phe, Orn, and Arg, more preferably one or more members selected from the group consisting of Leu and Phe.

In order to enhance the effect, it is preferable to use two or more of these amino acids in combination.

In the present invention, the keratin type II cytoskeleton 1b production promoter is preferably one or more members selected from the group consisting of His, Trp, and Pro, more preferably one or more members selected from the group consisting of His and Trp.

In order to enhance the effect, it is preferable to use two or more of these amino acids in combination.

In the present invention, the phosphatidylethanolamine-binding protein 1 production promoter is preferably one or more members selected from the group consisting of Val, Lys, His, PCA, Tau, and Glu, more preferably one or more members selected from the group consisting of Val, Lys, and His.

In order to enhance the effect, it is preferable to use two or more of these amino acids in combination.

In the present invention, the hornerin production promoter is preferably one or more members selected from the group consisting of Leu, His, Trp, Gly, Pro, and Cit, more preferably one or more members selected from the group consisting of Leu, His, and Trp.

In order to enhance the effect, it is preferable to use two or more of these amino acids in combination.

In the present invention, the keratin type I cuticle Ha1 production promoter is preferably one or more members selected from the group consisting of Met, Tau, Gln, and PCA, more preferably Met.

In order to enhance the effect, it is preferable to use two or more of these amino acids in combination.

In the present invention, the keratin type I cuticle Ha3-I production promoter is preferably one or more members selected from the group consisting of Met, Tau, and Orn, more preferably Met.

In order to enhance the effect, it is preferable to use two or more of these amino acids in combination.

In the present invention, the keratin type II cuticle Hb3 production promoter is preferably one or more members selected from the group consisting of Thr, Met, His, Phe, Tau, UCA, and PCA, more preferably one or more members selected from the group consisting of Thr, Met, His, and Phe.

In order to enhance the effect, it is preferable to use two or more of these amino acids in combination.

In the present invention, the keratin type II cuticle Hb6 production promoter is preferably one or more members selected from the group consisting of Phe, Tau, and PCA, more preferably Phe.

In order to enhance the effect, it is preferable to use two or more of these amino acids in combination.

In the present invention, the thioredoxin production promoter is preferably one or more members selected from the group consisting of Leu, His, and Phe.

In order to enhance the effect, it is preferable to use two or more of these amino acids in combination.

Also, the present invention relates to a composition for improving decreased barrier function of the skin, containing one or more members selected from the group consisting of Gly, Val, Leu, Met, Phe, Trp, His, Lys, Arg, Thr, Glu, Gln, Pro, Orn, Cit, Tau, UCA, and PCA (preferably, one or more members selected from the group consisting of Val, Leu, Met, Phe, Trp, His, Lys, and Thr), as an active ingredient.

The composition for improving decreased barrier function of the skin can be applied orally or parenterally. Examples of the composition to be applied parenterally include cosmetics and skin external preparations. Examples of the composition to be applied orally include foods (e.g., supplement) and pharmaceutical products for oral administration.

When the composition for improving decreased barrier function of the skin is a composition to be applied parenterally, the content of the active ingredient is, for example, 0.001 - 20 wt%, preferably 0.01 - 10 wt%, more preferably 0.05 - 5 wt%, further preferably 0.1 - 2 wt%.

When the composition for improving decreased barrier function of the skin is a composition to be applied orally, the content of the active ingredient is, for example, 0.01 - 100 wt%, preferably 0.1 - 95 wt%, more preferably 0.5 - 90 wt%, further preferably 2 - 90 wt%.

Examples of the form, additives, method of use, dosage, and the like of the composition for improving decreased barrier function of the skin are the same as the examples of the form, additives, method of use, dosage, and the like explained in the aforementioned "composition for improving decreased barrier function of the skin, containing at least one member selected from the group consisting of (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter, as an active ingredient".

Also, the present invention relates to a composition for promoting production of caspase-14 (particularly, a composition for promoting production of caspase-14 in epidermal keratinocytes), containing one or more members selected from the group consisting of Val, Leu, His, PCA, and Tau (preferably, one or more members selected from the group consisting of Val, Leu, and His) (the aforementioned caspase-14 production promoter), as an active ingredient;
a composition for promoting production of keratin type II cuticle Hb5 (particularly, a composition for promoting production of keratin type II cuticle Hb5 in epidermal keratinocytes), containing one or more members selected from the group consisting of Leu, Phe, Orn, and Arg (preferably, one or more members selected from the group consisting of Leu and Phe) (the aforementioned keratin type II cuticle Hb5 production promoter), as an active ingredient;
a composition for promoting production of keratin type II cytoskeleton 1b (particularly, a composition for promoting production of keratin type II cytoskeleton 1b in epidermal keratinocytes), containing one or more members selected from the group consisting of His, Trp, and Pro (preferably, one or more members selected from the group consisting of His and Trp) (the aforementioned keratin type II cytoskeleton 1b production promoter), as an active ingredient;
a composition for promoting production of phosphatidylethanolamine-binding protein 1 (particularly, a composition for promoting production of phosphatidylethanolamine-binding protein 1 in epidermal keratinocytes), containing one or more members selected from the group consisting of Val, Lys, His, PCA, Tau, and Glu (preferably, one or more members selected from the group consisting of Val, Lys, and His) (the aforementioned phosphatidylethanolamine-binding protein 1 production promoter), as an active ingredient;
a composition for promoting production of hornerin (particularly, a composition for promoting production of hornerin in epidermal keratinocytes), containing one or more members selected from the group consisting of Leu, His, Trp, Gly, Pro, and Cit (preferably, one or more members selected from the group consisting of Leu, His, and Trp) (the aforementioned hornerin production promoter), as an active ingredient;
a composition for promoting production of keratin type I cuticle Ha1 (particularly, a composition for promoting production of keratin type I cuticle Ha1 in epidermal keratinocytes), containing one or more members selected from the group consisting of Met, Tau, Gln, and PCA (preferably, Met) (the aforementioned keratin type I cuticle Ha1 production promoter), as an active ingredient;
a composition for promoting production of keratin type I cuticle Ha3-I (particularly, a composition for promoting production of keratin type I cuticle Ha3-I in epidermal keratinocytes), containing one or more members selected from the group consisting of Met, Tau, and Orn (preferably, Met) (the aforementioned keratin type I cuticle Ha3-I production promoter), as an active ingredient;
a composition for promoting production of keratin type II cuticle Hb3 (particularly, a composition for promoting production of keratin type II cuticle Hb3 in epidermal keratinocytes), containing one or more members selected from the group consisting of Thr, Met, His, Phe, Tau, UCA, and PCA (preferably, one or more members selected from the group consisting of Thr, Met, His, and Phe) (the aforementioned keratin type II cuticle Hb3 production promoter), as an active ingredient;
a composition for promoting production of keratin type II cuticle Hb6 (particularly, a composition for promoting production of keratin type II cuticle Hb6 in epidermal keratinocytes), containing one or more members selected from the group consisting of Phe, Tau, and PCA (preferably, Phe) (the aforementioned keratin type II cuticle Hb6 production promoter), as an active ingredient; and
a composition for promoting production of thioredoxin (particularly, a composition for promoting production of thioredoxin in epidermal keratinocytes), containing one or more members selected from the group consisting of Leu, His, and Phe (the aforementioned thioredoxin production promoter), as an active ingredient.

The composition for promoting production of caspase-14, the composition for promoting production of keratin type II cuticle Hb5, the composition for promoting production of keratin type II cytoskeleton 1b, the composition for promoting production of phosphatidylethanolamine-binding protein 1, the composition for promoting production of hornerin, the composition for promoting production of keratin type I cuticle Ha1, the composition for promoting production of keratin type I cuticle Ha3-I, the composition for promoting production of keratin type II cuticle Hb3, the composition for promoting production of keratin type II cuticle Hb6, and the composition for promoting production of thioredoxin, of the present invention, can be used for the improvement of diseases and conditions (e.g., improvement of decreased barrier function of the skin, etc.) that require promoted production of caspase-14, keratin type II cuticle Hb5, keratin type II cytoskeleton 1b, phosphatidylethanolamine-binding protein 1, hornerin, keratin type I cuticle Ha1, keratin type I cuticle Ha3-I, keratin type II cuticle Hb3, keratin type II cuticle Hb6, and thioredoxin.

Examples of the active ingredient content, form, additives, method of use, dosage, ratio of amino acid as active ingredient, and the like of the composition for promoting production of caspase-14, the composition for promoting production of keratin type II cuticle Hb5, the composition for promoting production of keratin type II cytoskeleton 1b, the composition for promoting production of phosphatidylethanolamine-binding protein 1, the composition for promoting production of hornerin, the composition for promoting production of keratin type I cuticle Ha1, the composition for promoting production of keratin type I cuticle Ha3-I, the composition for promoting production of keratin type II cuticle Hb3, the composition for promoting production of keratin type II cuticle Hb6, and the composition for promoting production of thioredoxin, of the present invention, are the same as the examples of the active ingredient content, form, additives, method of use, dosage, ratio of amino acid as active ingredient, and the like explained in the aforementioned "composition for improving decreased barrier function of the skin, containing at least one member selected from the group consisting of (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter, as an active ingredient".

The composition for improving decreased barrier function of the skin of the present invention can be applied orally or parenterally. Examples of the composition to be applied parenterally include cosmetics and skin external preparations. Examples of the composition to be applied orally include foods (e.g., supplement) and pharmaceutical products for oral administration.

When the composition for improving decreased barrier function of the skin of the present invention is a composition to be applied parenterally, the content of the active ingredient (at least one member selected from the group consisting of (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter) in the composition for improving barrier function of the skin of the present invention is, for example, 0.001 - 20 wt%, preferably 0.01 - 10 wt%, more preferably 0.05 - 5 wt%, further preferably 0.1 - 2 wt%.

When the composition for improving decreased barrier function of the skin of the present invention is a composition to be applied orally, the content of the active ingredient (at least one member selected from the group consisting of (1) caspase-14 production promoter, (2) keratin type II cuticle Hb5 production promoter, (3) keratin type II cytoskeleton 1b production promoter, (4) phosphatidylethanolamine-binding protein 1 production promoter, (5) hornerin production promoter, (6) keratin type I cuticle Ha1 production promoter, (7) keratin type I cuticle Ha3-I production promoter, (8) keratin type II cuticle Hb3 production promoter, (9) keratin type II cuticle Hb6 production promoter, and (10) thioredoxin production promoter) in the composition for improving barrier function of the skin of the present invention is, for example, 0.01 - 100 wt%, preferably 0.1 - 95 wt%, more preferably 0.5 - 90 wt%, further preferably 2 - 90 wt%.

### (D) Improvement of abnormal pH value of the skin surface

The composition for improving an abnormal pH value of the skin surface of the present invention contains at least one member selected from the group consisting of (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter, as an active ingredient.

The (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter are respectively particularly useful for promoting production, in epidermal keratinocytes, of 78 kDa glucose regulated protein, actin cytoplasmic 2, α-actin-4, apolipoprotein D, desmoglein-1, epiplakin, filaggrin, heat shock protein β-1, interleukin-36γ, protein S100-P, α-enolase, keratin type II cuticle Hb3, keratin type II cytoskeleton 75, protein-glutamine γ-glutamyltransferase K, serpin B3, and triose phosphoric acid isomerase.

Among them, (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, and (10) protein S100-P production promoter are preferably used to improve abnormal pH value in young people (e.g., 39 years old or younger), and (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter are preferably used to improve abnormal pH value in older people (e.g., 40 years old or older).

In the present invention, the "improvement of abnormal pH value of the skin surface" refers to improvement of the inherent function of the skin to maintain the pH of the skin surface at a weak acidic level (5 to 7). "Abnormal pH value of the skin surface" means, for example, that the pH of the skin surface becomes neutral or alkaline from weak acidic (pH increase).

In the present invention, pH can be measured, for example, in accordance with the below-mentioned Examples and using a combination of a glass electrode, Skin-pH-Meter PH905, and Cutometer DUAL MPA580 (both Courage+Khazaka).

In the present invention, the 78 kDa glucose regulated protein production promoter is preferably Orn.

In the present invention, the actin cytoplasmic 2 production promoter is preferably one or more members selected from the group consisting of Asn and Orn.

In order to enhance the effect, it is preferable to use two or more of these amino acids in combination.

In the present invention, the α-actin-4 production promoter is preferably one or more members selected from the group consisting of Leu, Lys, Phe, UCA, and Glu, more preferably one or more members selected from the group consisting of Leu, Lys, and Phe.

In order to enhance the effect, it is preferable to use two or more of these amino acids in combination.

In the present invention, the apolipoprotein D production promoter is preferably one or more members selected from the group consisting of Ile, Lys, Phe, Asp, and Arg, more preferably one or more members selected from the group consisting of Ile, Lys, and Phe.

In order to enhance the effect, it is preferable to use two or more of these amino acids in combination.

In the present invention, the desmoglein-1 production promoter is preferably one or more members selected from the group consisting of Leu, Met, Phe, Trp, PCA, and Asn, more preferably one or more members selected from the group consisting of Leu, Met, Phe, and Trp.

In order to enhance the effect, it is preferable to use two or more of these amino acids in combination. In the present invention, the desmoglein-1 production promoter is particularly preferably a combination of Leu, Met, Phe, and Trp.

In a preferred embodiment, the composition for improving an abnormal pH value of the skin surface of the present invention (e.g., skin lotion, milky lotion and cream) contains Leu, Met, Phe, and Trp, and is used at concentrations of Leu 0.00043 - 8.55 wt%, Met 0.00022 - 4.46 wt%, Phe 0.00028 - 5.61 wt%, and Trp 0.00007 - 1.39 wt%, in the composition for improving.

In the present invention, when the desmoglein-1 production promoter is a combination of Phe, Trp, Met, and Leu, the weight ratio of Phe, Trp, Met, and Leu (Phe:Trp:Met:Leu) is preferably 1:0.2 - 2.8:0.4 - 1.1:0.1 - 1.6, more preferably 1:0.2 - 0.3:0.4 - 0.8:1.5 - 1.6, further preferably 1:0.2 - 0.3:0.4 - 0.5:1.5 - 1.6.

In the present invention, the desmoglein-1 production promoter is particularly preferably a combination of Leu, Phe, and Trp.

In a preferred embodiment, the composition for improving an abnormal pH value of the skin surface of the present invention (e.g., skin lotion, milky lotion and cream) contains Leu, Phe, and Trp, and is used at concentrations of Leu 0.000029 - 0.575 wt%, Phe 0.00043 - 8.69 wt%, and Trp 0.00054 - 10.74 wt%, in the composition for improving.

In the present invention, when the desmoglein-1 production promoter is a combination of Phe, Trp, and Leu, the weight ratio of Phe, Trp, and Leu (Phe:Trp:Leu) is preferably 1:0.05 - 1.3:0.05 - 0.8, more preferably 1:1.2 - 1.3:0.05 - 0.8, further preferably 1:1.2 - 1.3:0.05 - 0.1.

In the present invention, the epiplakin production promoter is preferably one or more members selected from the group consisting of Thr, Met, PCA, and Arg, more preferably one or more members selected from the group consisting of Thr and Met.

In order to enhance the effect, it is preferable to use two or more of these amino acids in combination.

In the present invention, the filaggrin production promoter is preferably one or more members selected from the group consisting of Val and Ile.

In order to enhance the effect, it is preferable to use two or more of these amino acids in combination.

In the present invention, the heat shock protein β-1 production promoter is preferably Orn.

In the present invention, the interleukin-36γ production promoter is preferably one or more members selected from the group consisting of Ile, Leu, His, Phe, and Cit, more preferably one or more members selected from the group consisting of Ile, Leu, His, and Phe.

In order to enhance the effect, it is preferable to use two or more of these amino acids in combination.

In the present invention, the protein S100-P production promoter is preferably one or more members selected from the group consisting of Leu, Phe, Trp, Pro, and Glu, more preferably one or more members selected from the group consisting of Leu, Phe, and Trp.

In order to enhance the effect, it is preferable to use two or more of these amino acids in combination.

In the present invention, the α-enolase production promoter is preferably one or more members selected from the group consisting of Thr, Leu, Trp, Gln, and Glu, more preferably one or more members selected from the group consisting of Thr, Leu, and Trp.

In order to enhance the effect, it is preferable to use two or more of these amino acids in combination.

In the present invention, the keratin type II cuticle Hb3 production promoter is preferably one or more members selected from the group consisting of Thr, Met, His, Phe, Tau, UCA, and PCA, more preferably one or more members selected from the group consisting of Thr, Met, His, and Phe.

In order to enhance the effect, it is preferable to use two or more of these amino acids in combination.

In the present invention, the keratin type II cytoskeleton 75 production promoter is preferably one or more members selected from the group consisting of Leu, His, Trp, and Tau, more preferably one or more members selected from the group consisting of Leu, His, and Trp.

In order to enhance the effect, it is preferable to use two or more of these amino acids in combination.

In the present invention, the protein-glutamine γ-glutamyltransferase K production promoter is preferably one or more members selected from the group consisting of Val, Thr, Ile, His, Ser, and Orn, more preferably one or more members selected from the group consisting of Val, Thr, Ile, and His.

In order to enhance the effect, it is preferable to use two or more of these amino acids in combination.

In the present invention, the serpin B3 production promoter is preferably one or more members selected from the group consisting of Val, Leu, Asn, and Gln, more preferably one or more members selected from the group consisting of Val and Leu.

In order to enhance the effect, it is preferable to use two or more of these amino acids in combination.

In the present invention, the triose phosphoric acid isomerase production promoter is preferably one or more members selected from the group consisting of Thr, Leu, Asn, and Gln, more preferably one or more members selected from the group consisting of Thr and Leu.

In order to enhance the effect, it is preferable to use two or more of these amino acids in combination.

The present invention also relates to a composition for improving an abnormal pH value of the skin surface, containing one or more members selected from the group consisting of Val, Leu, Ile, Met, Phe, Trp, His, Lys, Arg, Ser, Thr, Asp, Glu, Asn, Gln, Pro, Orn, Cit, Tau, UCA, and PCA (one or more members selected from the group consisting of Val, Leu, Ile, Met, Phe, Trp, His, Lys, and Thr), as an active ingredient.

The composition for improving an abnormal pH value of the skin surface can be applied orally or parenterally. Examples of the composition to be applied parenterally include cosmetics and skin external preparations. Examples of the composition to be applied orally include foods (e.g., supplement) and pharmaceutical products for oral administration.

When the composition for improving an abnormal pH value of the skin surface is a composition to be applied parenterally, the content of the active ingredient is, for example, 0.001 - 20 wt%, preferably 0.01 - 10 wt%, more preferably 0.05 - 5 wt%, further preferably 0.1 - 2 wt%.

When the composition for improving an abnormal pH value of the skin surface is a composition to be applied orally, the content of the active ingredient is, for example, 0.01 - 100 wt%, preferably 0.1 - 95 wt%, more preferably 0.5 - 90 wt%, further preferably 2 - 90 wt%.

Examples of the form, additives, method of use, dosage, and the like of the composition for improving an abnormal pH value of the skin surface are the same as the examples of the form, additives, method of use, dosage, and the like explained in the aforementioned "composition for improving a decreased moisture content of the stratum corneum containing at least one member selected from the group consisting of (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter, as an active ingredient".

Also, the present invention relates to a composition for promoting production of 78 kDa glucose regulated protein (particularly, a composition for promoting production of 78 kDa glucose regulated protein in epidermal keratinocytes), containing Orn (the aforementioned 78 kDa glucose regulated protein production promoter), as an active ingredient;
a composition for promoting production of actin cytoplasmic 2 (particularly, a composition for promoting production of actin cytoplasmic 2 in epidermal keratinocytes), containing one or more members selected from the group consisting of Asn and Orn (the aforementioned actin cytoplasmic 2 production promoter), as an active ingredient;
a composition for promoting production of α-actin-4 (particularly, a composition for promoting production of α-actin-4 in epidermal keratinocytes), containing one or more members selected from the group consisting of Leu, Lys, Phe, UCA, and Glu (preferably, one or more members selected from the group consisting of Leu, Lys, and Phe) (the aforementioned α-actin-4 production promoter), as an active ingredient;
a composition for promoting production of apolipoprotein D (particularly, a composition for promoting production of apolipoprotein D in epidermal keratinocytes), containing one or more members selected from the group consisting of Ile, Lys, Phe, Asp, and Arg (preferably, one or more members selected from the group consisting of Ile, Lys, and Phe) (the aforementioned apolipoprotein D production promoter), as an active ingredient;
a composition for promoting production of desmoglein-1 (particularly, a composition for promoting production of desmoglein-1 in epidermal keratinocytes), containing one or more members selected from the group consisting of Leu, Met, Phe, Trp, PCA, and Asn (preferably, one or more members selected from the group consisting of Leu, Met, Phe, and Trp, more preferably, a combination of Leu, Met, Phe, and Trp, or a combination of Leu, Phe, and Trp) (the aforementioned desmoglein-1 production promoter), as an active ingredient;
a composition for promoting production of epiplakin (particularly, a composition for promoting production of epiplakin in epidermal keratinocytes), containing one or more members selected from the group consisting of Thr, Met, PCA, and Arg (preferably, one or more members selected from the group consisting of Thr and Met) (the aforementioned epiplakin production promoter), as an active ingredient;
a composition for promoting production of filaggrin (particularly, a composition for promoting production of filaggrin in epidermal keratinocytes), containing one or more members selected from the group consisting of Val and Ile (the aforementioned filaggrin production promoter), as an active ingredient;
a composition for promoting production of heat shock protein β-1 (particularly, a composition for promoting production of heat shock protein β-1 in epidermal keratinocytes), containing Orn (the aforementioned heat shock protein β-1 production promoter), as an active ingredient;
a composition for promoting production of interleukin-36γ (particularly, a composition for promoting production of interleukin-36γ in epidermal keratinocytes), containing one or more members selected from the group consisting of Ile, Leu, His, Phe, and Cit (preferably, one or more members selected from the group consisting of Ile, Leu, His, and Phe) (the aforementioned interleukin-36γ production promoter), as an active ingredient;
a composition for promoting production of protein S100-P (particularly, a composition for promoting production of protein S100-P in epidermal keratinocytes), containing one or more members selected from the group consisting of Leu, Phe, Trp, Pro, and Glu (preferably, one or more members selected from the group consisting of Leu, Phe, and Trp) (the aforementioned protein S100-P production promoter), as an active ingredient;
a composition for promoting production of α-enolase (particularly, a composition for promoting production of α-enolase in epidermal keratinocytes), containing one or more members selected from the group consisting of Thr, Leu, Trp, Gln, and Glu (preferably, one or more members selected from the group consisting of Thr, Leu, and Trp) (the aforementioned α-enolase production promoter), as an active ingredient;
a composition for promoting production of keratin type II cuticle Hb3 (particularly, a composition for promoting production of keratin type II cuticle Hb3 in epidermal keratinocytes), containing one or more members selected from the group consisting of Thr, Met, His, Phe, Tau, UCA, and PCA (preferably, one or more members selected from the group consisting of Thr, Met, His, and Phe) (the aforementioned keratin type II cuticle Hb3 production promoter), as an active ingredient;
a composition for promoting production of keratin type II cytoskeleton 75 (particularly, a composition for promoting production of keratin type II cytoskeleton 75 in epidermal keratinocytes), containing one or more members selected from the group consisting of Leu, His, Trp, and Tau (preferably, one or more members selected from the group consisting of Leu, His, and Trp) (the aforementioned keratin type II cytoskeleton 75 production promoter), as an active ingredient;
a composition for promoting production of protein-glutamine γ-glutamyltransferase K (particularly, a composition for promoting production of protein-glutamine γ-glutamyltransferase K in epidermal keratinocytes), containing one or more members selected from the group consisting of Val, Thr, Ile, His, Ser, and Orn (preferably, one or more members selected from the group consisting of Val, Thr, Ile, and His) (the aforementioned protein-glutamine γ-glutamyltransferase K production promoter), as an active ingredient;
a composition for promoting production of serpin B3 (particularly, a composition for promoting production of serpin B3 in epidermal keratinocytes), containing one or more members selected from the group consisting of Val, Leu, Asn, and Gln (preferably, one or more members selected from the group consisting of Val and Leu) (the aforementioned serpin B3 production promoter), as an active ingredient; and a composition for promoting production of triose phosphoric acid isomerase (particularly, a composition for promoting production of triose phosphoric acid isomerase in epidermal keratinocytes), containing one or more members selected from the group consisting of Thr, Leu, Asn, and Gln (preferably, one or more members selected from the group consisting of Thr and Leu) (the aforementioned triose phosphoric acid isomerase production promoter), as an active ingredient.

The composition for promoting production of 78 kDa glucose regulated protein, the composition for promoting production of actin cytoplasmic 2, the composition for promoting production of α-actin-4, the composition for promoting production of apolipoprotein D, the composition for promoting production of desmoglein-1, the composition for promoting production of epiplakin, the composition for promoting production of filaggrin, the composition for promoting production of heat shock protein β-1, the composition for promoting production of interleukin-36γ, the composition for promoting production of protein S100-P, the composition for promoting production of α-enolase, the composition for promoting production of keratin type II cuticle Hb3, the composition for promoting production of keratin type II cytoskeleton 75, the composition for promoting production of protein-glutamine γ-glutamyltransferase K, the composition for promoting production of serpin B3, and the composition for promoting production of triose phosphoric acid isomerase, of the present invention, can be used for the improvement of diseases and conditions (e.g., improvement of abnormal pH value of the skin surface, etc.) that require promoted production of 78 kDa glucose regulated protein, actin cytoplasmic 2, α-actin-4, apolipoprotein D, desmoglein-1, epiplakin, filaggrin, heat shock protein β-1, interleukin-36γ, protein S100-P, α-enolase, keratin type II cuticle Hb3, keratin type II cytoskeleton 75, protein-glutamine γ-glutamyltransferase K, serpin B3, and triose phosphoric acid isomerase.

Examples of the active ingredient content, form, additives, method of use, dosage, ratio of amino acid as active ingredient, and the like of the composition for promoting production of 78 kDa glucose regulated protein, the composition for promoting production of actin cytoplasmic 2, the composition for promoting production of α-actin-4, the composition for promoting production of apolipoprotein D, the composition for promoting production of desmoglein-1, the composition for promoting production of epiplakin, the composition for promoting production of filaggrin, the composition for promoting production of heat shock protein β-1, the composition for promoting production of interleukin-36γ, the composition for promoting production of protein S100-P, the composition for promoting production of α-enolase, the composition for promoting production of keratin type II cuticle Hb3, the composition for promoting production of keratin type II cytoskeleton 75, the composition for promoting production of protein-glutamine γ-glutamyltransferase K, the composition for promoting production of serpin B3, and the composition for promoting production of triose phosphoric acid isomerase, of the present invention, are the same as the examples of the active ingredient content, form, additives, method of use, dosage, ratio of amino acid as active ingredient, and the like explained in the aforementioned "composition for improving an abnormal pH value of the skin surface, containing at least one member selected from the group consisting of (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter, as an active ingredient".

The composition for improving an abnormal pH value of the skin surface of the present invention can be applied orally or parenterally. Examples of the composition to be applied parenterally include cosmetics and skin external preparations. Examples of the composition to be applied orally include foods (e.g., supplement) and pharmaceutical products for oral administration.

When the composition for improving an abnormal pH value of the skin surface of the present invention is a composition to be applied parenterally, the content of the active ingredient (at least one member selected from the group consisting of (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter) in the composition for improving an abnormal pH value of the skin surface of the present invention is, for example, 0.001 - 20 wt%, preferably 0.01 - 10 wt%, more preferably 0.05 - 5 wt%, further preferably 0.1 - 2 wt%.

When the composition for improving an abnormal pH value of the skin surface of the present invention is a composition to be applied orally, the content of the active ingredient (at least one member selected from the group consisting of (1) 78 kDa glucose regulated protein production promoter, (2) actin cytoplasmic 2 production promoter, (3) α-actin-4 production promoter, (4) apolipoprotein D production promoter, (5) desmoglein-1 production promoter, (6) epiplakin production promoter, (7) filaggrin production promoter, (8) heat shock protein β-1 production promoter, (9) interleukin-36γ production promoter, (10) protein S100-P production promoter, (11) α-enolase production promoter, (12) keratin type II cuticle Hb3 production promoter, (13) keratin type II cytoskeleton 75 production promoter, (14) protein-glutamine γ-glutamyltransferase K production promoter, (15) serpin B3 production promoter, and (16) triose phosphoric acid isomerase production promoter) in the composition for improving an abnormal pH value of the skin surface of the present invention is, for example, 0.01 - 100 wt%, preferably 0.1 - 95 wt%, more preferably 0.5 - 90 wt%, further preferably 2 - 90 wt%.

The composition for improving a decreased moisture content of the stratum corneum, the composition for improving skin spots, the composition for improving decreased barrier function of the skin, and the composition for improving an abnormal pH value of the skin surface, of the present invention, are not particularly limited in form, and can take any form, such as liquid, paste, gel, solid, powder, and the like.

Specifically, when used as cosmetics, for example, skin lotion, toner, cream, milky lotion, beauty serum, enamel, foundation, eyeliner, eyebrow pencil, mascara, eye shadow, blush, lipstick, face powder, powder, sun protectant, facial mask, lip cream, makeup base, body toner, body cream, massage cream, and skin cleanser can be mentioned.

The composition for improving a decreased moisture content of the stratum corneum, the composition for improving skin spots, the composition for improving decreased barrier function of the skin, and the composition for improving an abnormal pH value of the skin surface, of the present invention, can be formulated by known methods into cosmetics, skin external preparations, foods (supplements, etc.), pharmaceutical products, and the like by blending various additives that can be generally used in cosmetics, skin external preparations, foods, and pharmaceutical products, in addition to the active ingredients of the present invention described above, within a range that does not inhibit the effects of the present invention.

When used as cosmetics or skin external preparations, examples of the additive include oily component, surfactant, lower alcohol, higher alcohol, polyhydric alcohol, sugar alcohol and alkylene oxide adduct thereof, water-soluble polymer, gelling agent, humectant, bactericidal agent and antibacterial agent, anti-inflammatory agent, analgesic, antifungal agent, keratin softening and peeling agent, skin colorant, hormonal agent, UV absorber, UV scattering agent, hair-growth drug, antiperspirant and astringent active ingredient, perspiration deodorant, vitamin, vasodilator, herbal medicine, pH adjuster, metal ion sequestrant, viscosity modifier, pearlizing agent, natural flavor, synthetic flavor, dye, pigment, antioxidant, antiseptic, emulsifier, fat and wax, silicone compound, balm, and the like.

When used as food or pharmaceutical product for oral administration, examples of additive include starch, dextrin, cyclodextrin, sugars, sugar alcohol, protein, peptide, inorganic salts, organic acids and salts thereof, solid fat, silicon dioxide, yeast cell, various powder extracts, water, excipient, pH adjuster, antioxidant, thickening stabilizer, sweetener, acidulant, spice, colorant, and the like.

In the present specification, the "food" is a concept including foods with health claims, foods for specified health use, foods with nutritional function, supplement (dietary supplement), nutritional supplementary food, health supplement product, foods for medical use, medical food, and the like.

When applied parenterally, the composition for improving a decreased moisture content of the stratum corneum, the composition for improving skin spots, the composition for improving decreased barrier function of the skin, and the composition for improving an abnormal pH value of the skin surface, of the present invention, can be used by applying to the skin once to several times a day.

When applied orally, the composition for improving a decreased moisture content of the stratum corneum, the composition for improving skin spots, the composition for improving decreased barrier function of the skin, and the composition for improving an abnormal pH value of the skin surface, of the present invention, about 0.05 to 15 g as the active ingredient can be ingested or administered once or in several divided doses per day.

In a preferred embodiment, the composition for promoting production of desmoglein-1 of the present invention contains Leu, Met, Phe, and Trp (preferably contains only Leu, Met, Phe, and Trp as essential amino acids), and the wt% concentration of Leu is preferably 0.00043 - 8.55 wt%, more preferably 0.0043 - 4.27 wt%, further preferably 0.0214 - 2.14 wt%, further more preferably 0.0427 - 0.85 wt%, the wt% concentration of Met is preferably 0.00022 - 4.46 wt%, more preferably 0.0022 - 2.23 wt%, further preferably 0.0111 - 1.11 wt%, further more preferably 0.0223 - 0.45 wt%, the wt% concentration of Phe is preferably 0.00028 - 5.61 wt%, more preferably 0.0028 - 2.80%, further preferably 0.0140 - 1.40 wt%, further more preferably 0.0280 - 0.56 wt%, and the wt% concentration of Trp is preferably 0.00007 - 1.39 wt%, more preferably 0.0007 - 0.69 wt%, further preferably 0.0035 - 0.35 wt%, further more preferably 0.0069 - 0.14 wt%.

In a preferred embodiment, the composition for promoting production of desmoglein-1 of the present invention contains Leu, Met, Phe, and Trp (preferably contains only Leu, Met, Phe, and Trp as essential amino acids), and the molar concentration of Leu is preferably 17% or more, more preferably 20% or more, further preferably 25% or more, further more preferably 30% or more, 35% or more, 40% or more, 45% or more, 50% or more, based on the total molar concentration of essential amino acids in the composition, the molar concentration of Met is preferably 5% or more, more preferably 6% or more, further preferably 7% or more, further more preferably 8% or more, 9% or more, 10% or more, 11% or more, 12% or more, 13% or more, based on the total molar concentration of essential amino acids in the composition, the molar concentration of Phe is preferably 9% or more, more preferably 10% or more, further preferably 13% or more, further more preferably 15% or more, 18% or more, 20% or more, 25% or more, based on the total molar concentration of essential amino acids in the composition, and the molar concentration of Trp is preferably 2% or more, more preferably 3% or more, further preferably 4% or more, further more preferably 5% or more, based on the total molar concentration of essential amino acids in the composition.

In a preferred embodiment, the composition for promoting production of desmoglein-1 of the present invention contains Leu, Met, Phe, and Trp (preferably contains only Leu, Met, Phe, and Trp as essential amino acids), and the molar concentration of Leu is preferably 17 - 84% based on the total molar concentration of essential amino acids in the composition, the molar concentration of Met is preferably 5 - 72% based on the total molar concentration of essential amino acids in the composition, the molar concentration of Phe is preferably 9 - 76% based on the total molar concentration of essential amino acids in the composition, and the molar concentration of Trp is preferably 2 - 69% based on the total molar concentration of essential amino acids in the composition.

In a preferred embodiment, the composition for promoting production of desmoglein-1 of the present invention (e.g., skin lotion, milky lotion and cream) contains Leu, Met, Phe, and Trp, and is used at concentrations of Leu 0.00043 - 8.55 wt%, Met 0.00022 - 4.46 wt%, Phe 0.00028 - 5.61 wt%, and Trp 0.00007 - 1.39 wt%, in the composition for improving.

In a preferred embodiment, the composition for promoting production of desmoglein-1 of the present invention contains Leu, Phe, and Trp (preferably contains only Leu, Phe and Trp as essential amino acids), the wt% concentration of Leu is preferably 0.000029 - 0.575 wt%, more preferably 0.00029 - 0.287 wt%, further preferably 0.00144 - 0.144 wt%, further more preferably 0.00287 - 0.0575 wt%, the wt% concentration of Phe is preferably 0.00043 - 8.69 wt%, more preferably 0.00434 - 4.34 wt%, further preferably 0.0217 - 2.17 wt%, further more preferably 0.0434 - 0.869 wt%, and the wt% concentration of Trp is preferably 0.000537 - 10.74 wt%, more preferably 0.00537 - 5.37 wt%, further preferably 0.0268 - 2.68 wt%, further more preferably 0.0537 - 1.074 wt%.

In a preferred embodiment, the composition for promoting production of desmoglein-1 of the present invention contains Leu, Phe, and Trp (preferably contains only Leu, Phe, and Trp as essential amino acids), and the molar concentration of Leu is preferably 3% or more, more preferably 4% or more, further preferably 5% or more, further more preferably 10% or more, based on the total molar concentration of essential amino acids in the composition, the molar concentration of Phe is preferably 45% or more, more preferably 48% or more, further preferably 50% or more, further more preferably 55% or more, based on the total molar concentration of essential amino acids in the composition, and the molar concentration of Trp is preferably 45% or more, more preferably 48% or more, further preferably 50% or more, further more preferably 55% or more, based on the total molar concentration of essential amino acids in the composition.

In a preferred embodiment, the composition for promoting production of desmoglein-1 of the present invention contains Leu, Phe, and Trp (preferably contains only Leu, Phe, and Trp as essential amino acids), and the molar concentration of Leu is preferably 3 - 50% based on the total molar concentration of essential amino acids in the composition, the molar concentration of Phe is preferably 30 - 50% based on the total molar concentration of essential amino acids in the composition, and the molar concentration of Trp is preferably 2 - 50% based on the total molar concentration of essential amino acids in the composition.

In a preferred embodiment, the composition for promoting production of desmoglein-1 (e.g., skin lotion, milky lotion and cream) of the present invention contains Leu, Phe, and Trp, and is used at concentrations of Leu 0.000029 - 0.575 wt%, Phe 0.00043 - 8.69 wt%, and Trp 0.00054 - 10.74 wt%, in the composition for improving.

In a preferred embodiment, the composition for promoting production of protein-glutamine γ-glutamyltransferase E of the present invention contains Trp and Val (preferably contains only Trp and Val as essential amino acids), and the wt% concentration of Trp is preferably 0.00033 - 6.59 wt%, more preferably 0.0033 - 3.30 wt%, further preferably 0.0165 - 1.65%, further more preferably 0.0330 - 0.66 wt%, and the wt% concentration of Val is preferably 0.00067 - 13.41 wt%, more preferably 0.0067 - 6.70 wt%, further preferably 0.0335 - 3.35 wt%, further more preferably 0.0670 - 1.34 wt%.

In a preferred embodiment, the composition for promoting production of protein-glutamine γ-glutamyltransferase E of the present invention contains Trp and Val (preferably contains only Trp and Val as essential amino acids), and the molar concentration of Trp is preferably 2% or more, more preferably 3% or more, further preferably 4% or more, further more preferably 5% or more, 6% or more, 7% or more, 8% or more, 9% or more, based on the total molar concentration of essential amino acids in the composition, and the molar concentration of Val is preferably 17% or more, more preferably 25% or more, further preferably 30% or more, further more preferably 40% or more, 50% or more, 60% or more, 70% or more, 80% or more, 90% or more, based on the total molar concentration of essential amino acids in the composition.

In a preferred embodiment, the composition for promoting production of protein-glutamine γ-glutamyltransferase E of the present invention contains Trp and Val (preferably contains only Trp and Val as essential amino acids), and the molar concentration of Trp is preferably 2 - 83% based on the total molar concentration of essential amino acids in the composition, and the molar concentration of Val is preferably 17 - 98% based on the total molar concentration of essential amino acids in the composition.

In a preferred embodiment, the composition for promoting production of protein-glutamine γ-glutamyltransferase E (e.g., skin lotion, milky lotion and cream) of the present invention contains Val and Trp, and is used at concentrations of Val 0.00067 - 13.41 wt% and Trp 0.00033 - 6.59 wt%, in the composition for improving.

In a preferred embodiment, the composition for promoting production of caspase-14 of the present invention contains Val, Leu, and His(preferably contains only Val, Leu, and His as essential amino acids), and the wt% concentration of Val is preferably 0.00032 - 6.35%, more preferably 0.0032 - 3.17 wt%, further preferably 0.0159 - 1.59 wt%, further more preferably 0.0317 - 0.63 wt%, the wt% concentration of Leu is preferably 0.00054 - 10.77 wt%, more preferably 0.0054 - 5.38 wt%, further preferably 0.0269 - 2.69 wt%, further more preferably 0.0538 - 1.08 wt%, and the wt% concentration of His is preferably 0.00014 - 2.88 wt%, more preferably 0.0014 - 1.44 wt%, further preferably 0.0072 - 0.72 wt%, further more preferably 0.0144 - 0.29 wt%.

In a preferred embodiment, the composition for promoting production of caspase-14 of the present invention contains Val, Leu, and His (preferably contains only Val, Leu, and His as essential amino acids), and the molar concentration of Val is preferably 17% or more, more preferably 20% or more, further preferably 25% or more, further more preferably 30% or more, 35% or more, 40% or more, based on the total molar concentration of essential amino acids in the composition, the molar concentration of Leu is preferably 17% or more, more preferably 20% or more, further preferably 25% or more, further more preferably 30% or more, 35% or more, 40% or more, based on the total molar concentration of essential amino acids in the composition, and the molar concentration of His is preferably 5% or more, more preferably 6% or more, further preferably 7% or more, further more preferably 8% or more, 9% or more, 10% or more, 11% or more, based on the total molar concentration of essential amino acids in the composition.

In a preferred embodiment, the composition for promoting production of caspase-14 of the present invention contains Val, Leu, and His (preferably contains only Val, Leu, and His as essential amino acids), and the molar concentration of Val is preferably 17 - 78% based on the total molar concentration of essential amino acids in the composition, the molar concentration of Leu is preferably 17 - 78% based on the total molar concentration of essential amino acids in the composition, and the molar concentration of His is preferably 5 - 66% based on the total molar concentration of essential amino acids in the composition.

In a preferred embodiment, the composition for promoting production of caspase-14 (e.g., skin lotion, milky lotion and cream) of the present invention contains Val, Leu, and His, and is used at concentrations of Val 0.00032 - 6.35 wt%, Leu 0.00054 - 10.77 wt%, and His 0.00014 - 2.88 wt%, in the production-promoting composition.

In a preferred embodiment, the composition for promoting production of caspase-14 of the present invention contains His (preferably contains only His as essential amino acid), and the wt% concentration of His is preferably 0.001 - 20 wt%, more preferably 0.01 - 10 wt%, further preferably 0.05 - 5 wt%, further more preferably 0.1 - 2 wt%.

In a preferred embodiment, the composition for promoting production of caspase-14 of the present invention contains His (preferably contains only His as essential amino acid), and the molar concentration of His is preferably 5% or more, more preferably 10% or more, further preferably 20% or more, further more preferably 30% or more, 40% or more, 50% or more, 60% or more, 70% or more, 80% or more 90% or more, particularly preferably 100%, based on the total molar concentration of essential amino acids in the composition.

In a preferred embodiment, the composition for promoting production of caspase-14 of the present invention contains His (preferably contains only His as essential amino acid), and the molar concentration of His is preferably 5 - 100% based on the total molar concentration of essential amino acids in the composition.

In a preferred embodiment, the composition for promoting production of caspase-14 (e.g., skin lotion, milky lotion and cream) of the present invention contains His, and is used at a concentration of His 0.001 - 20 wt% in the production-promoting composition.

The present invention is described in detail below with reference to Examples; however, the present invention is not limited by the Examples.

In the present specification, the unit "mM" means "mmol/L".

### [Example]

### [Experimental Example 1]

### (Measurement of moisture content of stratum corneum, melanin content, transepidermal water transpiration amount (barrier function), and pH)

Test subjects (all female, age distribution: 30 people in 20s-30s, 35 people in 40s-60s: total 65 people) washed their faces and then acclimatized for 20 min in a constant temperature and humidity room (humidity 40%, room temperature 20°C) .

After acclimation, the moisture content of stratum corneum (SKICON-200EX, Yayoi Co., Ltd.), melanin content (spectrophotometric system (CM-700d), skin analysis software (CM-SA), Konica Minolta Japan, Inc.), transepidermal water transpiration amount (Tewameter TM300, Cutometer DUAL MPA580, Courage+Khazaka), pH (pH meter (LAQUA F-71), and flat pH composite electrode (6261-10C), Horiba, Ltd.) were measured.

### (Stratum corneum collection by tape stripping method)

After completion of the above-mentioned measurements, a tape cut into 3 cm x 3 cm was applied to the center of the test subject's cheek to collect the stratum corneum. The outermost layer of the stratum corneum collected the first time was not used for analysis, and the stratum corneum collected the second or third time was used for analysis.

### (Extraction of L-amino acid)

The collected tape was placed in a conical tube (50 ml), sealed, and stored at -80°C. The collected tape (stratum corneum) was subjected to ultrasonication twice using 95% methyl alcohol aqueous solution to extract the water-soluble components. An internal standard solution (containing multiple types of L-amino acid stable isotopes) was added to the extract. The obtained methyl alcohol aqueous solution was dried using a centrifugal evaporator and used as a sample for L-amino acid analysis.

### (Quantitative analysis of L-amino acid)

50 µL of water was added to the concentrated and dried sample, and the mixture was redissolved by stirring using a vortex mixer. 10 µL of the redissolved sample was mixed with 10 µL of acetonitrile, and the supernatant was centrifuged and used as a sample solution. 30 µL of APDSTAG Wako Borate Buffer was added to 10 µL of the sample solution and stirred, 10 µL of APDS reagent was added and the mixture was reacted at 55°C for 10 min. 200 µL of 0.1% formic acid/APDS Tagwako eluent (1/3, v/v) was added to the reaction solution and used as an analysis sample. LC/MS was used for the analysis which was performed under the following conditions.

### (LC/MS conditions)

An Agilent 1290 Infinity LC system (Agilent) and an API 4000 (Sciex) system were used for the LC/MS analysis. For LC, a guard column Inertsil C8-3 (inner diameter 2.1 mm, length 10 mm) and an analysis column Inertsil C8-3 (inner diameter 2.1 mm, length 100 mm) were used for separation. APDSTAG Wako Eluent was used as mobile phase A, and 60% acetonitrile solution was used as mobile phase B, and stepwise elution was performed at a flow rate of 0.3 mL/min. MS data was collected by MRM measurement in positive mode by an electrospray ionization method.

Transitions are shown in Tables 1-1 and 1-2.

**[Table 1-1]**

| amino acid | m/z (Q1) | m/z (Q3) | Dwell time (msec) | DP (V) | EP (V) | CE (V) | CXP (V) |
|---|---|---|---|---|---|---|---|
| EtOH NH2 | 182 | 121.1 | 21 | 71 | 10 | 29 | 10 |
| Gly | 196 | 121.1 | 21 | 71 | 10 | 29 | 10 |
| Sar | 210 | 121.1 | 21 | 71 | 10 | 29 | 10 |
| Ala | 210.1 | 121.1 | 21 | 71 | 10 | 29 | 10 |
| GABA | 224.2 | 121.1 | 21 | 71 | 10 | 29 | 10 |
| bAiBA | 224.1 | 121.1 | 21 | 71 | 10 | 29 | 10 |
| aABA | 224 | 121.1 | 21 | 71 | 10 | 29 | 10 |
| Ser | 226 | 121.1 | 21 | 71 | 10 | 29 | 10 |
| Pro | 236 | 121.1 | 21 | 71 | 10 | 29 | 10 |
| Val | 238 | 121.1 | 21 | 71 | 10 | 29 | 10 |
| Thr | 240 | 121.1 | 21 | 71 | 10 | 29 | 10 |
| Tau | 246 | 121.1 | 21 | 56 | 10 | 15 | 8 |
| HyPro | 252.2 | 121.1 | 21 | 71 | 10 | 29 | 10 |
| Ile | 252 | 121.1 | 21 | 71 | 10 | 29 | 10 |
| Leu | 252.1 | 121.1 | 21 | 71 | 10 | 29 | 10 |
| Asn | 253 | 121.1 | 21 | 71 | 10 | 29 | 10 |
| Orn | 373 | 133.1 | 21 | 71 | 10 | 23 | 10 |
| Asp | 254 | 121.1 | 21 | 71 | 10 | 29 | 10 |
| Gln | 267 | 121.1 | 21 | 71 | 10 | 29 | 10 |
| Lys | 387 | 147.1 | 21 | 71 | 10 | 23 | 10 |
| Glu | 268 | 121.1 | 21 | 71 | 10 | 29 | 10 |
| Met | 270 | 121.1 | 21 | 71 | 10 | 29 | 10 |
| His | 276 | 156.1 | 21 | 36 | 10 | 15 | 14 |
| aAAA | 282 | 121.1 | 21 | 71 | 10 | 29 | 10 |
| HyLys | 403 | 163.1 | 21 | 71 | 10 | 23 | 10 |
| Phe | 286 | 121.1 | 21 | 71 | 10 | 29 | 10 |
| 1 MeHis | 290 | 170.1 | 21 | 60 | 10 | 23 | 10 |
| 3MeHis | 290.1 | 170.1 | 21 | 60 | 10 | 23 | 10 |
| Arg | 295 | 175.1 | 21 | 71 | 10 | 29 | 10 |
| Cit | 296 | 121.1 | 21 | 71 | 10 | 29 | 10 |
| Tyr | 302 | 121.1 | 21 | 71 | 10 | 29 | 10 |

**[Table 1-2]**

| amino acid | m/z (Q1) | m/z (Q3) | Dwell time (msec) | DP (V) | EP (V) | CE (V) | CXP (V) |
|---|---|---|---|---|---|---|---|
| Trp | 325 | 121.1 | 21 | 71 | 10 | 29 | 10 |
| Cysthi | 463 | 223.1 | 21 | 71 | 10 | 29 | 10 |
| Car | 347 | 227.1 | 21 | 71 | 10 | 15 | 14 |
| Ans | 361 | 241.1 | 21 | 71 | 10 | 29 | 10 |
| Cys2 | 481 | 121.1 | 21 | 71 | 10 | 29 | 10 |
| Phed5 | 291 | 121.1 | 21 | 71 | 10 | 29 | 10 |
| Phe IS | 296.1 | 121.1 | 21 | 71 | 10 | 29 | 10 |
| Ala IS | 213 | 121.1 | 21 | 71 | 10 | 29 , | 10 |
| Gln IS | 274 | 121.1 | 21 | 71 | 10 | 29 | 10 |
| Glu IS | 274.1 | 121.1 | 21 | 71 | 10 | 29 | 10 |
| Ser IS | 230 | 121.1 | 21 | 71 | 10 | 29 | 10 |
| Lys IS | 395 | 155.1 | 21 | 71 | 10 | 23 | 10 |
| Arg IS | 299 | 179.1 | 21 | 71 | 10 | 21 | 8 |
| Gly IS | 199 | 121.1 | 21 | 71 | 10 | 29 | 10 |
| Pro IS | 242 | 121.1 | 21 | 71 | 10 | 29 | 10 |
| Leu IS | 255 | 121.1 | 21 | 71 | 10 | 29 | 10 |
| Met IS | 276.1 | 121.1 | 21 | 71 | 10 | 29 | 10 |
| His IS | 285 | 165.1 | 21 | 36 | 10 | 15 | 14 |
| Cit IS | 300 | 121.1 | 21 | 71 | 10 | 29 | 10 |
| Orn IS | 378 | 121.1 | 21 | 71 | 10 | 29 | 10 |
| Val IS | 244 | 121.1 | 21 | 71 | 10 | 29 | 10 |
| Thr IS | 244.1 | 121.1 | 21 | 71 | 10 | 29 | 10 |
| Trp IS | 338 | 121.1 | 21 | 71 | 10 | 29 | 10 |
| Tau IS | 248 | 121.1 | 21 | 71 | 10 | 29 | 10 |
| Tyr IS | 308 | 121.1 | 21 | 71 | 10 | 29 | 10 |
| EtOHNH2 IS | 186 | 121.1 | 21 | 71 | 10 | 29 | 10 |
| Asp IS | 257 | 121.1 | 21 | 71 | 10 | 29 | 10 |
| Asn IS | 259.1 | 121.1 | 21 | 71 | 10 | 29 | 10 |
| Ile IS | 259 | 121.1 | 21 | 71 | 10 | 29 | 10 |
| 3MetHis IS | 293 | 121.1 | 21 | 71 | 10 | 29 | 10 |
| Cys2 IS | 485 | 121.1 | 21 | 71 | 10 | 29 | 10 |

### (Extraction of protein)

Among the collected stratum corneum samples, a stratum corneum sample different from the one from which the water-soluble components were extracted was used to obtain an insoluble fraction. The collected tape (stratum corneum) was subjected to ultrasonication three times using hexane to separate the stratum corneum from the tape. This separated stratum corneum was subjected to ultrasonication using hexane and centrifugation three times to remove the adhesive from the tape. A dissolving solution (ammonium bicarbonate, sodium lauryl sulfate, urea) was added to the remaining precipitate, which was then subjected to ultrasonication, and the supernatant was used as a sample for protein analysis.

### (Quantification of protein)

A portion of the sample for protein analysis was collected, and the protein concentration of the sample for protein analysis was determined using a commercially available quantification kit (Micro BCA Protein Assay Kit (model number: 23235), manufactured by ThermoFisher Scientific).

### (Trypsin digestion of protein)

A 100 mM dithiothreitol solution was added to the sample for protein analysis (about 10 µg was used) (adjusted depending on the amount of protein solution to give a final concentration of about 10 mM), and the sample was heated at 80°C for 15 min for reduction. A 150 mM iodoacetic acid solution was added (adjusted by the amount of protein solution to give a final concentration of about 20 mM), and alkylation was performed in the dark for 30 min at room temperature. After diluting the urea concentration to 2 mM or less with 100 mM ammonium bicarbonate solution, 200 ng/µL trypsin solution was added (about 1/20 to 1/5 of the amount of protein) and digestion was performed at 37°C for about 18 hr. The digestion solution was desalted using MonoSpin(R), dried using a centrifugal concentrator, and stored at -80°C. For analysis, the solution was dissolved in 0.01% trifluoroacetic acid and 2% acetonitrile and subjected to LC/MS analysis.

### (LC/MS conditions)

For LC/MS analysis, a Prominence nano system (Shimadzu Corporation) and an LTQ Orbitrap XL ETD (Thermo Fisher Scientific) system were used. For LC, an ion exchange column (inner diameter 1.0 mm, length 50 mm) containing a mixture of PolySULFOETHYL A and PolyWAX LP resins was used for the first-dimension separation, and stepwise elution was performed using 0.01% formic acid, 2% acetonitrile solution as mobile phase A and 500 mM ammonium formate, 2% acetonitrile solution as mobile phase B. For the second-dimension nano-HPLC, a trap column (inner diameter 0.2 mm, length 50 mm) and a separation column (inner diameter 0.05 mm, length 50 mm) MonoCap(R) C18 (GL Sciences) were used, and a gradient with a flow rate of 200 nL/min was used with 0.1% formic acid, 2% acetonitrile solution as mobile phase A and 0.1% formic acid, 90% acetonitrile solution as mobile phase B, and the protein content of about 0.2 µg was analyzed. The mass spectrometer collected data by MS/MS measurement using electrospray ionization method with a micrometal chip (Eisho Metals).

### (Identification of protein)

Mascot (Matrix Science) was used as the search engine for protein identification, and Scaffold software (proteome software) was used for data comparison. Sprot (Swiss-Prot) was used as the search database, and carboxymethylation of cysteine residue was used as the Fixed Modifications during the search, and oxidation of methionine and acetylation of the N-terminus were used as the Variable Modifications. Trypsin was used as the digestive enzyme, and Max Missed Cleavage was set to 2.

### (Selection of proteins important for moisture content of stratum corneum)

Women aged 39 years or younger (listed as "young people" in Table 2-1) were selected from the group of test subjects. Of these women, three with high stratum corneum moisture content and three with low stratum corneum moisture content were selected. The correlation coefficient between the stratum corneum moisture content and the amount of identified proteins for these 6 women was calculated. A threshold for the correlation coefficient was set, and proteins with a threshold of 0.9 or higher were selected (A). In Table 2-1, combinations that exceeded the threshold are marked with O, and combinations that did not exceed are marked with ×. Similarly, 10 people (5 with high stratum corneum moisture content and 5 with low stratum corneum moisture content) were selected, and proteins with a threshold of 0.7 or higher were selected (B). Similarly, 14 people (7 with high stratum corneum moisture content and 7 with low stratum corneum moisture content) were selected, and proteins with a threshold of 0.6 or higher were selected (C). Similarly, 20 people (10 with high stratum corneum moisture content and 10 with low stratum corneum moisture content) were selected, and proteins with a threshold of 0.5 or higher were selected (D). Proteins that were selected three or more times in the selection of A to D were selected as proteins important for the moisture content of stratum corneum (selection method (1)).

Furthermore, using the identified proteins, proteins with a strong positive correlation between stratum corneum moisture content and protein amount (a threshold of 0.6 was set) were selected, wherein the targets were only those test subjects in which the protein was detected among women aged 39 or younger, and selected as proteins important for the moisture content of stratum corneum (selection method (2)).

Next, women aged 40 or older (listed as "older people" in Table 2-2) were selected from the group of test subjects. As in the above-mentioned case of women aged 39 or younger, proteins important for the moisture content of stratum corneum were selected by the selection method (1) and selection metho (2). The threshold for the correlation coefficient was set according to the data of the subject, and the numerical values thereof are shown in Table 2-2.

From the above-mentioned results, it was found that arginase-1, catalase, cystatin-M, desmoglein-1, glutathione S-transferase P, keratin type II cytoskeleton 78, myosin-14, myosin-9, protein-glutamine γ-glutamyltransferase E, γ-glutamyl cyclotransferase, and keratin type I cuticle Ha3-I are important proteins that increase the moisture content of stratum corneum as the protein amount increases. The correlation coefficients between each protein and the moisture content of stratum corneum are shown in Tables 2-1 and 2-2.

**[Table 2-1]**

| moisture content of stratum corneum (20 - 39 years old) | | | | | | |
|---|---|---|---|---|---|---|
| | | young people | | | | |
| | | selection method (1) | | | | selection method (2) |
| | | 3 people each | 5 people each | 7 people each | 10 people each | all in which protein was detected |
| threshold | | 0.9 | 0.7 | 0.6 | 0.5 | 0.6 |
| correlation coefficient | arginase-1 | O | ○ | ○ | ○ | |
| | catalase | | | | | ○ |
| | cystatin-M | | | | | ○ |
| | desmoglein-1 | × | ○ | ○ | ○ | |
| | glutathione S-transferase P | ○ | ○ | ○ | ○ | |
| | keratin type II | ○ | ○ | ○ | ○ | |
| | cytoskeleton 78 | | | | | |
| | myosin-14 | ○ | ○ | ○ | ○ | ○ |
| | myosin-9 | | | | | ○ |
| | protein-glutamine γ-glutamyltransferase E | | | | | ○ |

**[Table 2-2]**

| moisture content of stratum corneum (40 - 62 years old) | | | | | | |
|---|---|---|---|---|---|---|
| | | older people | | | | |
| | | selection method (1) | | | | selection method (2) |
| | | 3 people each | 5 people each | 7 people each | 10 people each | all in which protein was detected |
| threshold | | 0.7 | 0.6 | 0.5 | 0.45 | 0.5 |
| correlation coefficient | γ-glutamyl cyclotransferase | ○ | ○ | ○ | ○ | ○ |
| | keratin type I | | | | | ○ |
| | cuticle Ha3-I | | | | | |

### (Selection of proteins important for spots)

Women aged 39 years or younger (listed as "young people" in Table 3-1) were selected from the group of test subjects. Of these women, three with high melanin content and three with low melanin content were selected. The correlation coefficient between the melanin content and the amount of identified proteins for these 6 women was calculated. A threshold for the correlation coefficient was set, and proteins with a threshold of -0.75 or lower were selected (A). In Table 3-1, combinations that exceeded the threshold are marked with O, and combinations that did not exceed are marked with ×. Similarly, 10 people (5 with high melanin content and 5 with low melanin content) were selected, and proteins with a threshold of -0.65 or lower were selected (B). Similarly, 14 people (7 with high melanin content and 7 with low melanin content) were selected, and proteins with a threshold of -0.55 or lower were selected (C). Similarly, 20 people (10 with high melanin content and 10 with low melanin content) were selected, and proteins with a threshold of -0.5 or lower were selected (D). Proteins that were selected three or more times in the selection of A to D were selected as proteins important for spots (melanin content) (selection method (1)).

Furthermore, using the identified proteins, proteins with a strong negative correlation between melanin content and protein amount (a threshold of -0.5 was set) were selected, wherein the targets were only those test subjects in which the protein was detected among women aged 39 or younger, and selected as proteins important for spots (melanin content) (selection method (2)).

Next, women aged 40 or older (listed as "older people" in Table 3-2) were selected from the group of test subjects. As in the above-mentioned case of women aged 39 or younger, proteins important for spots (melanin content) were selected by the selection method (1) and selection metho (2). The threshold for the correlation coefficient was set according to the data of the subject, and the numerical values thereof are shown in Table 3-2.

From the above-mentioned results, it was found that desmoglein-1, γ-glutamyl cyclotransferase, keratin type II cytoskeleton 5, keratin type II cytoskeleton 78, keratin type II cytoskeleton 80, lysosome C, neuroblast differentiation-related protein AHNAK, protein-glutamine γ-glutamyltransferase E, thioredoxin, zinc-α-2-glycoprotein, cystatin-M, and protein S100-A8 are important proteins for spots (melanin content). The correlation coefficients between each protein and spots (melanin content) are shown in Tables 3-1 and 3-2.

**[Table 3-1]**

| melanin content (20 - 39 years old) | | | | | | |
|---|---|---|---|---|---|---|
| | | young people | | | | |
| | | selection method (1) | | | | selection method (2) |
| | | 3 people each | 5 people each | 7 people each | 10 people each | all in which protein was detected |
| threshold | | -0.75 | -0.65 | -0.55 | -0.5 | -0.5 |
| correlation coefficient | desmoglein-1 | × | ○ | ○ | ○ | |
| | γ-glutamyl cyclotransferase | ○ | ○ | ○ | × | |
| | keratin type II | × | ○ | ○ | ○ | |
| | cytoskeleton 5 | | | | | |
| | keratin type II | | | | | ○ |
| | cytoskeleton 78 | | | | | |
| | keratin type II | × | ○ | ○ | ○ | |
| | cytoskeleton 80 | | | | | |
| | lysosome C | ○ | ○ | ○ | ○ | |
| | neuroblast differentiation-related protein AHNAK | | | | | ○ |
| | protein-glutamine γ-glutamyltransferase E | × | ○ | ○ | ○ | |
| | thioredoxin | ○ | ○ | ○ | ○ | |
| | zinc-α-2-glycoprotein | × | ○ | ○ | ○ | ○ |

**[Table 3-2]**

| melanin content (40 - 62 years old) | | | | | | |
|---|---|---|---|---|---|---|
| | | older people | | | | |
| | | selection method (1) | | | | selection method (2) |
| | | 3 people each | 5 people each | 7 people each | 10 people each | all in which protein was detected |
| threshold | | -0.5 | -0.45 | -0.4 | -0.35 | -0.45 |
| correlation coefficient | cystatin-M | ○ | ○ | ○ | ○ | |
| | protein S100-A8 | ○ | ○ | ○ | ○ | ○ |

### (Selection of proteins important for barrier function)

Women aged 39 years or younger (listed as "young people" in Table 4-1) were selected from the group of test subjects. Of these women, three with high transepidermal water transpiration amount and three with low transepidermal water transpiration amount were selected. The correlation coefficient between the transepidermal water transpiration amount and the amount of identified proteins for these 6 women was calculated. A threshold for the correlation coefficient was set, and proteins with a threshold of -0.6 or lower were selected (A). In Table 4-1, combinations that exceeded the threshold are marked with O, and combinations that did not exceed are marked with ×. Similarly, 10 people (5 with high transepidermal water transpiration amount and 5 with low transepidermal water transpiration amount) were selected, and proteins with a threshold of -0.4 or lower were selected (B). Similarly, 14 people (7 with high transepidermal water transpiration amount and 7 with low transepidermal water transpiration amount) were selected, and proteins with a threshold of -0.35 or lower were selected (C). Similarly, 20 people (10 with high transepidermal water transpiration amount and 10 with low transepidermal water transpiration amount) were selected, and proteins with a threshold of -0.3 or lower were selected (D). Proteins that were selected three or more times in the selection of A to D were selected as proteins important for barrier function (transepidermal water transpiration amount) (selection method (1)).

Furthermore, using the identified proteins, proteins with a strong negative correlation between transepidermal water transpiration amount and protein amount (a threshold of -0.5 was set) were selected, wherein the targets were only those test subjects in which the protein was detected among women aged 39 or younger, and selected as proteins important for barrier function (transepidermal water transpiration amount)

### (selection method (2)).

Next, women aged 40 or older (listed as "older people" in Table 4-2) were selected from the group of test subjects. As in the above-mentioned case of women aged 39 or younger, proteins important for barrier function (transepidermal water transpiration amount) were selected by the selection method (1) and selection method (2). The threshold for the correlation coefficient was set according to the data of the subject, and the numerical values thereof are shown in Table 4-2.

From the above-mentioned results, it was found that caspase-14, keratin type II cuticle Hb5, keratin type II cytoskeleton 1b, phosphatidylethanolamine-binding protein 1, hornerin, keratin type I cuticle Ha1, keratin type I cuticle Ha3-I, keratin type II cuticle Hb3, keratin type II cuticle Hb6, and thioredoxin are important proteins for barrier function (transepidermal water transpiration amount). The correlation coefficients between each protein and barrier function (transepidermal water transpiration amount) are shown in Tables 4-1 and 4-2.

**[Table 4-1]**

| transepidermal water transpiration amount (20 - 39 years old) | | | | | | |
|---|---|---|---|---|---|---|
| | | young people | | | | |
| | | selection method (1) | | | | selection method (2) |
| | | 3 people each | 5 people each | 7 people each | 10 people each | all in which protein was detected |
| threshold | | -0.6 | -0.4 | -0.35 | -0.3 | -0.5 |
| correlation coefficient | caspase-14 | ○ | ○ | ○ | ○ | |
| | keratin type II | × | ○ | ○ | ○ | |
| | cuticle Hb5 | | | | | |
| | keratin type II | ○ | ○ | ○ | × | |
| | cytoskeleton 1b | | | | | |
| | phosphatidylethanolamine-binding protein 1 | | | | | ○ |

**[Table 4-2]**

| transepidermal water transpiration amount (40 - 62 years old) | | | | | | |
|---|---|---|---|---|---|---|
| | | older people | | | | |
| | | selection method (1) | | | | selection method (2) |
| | | 3 people each | 5 people each | 7 people each | 10 people each | all in which protein was detected |
| threshold | | -0.7 | -0.6 | -0.5 | -0.45 | -0.5 |
| correlation coefficient | hornerin | | | | | ○ |
| | keratin type I | ○ | ○ | ○ | ○ | |
| | cuticle Ha1 | | | | | |
| | keratin type I | ○ | ○ | ○ | ○ | |
| | cuticle Ha3-I | | | | | |
| | keratin type II | ○ | × | ○ | ○ | |
| | cuticle Hb3 | | | | | |
| | keratin type II | ○ | ○ | ○ | × | |
| | cuticle Hb6 | | | | | |
| | thioredoxin | × | ○ | ○ | ○ | |

### (Selection of proteins important for pH value)

Women aged 39 years or younger (listed as "young people" in Table 5-1) were selected from the group of test subjects. Of these women, three with high pH value and three with low pH value were selected. The correlation coefficient between the pH value and the amount of identified proteins for these 6 women was calculated. A threshold for the correlation coefficient was set, and proteins with a threshold of -0.7 or lower were selected (A). In Table 5-1, combinations that exceeded the threshold are marked with O, and combinations that did not exceed are marked with ×. Similarly, 10 people (5 with high pH value and 5 with low pH value) were selected, and proteins with a threshold of -0.5 or lower were selected (B). Similarly, 14 people (7 with high pH value and 7 with low pH value) were selected, and proteins with a threshold of -0.45 or lower were selected (C). Similarly, 20 people (10 with high pH value and 10 with low pH value) were selected, and proteins with a threshold of -0.35 or lower were selected (D). Proteins that were selected three or more times in the selection of A to D were selected as proteins important for pH value (selection method (1)).

Furthermore, using the identified proteins, proteins with a strong negative correlation between pH value and protein amount (a threshold of -0.5 was set) were selected, wherein the targets were only those test subjects in which the protein was detected among women aged 39 or younger, and selected as proteins important for pH value (selection method (2)).

Next, women aged 40 or older (listed as "older people" in Table 5-2) were selected from the group of test subjects. As in the above-mentioned case of women aged 39 or younger, proteins important for pH value were selected by the selection method (1) and selection method (2). The threshold for the correlation coefficient was set according to the data of the subject, and the numerical values thereof are shown in Table 5-2.

From the above-mentioned results, it was found that 78 kDa glucose regulated protein, actin cytoplasmic 2, α-actin-4, apolipoprotein D, desmoglein-1, epiplakin, filaggrin, heat shock protein β-1, interleukin-36γ, protein S100-P, α-enolase, keratin type II cuticle Hb3, keratin type II cytoskeleton 75, protein-glutamine γ-glutamyltransferase K, serpin B3, and triose phosphoric acid isomerase are important proteins for pH value. The correlation coefficients between each protein and pH value are shown in Tables 5-1 and 5-2.

**[Table 5-1]**

| pH (20 - 39 years old) | | | | | | |
|---|---|---|---|---|---|---|
| | | young people | | | | |
| | | selection method (1) | | | | selection method (2) |
| | | 3 people each | 5 people each | 7 people each | 10 people each | all in which protein was detected |
| threshold | | -0.7 | -0.5 | -0.45 | -0.35 | -0.5 |
| correlation coefficient | 78 kDa glucose regulated protein | | | | | ○ |
| | actin cytoplasmic 2 | ○ | ○ | ○ | ○ | |
| | α-actin-4 | × | ○ | ○ | ○ | |
| | apolipoprotein | | | | | ○ |
| | desmoglein-1 | ○ | × | ○ | ○ | |
| | epiplakin | × | ○ | ○ | ○ | |
| | filaggrin | ○ | ○ | ○ | ○ | |
| | heat shock protein β-1 | × | ○ | ○ | ○ | ○ |
| | interleukin-36γ | × | ○ | ○ | ○ | |
| | protein S100-P | ○ | × | ○ | ○ | |

**[Table 5-2]**

| pH (40 - 62 years old) | | | | | | |
|---|---|---|---|---|---|---|
| | | older people | | | | |
| | | selection method (1) | | | | selection method (2) |
| | | 3 people each | 5 people each | 7 people each | 10 people each | all in which protein was detected |
| threshold | | -0.5 | -0.45 | -0.4 | -0.35 | -0.35 |
| correlation coefficient | α-enolase | × | ○ | ○ | ○ | |
| | keratin type II | | | | | ○ |
| | cuticle Hb3 | | | | | |
| | keratin type II | × | ○ | ○ | ○ | |
| | cytoskeleton 75 | | | | | |
| | protein-glutamine γ-glutamyltransferase K | × | ○ | ○ | ○ | |
| | serpin B3 | | | | | ○ |
| | triose phosphoric acid isomerase | | | | | ○ |

### (Correlation between protein and L-amino acid)

The correlation coefficient between each selected protein and the amount of L-amino acid (the ratio of each L-amino acid to all detected L-amino acids was calculated and used for each subject) was calculated, and a correlation coefficient threshold was set for each protein. Furthermore, different correlation coefficient thresholds were set for essential amino acids and non-essential amino acids. A strong correlation was determined when the absolute value of the correlation coefficient was 0.1 or more higher than the set threshold, and the presence of a correlation was determined when the absolute value was equal to or less than 0.1 higher than the set threshold.

Tables 6-1 to 6-11 show the presence or absence of a correlation between proteins selected as proteins important for the moisture content of stratum corneum and each amino acid, Tables 7-1 to 7-12 show the presence or absence of a correlation between proteins selected as proteins important for spots and each amino acid, Tables 8-1 to 8-10 show the presence or absence of a correlation between proteins selected as proteins important for barrier function and each amino acid, and Tables 9-1 to 9-16 show the presence or absence of a correlation between proteins important for pH value and each amino acid.

In each table, in the "presence or absence of correlation", ⊙ indicates that the absolute value of the correlation coefficient is 0.1 or more higher than the set threshold (strong correlation), and O indicates that the absolute value of the correlation coefficient is equal to or less than 0.1 higher than the set threshold (correlation is present).

**[Table 6-1]**

| moisture content of the stratum corneum 20 - 39 years old | | | | | | |
|---|---|---|---|---|---|---|
| | presence or absence of correlation between arginase-1 and each amino acid | | | | | |
| amino acid | essential amino acid | | | | non-essential amino acid | |
| | Val | Leu | Phe | Trp | PCA | Orn |
| presence or absence of correlation | ○ | ○ | ○ | ⊙ | ⊙ | ○ |

| | | | | | | |
|---|---|---|---|---|---|---|
| [set threshold] essential amino acid: strong correlation 0.5, correlation 0.4; non-essential amino acid: strong correlation 0.6, correlation 0.5 | | | | | | |

**[Table 6-2]**

| moisture content of the stratum corneum 20 - 39 years old | | | | | | |
|---|---|---|---|---|---|---|
| | presence or absence of correlation between catalase and each amino acid | | | | | |
| amino acid | essential amino acid | | | non-essential amino acid | | |
| | Val | Ile | His | Asn | Arg | Tyr |
| presence or absence of correlation | ○ | ○ | ⊙ | ○ | ○ | ⊙ |

| | | | | | | |
|---|---|---|---|---|---|---|
| [set threshold] essential amino acid: strong correlation 0.6, correlation 0.5; non-essential amino acid: strong correlation 0.5, correlation 0.4 | | | | | | |

**[Table 6-3]**

| moisture content of the stratum corneum 20 - 39 years old | | | | | | |
|---|---|---|---|---|---|---|
| | presence or absence of correlation between cystatin-M and each amino acid | | | | | |
| amino acid | essential amino acid | | | | non-essential amino acid | |
| | Thr | Leu | Phe | Trp | UCA | PCA |
| presence or absence of correlation | ○ | ⊙ | ○ | ○ | ⊙ | ○ |

| | | | | | | |
|---|---|---|---|---|---|---|
| [set threshold] essential amino acid: strong correlation 0.5, correlation 0.4; non-essential amino acid: strong correlation 0.7, correlation 0.6 | | | | | | |

**[Table 6-4]**

| moisture content of the stratum corneum 20 - 39 years old | | | | | | |
|---|---|---|---|---|---|---|
| | presence or absence of correlation between desmoglein-1 and each amino acid | | | | | |
| amino acid | essential amino acid | | | | non-essential amino acid | |
| | Leu | Met | Phe | Trp | PCA | Asn |
| presence or absence of correlation | ○ | ○ | ○ | ○ | ○ | ○ |

| | | | | | | |
|---|---|---|---|---|---|---|
| [set threshold] essential amino acid: strong correlation 0.4, correlation 0.3; non-essential amino acid: strong correlation 0.5, correlation 0.4 | | | | | | |

**[Table 6-5]**

| moisture content of the stratum corneum 20 - 39 years old | | | | | | | | |
|---|---|---|---|---|---|---|---|---|
| | presence or absence of correlation between glutathione S-transferase P and each amino acid | | | | | | | |
| amino acid | essential amino acid | | | | non-essential amino acid | | | |
| | Ile | Lys | Met | His | Gly | Ser | Asn | Tyr |
| presence or absence of correlation | ⊙ | ○ | ○ | ⊙ | ○ | ⊙ | ⊙ | ○ |

| | | | | | | | | |
|---|---|---|---|---|---|---|---|---|
| [set threshold] essential amino acid: strong correlation 0.6, correlation 0.5; non-essential amino acid: strong correlation 0.7, correlation 0.6 | | | | | | | | |

**[Table 6-6]**

| moisture content of the stratum corneum 20 - 39 years old | | | | | |
|---|---|---|---|---|---|
| | presence or absence of correlation between keratin type II cytoskeleton 78 and each amino acid | | | | |
| amino acid | essential amino acid | | | non-essential amino acid | |
| | Met | His | Trp | PCA | Orn |
| presence or absence of correlation | ○ | ○ | ○ | ○ | ○ |

| | | | | | |
|---|---|---|---|---|---|
| [set threshold] essential amino acid: strong correlation 0.5, correlation 0.4; non-essential amino acid: strong correlation 0.7, correlation 0.6 | | | | | |

**[Table 6-7]**

| moisture content of the stratum corneum 20 - 39 years old | | | | |
|---|---|---|---|---|
| | presence or absence of correlation between myosin-14 and each amino acid | | | |
| amino acid | essential amino acid | non-essential amino acid | | |
| | Trp | PCA | Orn | Cit |
| presence or absence of correlation | ○ | ⊙ | ○ | ○ |

| | | | | |
|---|---|---|---|---|
| [set threshold] essential amino acid: strong correlation 0.5, correlation 0.4; non-essential amino acid: strong correlation 0.5, correlation 0.4 | | | | |

**[Table 6-8]**

| moisture content of the stratum corneum 20 - 39 years old | | | |
|---|---|---|---|
| | presence or absence of correlation between myosin-9 and each amino acid | | |
| amino acid | non-essential amino acid | | |
| | UCA | Asp | Cit |
| presence or absence of correlation | ○ | ○ | ⊙ |

| | | | |
|---|---|---|---|
| [set threshold] essential amino acid: strong correlation 0.4, correlation 0.3; non-essential amino acid: strong correlation 0.5, correlation 0.4 | | | |

**[Table 6-9]**

| moisture content of the stratum corneum 20 - 39 years old | | | | | | |
|---|---|---|---|---|---|---|
| | presence or absence of correlation between protein-glutamine γ-glutamyltransferase E and each amino acid | | | | | |
| amino acid | essential amino acid | | non-essential amino acid | | | |
| | Val | Trp | PCA | Tau | Orn | Tyr |
| presence or absence of correlation | ○ | ⊙ | ○ | ○ | ⊙ | ○ |

| | | | | | | |
|---|---|---|---|---|---|---|
| [set threshold] essential amino acid: strong correlation 0.4, correlation 0.3; non-essential amino acid: strong correlation 0.5, correlation 0.4 | | | | | | |

**[Table 6-10]**

| moisture content of the stratum corneum 40 - 62 years old | | | |
|---|---|---|---|
| | presence or absence of correlation between γ-glutamyl cyclotransferase and each amino acid | | |
| amino acid | essential amino acid | non-essential amino acid | |
| | Thr | Asn | Asp |
| presence or absence of correlation | ○ | ○ | ⊙ |

| | | | |
|---|---|---|---|
| [set threshold] essential amino acid: strong correlation 0.4, correlation 0.3; non-essential amino acid: strong correlation 0.5, correlation 0.4 | | | |

**[Table 6-11]**

| moisture content of the stratum corneum 40 - 62 years old | | | |
|---|---|---|---|
| | presence or absence of correlation between keratin type I cuticle Ha3-I and each amino acid | | |
| amino acid | essential amino acid | non-essential amino acid | |
| | Met | Tau | Orn |
| presence or absence of correlation | ○ | ○ | ○ |

| | | | |
|---|---|---|---|
| [set threshold] essential amino acid: strong correlation 0.4, correlation 0.3; non-essential amino acid: strong correlation 0.6, correlation 0.5 | | | |

From the results of Table A6-1, it was shown that specific amino acids (i.e., Val, Leu, Phe, Trp, PCA, and Orn) are correlated with arginase-1, which is a protein important for the moisture content of stratum corneum. Among these, the essential amino acids Val, Leu, Phe, and Trp are considered to be more strongly involved in protein production. These results suggest that the above-mentioned specific amino acids promote the production of arginase-1, and are therefore effective in improving a decrease in the moisture content of stratum corneum (especially a decrease in the moisture content of stratum corneum in younger people).

From the results of Table 6-2, it was shown that specific amino acids (i.e., Val, Ile, His, Asn, Arg, and Tyr) are correlated with catalase, which is a protein important for the moisture content of stratum corneum. Among these, the essential amino acids Val, Ile, and His are considered to be more strongly involved in protein production. These results suggest that the above-mentioned specific amino acids promote the production of catalase, and are therefore effective in improving a decrease in the moisture content of stratum corneum (especially a decrease in the moisture content of stratum corneum in younger people).

From the results of Table 6-3, it was shown that specific amino acids (i.e., Thr, Leu, Phe, Trp, UCA, and PCA) are correlated with cystatin-M, which is a protein important for the moisture content of stratum corneum. Among these, the essential amino acids Thr, Leu, Phe, and Trp are considered to be more strongly involved in protein production. These results suggest that the above-mentioned specific amino acids promote the production of cystatin-M, and are therefore effective in improving a decrease in the moisture content of stratum corneum (especially a decrease in the moisture content of stratum corneum in younger people).

From the results of Table 6-4, it was shown that specific amino acids (i.e., Leu, Met, Phe, Trp, PCA, and Asn) are correlated with desmoglein-1, which is a protein important for the moisture content of stratum corneum. Among these, the essential amino acids Leu, Met, Phe, and Trp are considered to be more strongly involved in protein production. These results suggest that the above-mentioned specific amino acids promote the production of desmoglein-1, and are therefore effective in improving a decrease in the moisture content of stratum corneum (especially a decrease in the moisture content of stratum corneum in younger people).

From the results of Table 6-5, it was shown that specific amino acids (i.e., Ile, Lys, Met, His, Gly, Ser, Asn, and Tyr) are correlated with glutathione S-transferase P, which is a protein important for the moisture content of stratum corneum. Among these, the essential amino acids Ile, Lys, Met, and His are considered to be more strongly involved in protein production. These results suggest that the above-mentioned specific amino acids promote the production of glutathione S-transferase P, and are therefore effective in improving a decrease in the moisture content of stratum corneum (especially a decrease in the moisture content of stratum corneum in younger people).

From the results of Table 6-6, it was shown that specific amino acids (i.e., Met, His, Trp, PCA, and Orn) are correlated with keratin type II cytoskeleton 78, which is a protein important for the moisture content of stratum corneum. Among these, the essential amino acids Met, His, and Trp are considered to be more strongly involved in protein production. These results suggest that the above-mentioned specific amino acids promote the production of keratin type II cytoskeleton 78, and are therefore effective in improving a decrease in the moisture content of stratum corneum (especially a decrease in the moisture content of stratum corneum in younger people).

From the results of Table 6-7, it was shown that specific amino acids (i.e., Trp, PCA, Orn, and Cit) are correlated with myosin-14, which is a protein important for the moisture content of stratum corneum. Among these, the essential amino acid Trp is considered to be more strongly involved in protein production. These results suggest that the above-mentioned specific amino acid promotes the production of myosin-14, and are therefore effective in improving a decrease in the moisture content of stratum corneum (especially a decrease in the moisture content of stratum corneum in younger people).

From the results of Table 6-8, it was shown that specific amino acids (i.e., UCA, Asp, and Cit) are correlated with myosin-9, which is a protein important for the moisture content of stratum corneum. These results suggest that the above-mentioned specific amino acids promote the production of myosin-9, and are therefore effective in improving a decrease in the moisture content of stratum corneum (especially a decrease in the moisture content of stratum corneum in younger people).

From the results of Table 6-9, it was shown that specific amino acids (i.e., Val, Trp, PCA, Tau, Orn, and Tyr) are correlated with protein-glutamine γ-glutamyltransferase E, which is a protein important for the moisture content of stratum corneum. Among these, the essential amino acids Val and Trp are considered to be more strongly involved in protein production. These results suggest that the above-mentioned specific amino acids promote the production of protein-glutamine γ-glutamyltransferase E, and are therefore effective in improving a decrease in the moisture content of stratum corneum (especially a decrease in the moisture content of stratum corneum in younger people).

From the results of Table 6-10, it was shown that specific amino acids (i.e., Thr, Asn, and Asp) are correlated with γ-glutamyl cyclotransferase, which is a protein important for the moisture content of stratum corneum. Among these, the essential amino acid Thr is considered to be more strongly involved in protein production. These results suggest that the above-mentioned specific amino acid promotes the production of γ-glutamyl cyclotransferase, and are therefore effective in improving a decrease in the moisture content of stratum corneum (especially a decrease in the moisture content of stratum corneum in older people).

From the results of Table 6-11, it was shown that specific amino acids (i.e., Met, Tau, and Orn) are correlated with keratin type I cuticle Ha3-I, which is a protein important for the moisture content of stratum corneum. Among these, the essential amino acid Met is considered to be more strongly involved in protein production. These results suggest that the above-mentioned specific amino acid promotes the production of keratin type I cuticle Ha3-I, and are therefore effective in improving a decrease in the moisture content of stratum corneum (especially a decrease in the moisture content of stratum corneum in older people).

**[Table 7-1]**

| melanin content 20 - 39 years old | | | | | | |
|---|---|---|---|---|---|---|
| | presence or absence of correlation between desmoglein-1 and each amino acid | | | | | |
| amino acid | essential amino acid | | | | non-essential amino acid | |
| | Leu | Met | Phe | Trp | PCA | Asn |
| presence or absence of correlation | ○ | ○ | ○ | ○ | ○ | ○ |

| | | | | | | |
|---|---|---|---|---|---|---|
| [set threshold] essential amino acid: strong correlation 0.4, correlation 0.3; non-essential amino acid: strong correlation 0.5, correlation 0.4 | | | | | | |

**[Table 7-2]**

| melanin content 20 - 39 years old | | | | | | | |
|---|---|---|---|---|---|---|---|
| | presence or absence of correlation between γ-glutamyl cyclotransferase and each amino acid | | | | | | |
| amino acid | essential amino acid | | | | non-essential amino acid | | |
| | Val | Ile | Lys | Met | PCA | Ala | Asn |
| presence or absence of correlation | ○ | ○ | ○ | ○ | ○ | ○ | ⊙ |

| | | | | | | | |
|---|---|---|---|---|---|---|---|
| [set threshold] essential amino acid: strong correlation 0.5, correlation 0.4; non-essential amino acid: strong correlation 0.5, correlation 0.4 | | | | | | | |

**[Table 7-3]**

| melanin content 20 - 39 years old | | | |
|---|---|---|---|
| | presence or absence of correlation between keratin type II cytoskeleton 5 and each amino acid | | |
| amino acid | essential amino acid | | |
| | Val | Lys | His |
| presence or absence of correlation | ○ | ○ | ⊙ |

| | | | |
|---|---|---|---|
| [set threshold] essential amino acid: strong correlation 0.4, correlation 0.3 | | | |

**[Table 7-4]**

| melanin content 20 - 39 years old | | | | | |
|---|---|---|---|---|---|
| | presence or absence of correlation between keratin type II cytoskeleton 78 and each amino acid | | | | |
| amino acid | essential amino acid | | | non-essential amino acid | |
| | Met | His | Trp | PCA | Orn |
| presence or absence of correlation | ○ | ○ | ○ | ○ | ○ |

| | | | | | |
|---|---|---|---|---|---|
| [set threshold] essential amino acid: strong correlation 0.5, correlation 0.4; non-essential amino acid: strong correlation 0.7, correlation 0.6 | | | | | |

**[Table 7-5]**

| melanin content 20 - 39 years old | | | |
|---|---|---|---|
| | presence or absence of correlation between keratin type II cytoskeleton 80 and each amino acid | | |
| amino acid | essential amino acid | non-essential amino acid | |
| | Thr | UCA | PCA |
| presence or absence of correlation | ○ | ○ | ⊙ |

| | | | |
|---|---|---|---|
| [set threshold] essential amino acid: strong correlation 0.4, correlation 0.3; non-essential amino acid: strong correlation 0.6, correlation 0.5 | | | |

**[Table 7-6]**

| melanin content 20 - 39 years old | | | | | | | | |
|---|---|---|---|---|---|---|---|---|
| | presence or absence of correlation between lysosome C and each amino acid | | | | | | | |
| amino acid | essential amino acid | | | | | non-essential amino acid | | |
| | Val | Ile | Leu | Phe | Trp | Ala | Ser | Tau |
| presence or absence of correlation | ⊙ | ⊙ | ⊙ | ⊙ | ⊙ | ○ | ○ | ○ |

| | | | | | | | | |
|---|---|---|---|---|---|---|---|---|
| [set threshold] essential amino acid: strong correlation 0.6, correlation 0.5; non-essential amino acid: strong correlation 0.7, correlation 0.6 | | | | | | | | |

**[Table 7-7]**

| melanin content 20 - 39 years old | | | | | | |
|---|---|---|---|---|---|---|
| | presence or absence of correlation between neuroblast differentiation-related protein AHNAK and each amino acid | | | | | |
| amino acid | essential amino acid | | | | non-essential amino acid | |
| | Val | Met | His | Trp | Asn | Orn |
| presence or absence of correlation | ○ | ○ | ⊙ | ⊙ | ⊙ | ○ |

| | | | | | | |
|---|---|---|---|---|---|---|
| [set threshold] essential amino acid: strong correlation 0.5, correlation 0.4; non-essential amino acid: strong correlation 0.7, correlation 0.6 | | | | | | |

**[Table 7-8]**

| melanin content 20 - 39 years old | | | |
|---|---|---|---|
| | presence or absence of correlation between protein-glutamine γ-glutamyltransferase E and each amino acid | | |
| amino acid | essential amino acid | | non-essential amino acid |
| | Val | Trp | Orn |
| presence or absence of correlation | ○ | ⊙ | ○ |

| | | | |
|---|---|---|---|
| [set threshold] essential amino acid: strong correlation 0.4, correlation 0.3; non-essential amino acid: strong correlation 0.7, correlation 0.6 | | | |

**[Table 7-9]**

| melanin content 20 - 39 years old | | | |
|---|---|---|---|
| | presence or absence of correlation between thioredoxin and each amino acid | | |
| amino acid | non-essential amino acid | | |
| | PCA | Ser | Asn |
| presence or absence of correlation | ○ | ○ | ○ |

| | | | |
|---|---|---|---|
| [set threshold] non-essential amino acid: strong correlation 0.5, correlation 0.4 | | | |

**[Table 7-10]**

| melanin content 20 - 39 years old | |
|---|---|
| | presence or absence of correlation between zinc-α-2-glycoprotein and each amino acid |
| amino acid | non-essential amino acid |
| | Asn |
| presence or absence of correlation | ○ |

| | |
|---|---|
| [set threshold] non-essential amino acid: strong correlation 0.6, correlation 0.5 | |

**[Table 7-11]**

| melanin content 40 - 62 years old | | | | | | |
|---|---|---|---|---|---|---|
| | presence or absence of correlation between cystatin-M and each amino acid | | | | | |
| amino acid | essential amino acid | | | | non-essential amino acid | |
| | Leu | Met | Phe | Trp | Asn | Gln |
| presence or absence of correlation | ⊙ | ⊙ | ○ | ○ | ○ | ○ |

| | | | | | | |
|---|---|---|---|---|---|---|
| [set threshold] essential amino acid: strong correlation 0.5, correlation 0.4; non-essential amino acid: strong correlation 0.5, correlation 0.4 | | | | | | |

**[Table 7-12]**

| melanin content 40 - 62 years old | | |
|---|---|---|
| | presence or absence of correlation between protein S100-A8 and each amino acid | |
| amino acid | essential amino acid | |
| | Leu | Met |
| presence or absence of correlation | ○ | ○ |

| | | |
|---|---|---|
| [set threshold] essential amino acid: strong correlation 0.4, correlation 0.3 | | |

From the results of Table 7-1, it was shown that specific amino acids (i.e., Leu, Met, Phe, Trp, PCA, and Asn) are correlated with desmoglein-1, which is a protein important for spots. Among these, the essential amino acids Leu, Met, Phe, and Trp are considered to be more strongly involved in protein production. These results suggest that the above-mentioned specific amino acids promote the production of desmoglein-1, and are therefore effective in improving spots (especially spots in younger people).

From the results of Table 7-2, it was shown that specific amino acids (i.e., Val, Ile, Lys, Met, PCA, Ala, and Asn) are correlated with γ-glutamyl cyclotransferase, which is a protein important for spots. Among these, the essential amino acids Val, Ile, Lys, and Met are considered to be more strongly involved in protein production. These results suggest that the above-mentioned specific amino acids promote the production of γ-glutamyl cyclotransferase, and are therefore effective in improving spots (especially spots in younger people).

From the results of Table 7-3, it was shown that specific amino acids (i.e., Val, Lys, and His) are correlated with keratin type II cytoskeleton 5, which is a protein important for spots. These results suggest that the above-mentioned specific amino acids promote the production of keratin type II cytoskeleton 5, and are therefore effective in improving spots (especially spots in younger people).

From the results of Table 7-4, it was shown that specific amino acids (i.e., Met, His, Trp, PCA, and Orn) are correlated with keratin type II cytoskeleton 78, which is a protein important for spots. Among these, the essential amino acids Met, His, and Trp are considered to be more strongly involved in protein production. These results suggest that the above-mentioned specific amino acids promote the production of keratin type II cytoskeleton 78, and are therefore effective in improving spots (especially spots in younger people).

From the results of Table 7-5, it was shown that specific amino acids (i.e., Thr, UCA, and PCA) are correlated with keratin type II cytoskeleton 80, which is a protein important for spots. Among these, the essential amino acid Thr is considered to be more strongly involved in protein production. These results suggest that the above-mentioned specific amino acids promote the production of keratin type II cytoskeleton 80, and are therefore effective in improving spots (especially spots in younger people).

From the results of Table 7-6, it was shown that specific amino acids (i.e., Val, Ile, Leu, Phe, Trp, Ala, Ser, and Tau) are correlated with lysosome C, which is a protein important for spots. Among these, the essential amino acids Val, Ile, Leu, Phe, and Trp are considered to be more strongly involved in protein production. These results suggest that the above-mentioned specific amino acids promote the production of lysosome C, and are therefore effective in improving spots (especially spots in younger people).

From the results of Table 7-7, it was shown that specific amino acids (i.e., Val, Met, His, Trp, Asn, and Orn) are correlated with neuroblast differentiation-related protein AHNAK, which is a protein important for spots. Among these, the essential amino acids Val, Met, His, and Trp are considered to be more strongly involved in protein production. These results suggest that the above-mentioned specific amino acids promote the production of neuroblast differentiation-related protein AHNAK, and are therefore effective in improving spots (especially spots in younger people).

From the results of Table 7-8, it was shown that specific amino acids (i.e., Val, Trp, and Orn) are correlated with protein-glutamine γ-glutamyltransferase E, which is a protein important for spots. Among these, the essential amino acids Val and Trp are considered to be more strongly involved in protein production. These results suggest that the above-mentioned specific amino acids promote the production of protein-glutamine γ-glutamyltransferase E, and are therefore effective in improving spots (especially spots in younger people).

From the results of Table 7-9, it was shown that specific amino acids (i.e., PCA, Ser, and Asn) are correlated with thioredoxin, which is a protein important for spots. These results suggest that the above-mentioned specific amino acids promote the production of thioredoxin, and are therefore effective in improving spots (especially spots in younger people).

From the results of Table 7-10, it was shown that a specific amino acid (i.e., Asn) is correlated with zinc-α-2-glycoprotein, which is a protein important for spots. The result suggests that the above-mentioned specific amino acid promotes the production of zinc-α-2-glycoprotein, and is therefore effective in improving spots (especially spots in younger people).

From the results of Table 7-11, it was shown that specific amino acids (i.e., Leu, Met, Phe, Trp, Asn, and Gln) are correlated with cystatin-M, which is a protein important for spots. Among these, the essential amino acids Leu, Met, Phe, and Trp are considered to be more strongly involved in protein production. These results suggest that the above-mentioned specific amino acids promote the production of cystatin-M, and are therefore effective in improving spots (especially spots in older people).

From the results of Table 7-12, it was shown that specific amino acids (i.e., Leu and Met) are correlated with protein S100-A8, which is a protein important for spots. These results suggest that the above-mentioned specific amino acids promote the production of protein S100-A8, and are therefore effective in improving spots (especially spots in older people).

**[Table 8-1]**

| transepidermal water transpiration amount 20 - 39 years old | | | | | |
|---|---|---|---|---|---|
| | presence or absence of correlation between caspase-14 and each amino acid | | | | |
| amino acid | essential amino acid | | | non-essential amino acid | |
| | Val | Leu | His | PCA | Tau |
| presence or absence of correlation | ⊙ | ○ | ⊙ | ⊙ | ○ |

| | | | | | |
|---|---|---|---|---|---|
| [set threshold] essential amino acid: strong correlation 0.5, correlation 0.4; non-essential amino acid: strong correlation 0.5, correlation 0.4 | | | | | |

**[Table 8-2]**

| transepidermal water transpiration amount 20 - 39 years old | | | | |
|---|---|---|---|---|
| | presence or absence of correlation between keratin type II cuticle Hb5 and each amino acid | | | |
| amino acid | essential amino acid | | non-essential amino acid | |
| | Leu | Phe | Orn | Arg |
| presence or absence of correlation | ○ | ○ | ○ | ⊙ |

| | | | | |
|---|---|---|---|---|
| [set threshold] essential amino acid: strong correlation 0.4, correlation 0.3; non-essential amino acid: strong correlation 0.7, correlation 0.6 | | | | |

**[Table 8-3]**

| transepidermal water transpiration amount 20 - 39 years old | | | |
|---|---|---|---|
| | presence or absence of correlation between keratin type II cytoskeleton 1b and each amino acid | | |
| amino acid | essential amino acid | | non-essential amino acid |
| | His | Trp | Pro |
| presence or absence of correlation | ○ | ○ | ○ |

| | | | |
|---|---|---|---|
| [set threshold] essential amino acid: strong correlation 0.5, correlation 0.4; non-essential amino acid: strong correlation 0.7, correlation 0.6 | | | |

**[Table 8-4]**

| transepidermal water transpiration amount 20 - 39 years old | | | | | | |
|---|---|---|---|---|---|---|
| | presence or absence of correlation between phosphatidylethanolamine-binding protein 1 and each amino acid | | | | | |
| amino acid | essential amino acid | | | | non-essential amino acid | |
| | Val | Lys | His | PCA | Tau | Glu |
| presence or absence of correlation | ○ | ⊙ | ○ | ○ | ○ | ○ |

| | | | | | | |
|---|---|---|---|---|---|---|
| [set threshold] essential amino acid: strong correlation 0.5, correlation 0.4; non-essential amino acid: strong correlation 0.5, correlation 0.4 | | | | | | |

**[Table 8-5]**

| transepidermal water transpiration amount 40 - 62 years old | | | | | | |
|---|---|---|---|---|---|---|
| | presence or absence of correlation between hornerin and each amino acid | | | | | |
| amino acid | essential amino acid | | | non-essential amino acid | | |
| | Leu | His | Trp | Gly | Pro | Cit |
| presence or absence of correlation | ⊙ | ○ | ⊙ | ⊙ | ○ | ○ |

| | | | | | | |
|---|---|---|---|---|---|---|
| [set threshold] essential amino acid: strong correlation 0.4, correlation 0.3; non-essential amino acid: strong correlation 0.5, correlation 0.4 | | | | | | |

**[Table 8-6]**

| transepidermal water transpiration amount 40 - 62 years old | | | | |
|---|---|---|---|---|
| | presence or absence of correlation between keratin type I cuticle Ha1 and each amino acid | | | |
| amino acid | essential amino acid | non-essential amino acid | | |
| | Met | Tau | Gln | PCA |
| presence or absence of correlation | ○ | ⊙ | ○ | ○ |

| | | | | |
|---|---|---|---|---|
| [set threshold] essential amino acid: strong correlation 0.5, correlation 0.4; non-essential amino acid: strong correlation 0.5, correlation 0.4 | | | | |

**[Table 8-7]**

| transepidermal water transpiration amount 40 - 62 years old | | | |
|---|---|---|---|
| | presence or absence of correlation between keratin type I cuticle Ha3-I and each amino acid | | |
| amino acid | essential amino acid | non-essential amino acid | |
| | Met | Tau | Orn |
| presence or absence of correlation | ○ | ○ | ○ |

| | | | |
|---|---|---|---|
| [set threshold] essential amino acid: strong correlation 0.4, correlation 0.3; non-essential amino acid: strong correlation 0.6, correlation 0.5 | | | |

**[Table 8-8]**

| transepidermal water transpiration amount 40 - 62 years old | | | | | | | |
|---|---|---|---|---|---|---|---|
| | presence or absence of correlation between keratin type II cuticle Hb3 and each amino acid | | | | | | |
| amino acid | essential amino acid | | | | non-essential amino acid | | |
| | Thr | Met | His | Phe | Tau | UCA | PCA |
| presence or absence of correlation | ○ | ⊙ | ⊙ | ○ | ○ | ⊙ | ○ |

| | | | | | | | |
|---|---|---|---|---|---|---|---|
| [set threshold] essential amino acid: strong correlation 0.6, correlation 0.5; non-essential amino acid: strong correlation 0.7, correlation 0.6 | | | | | | | |

**[Table 8-9]**

| transepidermal water transpiration amount 40 - 62 years old | | | |
|---|---|---|---|
| | presence or absence of correlation between keratin type II cuticle Hb6 and each amino acid | | |
| amino acid | essential amino acid | non-essential amino acid | |
| | Phe | Tau | PCA |
| presence or absence of correlation | ○ | ⊙ | ○ |

| | | | |
|---|---|---|---|
| [set threshold] essential amino acid: strong correlation 0.5, correlation 0.4; non-essential amino acid: strong correlation 0.5, correlation 0.4 | | | |

**[Table 8-10]**

| transepidermal water transpiration amount 40 - 62 years old | | | |
|---|---|---|---|
| | presence or absence of correlation between thioredoxin and each amino acid | | |
| amino acid | essential amino acid | | |
| | Leu | His | Phe |
| presence or absence of correlation | ○ | ○ | ○ |

| | | | |
|---|---|---|---|
| [set threshold] essential amino acid: strong correlation 0.4, correlation 0.3 | | | |

From the results of Table 8-1, it was shown that specific amino acids (i.e., Val, Leu, His, PCA, and Tau) are correlated with caspase-14, which is a protein important for barrier function. Among these, the essential amino acids Val, Leu, and His are considered to be more strongly involved in protein production. These results suggest that the above-mentioned specific amino acid promotes the production of caspase-14, and are therefore effective in improving a decrease in barrier function (especially a decrease in barrier function in younger people).

From the results of Table 8-2, it was shown that specific amino acids (i.e., Leu, Phe, Orn, and Arg) are correlated with keratin type II cuticle Hb5, which is a protein important for barrier function. Among these, the essential amino acids Leu and Phe are considered to be more strongly involved in protein production. These results suggest that the above-mentioned specific amino acid promotes the production of keratin type II cuticle Hb5, and are therefore effective in improving a decrease in barrier function (especially a decrease in barrier function in younger people).

From the results of Table 8-3, it was shown that specific amino acids (i.e., His, Trp, and Pro) are correlated with keratin type II cytoskeleton 1b, which is a protein important for barrier function. Among these, the essential amino acids His and Trp are considered to be more strongly involved in protein production. These results suggest that the above-mentioned specific amino acid promotes the production of keratin type II cytoskeleton 1b, and are therefore effective in improving a decrease in barrier function (especially a decrease in barrier function in younger people).

From the results of Table 8-4, it was shown that specific amino acids (i.e., Val, Lys, His, PCA, Tau, and Glu) are correlated with phosphatidylethanolamine-binding protein 1, which is a protein important for barrier function. Among these, the essential amino acids Val, Lys, and His are considered to be more strongly involved in protein production. These results suggest that the above-mentioned specific amino acid promotes the production of phosphatidylethanolamine-binding protein 1, and are therefore effective in improving a decrease in barrier function (especially a decrease in barrier function in younger people).

From the results of Table 8-5, it was shown that specific amino acids (i.e., Leu, His, Trp, Gly, Pro, and Cit) are correlated with hornerin, which is a protein important for barrier function. Among these, the essential amino acids Leu, His, and Trp are considered to be more strongly involved in protein production. These results suggest that the above-mentioned specific amino acid promotes the production of hornerin, and are therefore effective in improving a decrease in barrier function (especially a decrease in barrier function in older people).

From the results of Table 8-6, it was shown that specific amino acids (i.e., Met, Tau, Gln, and PCA) are correlated with keratin type I cuticle Ha1, which is a protein important for barrier function. Among these, the essential amino acid Met is considered to be more strongly involved in protein production. These results suggest that the above-mentioned specific amino acid promotes the production of keratin type I cuticle Ha1, and are therefore effective in improving a decrease in barrier function (especially a decrease in barrier function in older people).

From the results of Table 8-7, it was shown that specific amino acids (i.e., Met, Tau, and Orn) are correlated with keratin type I cuticle Ha3-I, which is a protein important for barrier function. Among these, the essential amino acid Met is considered to be more strongly involved in protein production. These results suggest that the above-mentioned specific amino acid promotes the production of keratin type I cuticle Ha3-I, and are therefore effective in improving a decrease in barrier function (especially a decrease in barrier function in older people).

From the results of Table 8-8, it was shown that specific amino acids (i.e., Thr, Met, His, Phe, Tau, UCA, and PCA) are correlated with keratin type II cuticle Hb3, which is a protein important for barrier function. Among these, the essential amino acids Thr, Met, His, and Phe are considered to be more strongly involved in protein production. These results suggest that the above-mentioned specific amino acid promotes the production of keratin type II cuticle Hb3, and are therefore effective in improving a decrease in barrier function (especially a decrease in barrier function in older people).

From the results of Table 8-9, it was shown that specific amino acids (i.e., Phe, Tau, and PCA) are correlated with keratin type II cuticle Hb6, which is a protein important for barrier function. Among these, the essential amino acid Phe is considered to be more strongly involved in protein production. These results suggest that the above-mentioned specific amino acid promotes the production of keratin type II cuticle Hb6, and are therefore effective in improving a decrease in barrier function (especially a decrease in barrier function in older people).

From the results of Table 8-10, it was shown that specific amino acids (i.e., Leu, His, and Phe) are correlated with thioredoxin, which is a protein important for barrier function. These results suggest that the above-mentioned specific amino acid promotes the production of thioredoxin, and are therefore effective in improving a decrease in barrier function (especially a decrease in barrier function in older people).

**[Table 9-1]**

| pH 20 - 39 years old | |
|---|---|
| | presence or absence of correlation between 78 kDa glucose regulated protein and each amino acid |
| amino acid | non-essential amino acid |
| | Orn |
| presence or absence of correlation | ○ |

| | |
|---|---|
| [set threshold] essential amino acid: strong correlation 0.6, correlation 0.5 | |

**[Table 9-2]**

| pH 20 - 39 years old | | |
|---|---|---|
| | presence or absence of correlation between actin cytoplasmic 2 and each amino acid | |
| amino acid | non-essential amino acid | |
| | Asn | Orn |
| presence or absence of correlation | ○ | ○ |

| | | |
|---|---|---|
| [set threshold] non-essential amino acid: strong correlation 0.5, correlation 0.4 | | |

**[Table 9-3]**

| pH 20 - 39 years old | | | | | |
|---|---|---|---|---|---|
| | presence or absence of correlation between α-actin-4 and each amino acid | | | | |
| amino acid | essential amino acid | | | non-essential amino acid | |
| | Leu | Lys | Phe | UCA | Glu |
| presence or absence of correlation | ⊙ | ⊙ | ⊙ | ⊙ | ○ |

| | | | | | |
|---|---|---|---|---|---|
| [set threshold] essential amino acid: strong correlation 0.6, correlation 0.5; non-essential amino acid: strong correlation 0.6, correlation 0.5 | | | | | |

**[Table 9-4]**

| pH 20 - 39 years old | | | | | |
|---|---|---|---|---|---|
| | presence or absence of correlation between apolipoprotein D and each amino acid | | | | |
| amino acid | essential amino acid | | | non-essential amino acid | |
| | Ile | Lys | Phe | Asp | Arg |
| presence or absence of correlation | ⊙ | ⊙ | ○ | ○ | ⊙ |

| | | | | | |
|---|---|---|---|---|---|
| [set threshold] essential amino acid: strong correlation 0.4, correlation 0.3; non-essential amino acid: strong correlation 0.5, correlation 0.4 | | | | | |

**[Table 9-5]**

| pH 20 - 39 years old | | | | | | |
|---|---|---|---|---|---|---|
| | presence or absence of correlation between desmoglein-1 and each amino acid | | | | | |
| amino acid | essential amino acid | | | | non-essential amino acid | |
| | Leu | Met | Phe | Trp | PCA | Asn |
| presence or absence of correlation | ○ | ○ | ○ | ○ | ○ | ○ |

| | | | | | | |
|---|---|---|---|---|---|---|
| [set threshold] essential amino acid: strong correlation 0.4, correlation 0.3; non-essential amino acid: strong correlation 0.5, correlation 0.4 | | | | | | |

**[Table 9-6]**

| pH 20 - 39 years old | | | | |
|---|---|---|---|---|
| | presence or absence of correlation between epiplakin and each amino acid | | | |
| amino acid | essential amino acid | | non-essential amino acid | |
| | Thr | Met | PCA | Arg |
| presence or absence of correlation | ⊙ | ⊙ | ○ | ○ |

| | | | | |
|---|---|---|---|---|
| [set threshold] essential amino acid: strong correlation 0.6, correlation 0.5; non-essential amino acid: strong correlation 0.5, correlation 0.4 | | | | |

**[Table 9-7]**

| pH 20 - 39 years old | | |
|---|---|---|
| | presence or absence of correlation between filaggrin and each amino acid | |
| amino acid | essential amino acid | |
| | Val | Ile |
| presence or absence of correlation | ○ | ⊙ |

| | | |
|---|---|---|
| [set threshold] essential amino acid: strong correlation 0.4, correlation 0.3 | | |

**[Table 9-8]**

| pH 20 - 39 years old | |
|---|---|
| | presence or absence of correlation between heat shock protein β-1 and each amino acid |
| amino acid | non-essential amino acid |
| | Orn |
| presence or absence of correlation | ○ |

| | |
|---|---|
| [set threshold] non-essential amino acid: strong correlation 0.5, correlation 0.4 | |

**[Table 9-9]**

| pH 20 - 39 years old | | | | | |
|---|---|---|---|---|---|
| | presence or absence of correlation between interleukin-36γ and each amino acid | | | | |
| amino acid | essential amino acid | | | | non-essential amino acid |
| | Ile | Leu | His | Phe | Cit |
| presence or absence of correlation | ○ | ⊙ | ○ | ⊙ | ○ |

| | | | | | |
|---|---|---|---|---|---|
| [set threshold] essential amino acid: strong correlation 0.4, correlation 0.3; non-essential amino acid: strong correlation 0.5, correlation 0.4 | | | | | |

**[Table 9-10]**

| pH 20 - 39 years old | | | | | |
|---|---|---|---|---|---|
| | presence or absence of correlation between protein S100-P and each amino acid | | | | |
| amino acid | essential amino acid | | | non-essential amino acid | |
| | Leu | Phe | Trp | Pro | Glu |
| presence or absence of correlation | ⊙ | ○ | ⊙ | ⊙ | ○ |

| | | | | | |
|---|---|---|---|---|---|
| [set threshold] essential amino acid: strong correlation 0.5, correlation 0.4; non-essential amino acid: strong correlation 0.6, correlation 0.5 | | | | | |

**[Table 9-11]**

| pH 40 - 62 years old | | | | | |
|---|---|---|---|---|---|
| | presence or absence of correlation between α-enolase and each amino acid | | | | |
| amino acid | essential amino acid | | | non-essential amino acid | |
| | Thr | Leu | Trp | Gln | Glu |
| presence or absence of correlation | ○ | ⊙ | ⊙ | ○ | ○ |

| | | | | | |
|---|---|---|---|---|---|
| [set threshold] essential amino acid: strong correlation 0.5, correlation 0.4; non-essential amino acid: strong correlation 0.6, correlation 0.5 | | | | | |

**[Table 9-12]**

| pH 40 - 62 years old | | | | | | | |
|---|---|---|---|---|---|---|---|
| | presence or absence of correlation between keratin type II cuticle Hb3 and each amino acid | | | | | | |
| amino acid | essential amino acid | | | | non-essential amino acid | | |
| | Thr | Met | His | Phe | Tau | UCA | PCA |
| presence or absence of correlation | ○ | ⊙ | ⊙ | ○ | ○ | ⊙ | ○ |

| | | | | | | | |
|---|---|---|---|---|---|---|---|
| [set threshold] essential amino acid: strong correlation 0.6, correlation 0.5; non-essential amino acid: strong correlation 0.7, correlation 0.6 | | | | | | | |

**[Table 9-13]**

| pH 40 - 62 years old | | | | |
|---|---|---|---|---|
| | presence or absence of correlation between keratin type II cytoskeleton 75 and each amino acid | | | |
| amino acid | essential amino acid | | | non-essential amino acid |
| | Leu | His | Trp | Tau |
| presence or absence of correlation | ○ | ○ | ⊙ | ○ |

| | | | | |
|---|---|---|---|---|
| [set threshold] essential amino acid: strong correlation 0.4, correlation 0.3; non-essential amino acid: strong correlation 0.6, correlation 0.5 | | | | |

**[Table 9-14]**

| pH 40 - 62 years old | | | | | | |
|---|---|---|---|---|---|---|
| | presence or absence of correlation between protein-glutamine γ-glutamyltransferase K and each amino acid | | | | | |
| amino acid | essential amino acid | | | | non-essential amino acid | |
| | Val | Thr | Ile | His | Ser | Orn |
| presence or absence of correlation | ○ | ○ | ○ | ○ | ○ | ○ |

| | | | | | | |
|---|---|---|---|---|---|---|
| [set threshold] essential amino acid: strong correlation 0.4, correlation 0.3; non-essential amino acid: strong correlation 0.6, correlation 0.5 | | | | | | |

**[Table 9-15]**

| pH 40 - 62 years old | | | | |
|---|---|---|---|---|
| | presence or absence of correlation between serpin B3 and each amino acid | | | |
| amino acid | essential amino acid | | non-essential amino acid | |
| | Val | Leu | Asn | Gln |
| presence or absence of correlation | ○ | ○ | ○ | ○ |

| | | | | |
|---|---|---|---|---|
| [set threshold] essential amino acid: strong correlation 0.4, correlation 0.3; non-essential amino acid: strong correlation 0.5, correlation 0.4 | | | | |

**[Table 9-16]**

| pH 40 - 62 years old | | | | |
|---|---|---|---|---|
| | presence or absence of correlation between triose phosphoric acid isomerase and each amino acid | | | |
| amino acid | essential amino acid | | non-essential amino acid | |
| | Thr | Leu | Asn | Gln |
| presence or absence of correlation | ○ | ○ | ○ | ⊙ |

| | | | | |
|---|---|---|---|---|
| [set threshold] essential amino acid: strong correlation 0.4, correlation 0.3; non-essential amino acid: strong correlation 0.5, correlation 0.4 | | | | |

From the results of Table 9-1, it was shown that a specific amino acid (i.e., Orn) is correlated with 78 kDa glucose regulated protein, which is a protein important for pH value of the skin. The result suggests that the above-mentioned specific amino acid promotes the production of 78 kDa glucose regulated protein, and is therefore effective in improving abnormal pH value of the skin (especially abnormal pH value of the skin in younger people).

From the results of Table 9-2, it was shown that specific amino acids (i.e., Asn and Orn) are correlated with actin cytoplasmic 2, which is a protein important for pH value of the skin. These results suggest that the above-mentioned specific amino acids promote the production of actin cytoplasmic 2, and are therefore effective in improving abnormal pH value of the skin (especially abnormal pH value of the skin in younger people).

From the results of Table 9-3, it was shown that specific amino acids (i.e., Leu, Lys, Phe, UCA, and Glu) are correlated with α-actin-4, which is a protein important for pH value of the skin. Among these, the essential amino acids Leu, Lys, and Phe are considered to be more strongly involved in protein production. These results suggest that the above-mentioned specific amino acids promote the production of α-actin-4, and are therefore effective in improving abnormal pH value of the skin (especially abnormal pH value of the skin in younger people).

From the results of Table 9-4, it was shown that specific amino acids (i.e., Ile, Lys, Phe, Asp, and Arg) are correlated with apolipoprotein D, which is a protein important for pH value of the skin. Among these, the essential amino acids Ile, Lys, and Phe are considered to be more strongly involved in protein production. These results suggest that the above-mentioned specific amino acids promote the production of apolipoprotein D, and are therefore effective in improving abnormal pH value of the skin (especially abnormal pH value of the skin in younger people).

From the results of Table 9-5, it was shown that specific amino acids (i.e., Leu, Met, Phe, Trp, PCA, and Asn) are correlated with desmoglein-1, which is a protein important for pH value of the skin. Among these, the essential amino acids Leu, Met, Phe, and Trp are considered to be more strongly involved in protein production. These results suggest that the above-mentioned specific amino acids promote the production of desmoglein-1, and are therefore effective in improving abnormal pH value of the skin (especially abnormal pH value of the skin in younger people).

From the results of Table 9-6, it was shown that specific amino acids (i.e., Thr, Met, PCA, and Arg) are correlated with epiplakin, which is a protein important for pH value of the skin. Among these, the essential amino acids Thr and Met are considered to be more strongly involved in protein production. These results suggest that the above-mentioned specific amino acids promote the production of epiplakin, and are therefore effective in improving abnormal pH value of the skin (especially abnormal pH value of the skin in younger people).

From the results of Table 9-7, it was shown that specific amino acids (i.e., Val and Ile) are correlated with filaggrin, which is a protein important for pH value of the skin. These results suggest that the above-mentioned specific amino acids promote the production of filaggrin, and are therefore effective in improving abnormal pH value of the skin (especially abnormal pH value of the skin in younger people).

From the results of Table 9-8, it was shown that a specific amino acid (i.e., Orn) is correlated with heat shock protein β-1, which is a protein important for pH value of the skin. The result suggests that the above-mentioned specific amino acid promotes the production of heat shock protein β-1, and is therefore effective in improving abnormal pH value of the skin (especially abnormal pH value of the skin in younger people).

From the results of Table 9-9, it was shown that specific amino acids (i.e., Ile, Leu, His, Phe, and Cit) are correlated with interleukin-36γ, which is a protein important for pH value of the skin. Among these, the essential amino acids Ile, Leu, His, and Phe are considered to be more strongly involved in protein production. These results suggest that the above-mentioned specific amino acids promote the production of interleukin-36y, and are therefore effective in improving abnormal pH value of the skin (especially abnormal pH value of the skin in younger people).

From the results of Table 9-10, it was shown that specific amino acids (i.e., Leu, Phe, Trp, Pro, and Glu) are correlated with protein S100-P, which is a protein important for pH value of the skin. Among these, the essential amino acids Leu, Phe, and Trp are considered to be more strongly involved in protein production. These results suggest that the above-mentioned specific amino acids promote the production of protein S100-P, and are therefore effective in improving abnormal pH value of the skin (especially abnormal pH value of the skin in younger people).

From the results of Table 9-11, it was shown that specific amino acids (i.e., Thr, Leu, Trp, Gln, and Glu) are correlated with α-enolase, which is a protein important for pH value of the skin. Among these, the essential amino acids Thr, Leu, and Trp are considered to be more strongly involved in protein production. These results suggest that the above-mentioned specific amino acids promote the production of α-enolase, and are therefore effective in improving abnormal pH value of the skin (especially abnormal pH value of the skin in older people).

From the results of Table 9-12, it was shown that specific amino acids (i.e., Thr, Met, His, Phe, Tau, UCA, and PCA) are correlated with keratin type II cuticle Hb3, which is a protein important for pH value of the skin. Among these, the essential amino acids Thr, Met, His, and Phe are considered to be more strongly involved in protein production. These results suggest that the above-mentioned specific amino acids promote the production of keratin type II cuticle Hb3, and are therefore effective in improving abnormal pH value of the skin (especially abnormal pH value of the skin in older people).

From the results of Table 9-13, it was shown that specific amino acids (i.e., Leu, His, Trp, and Tau) are correlated with keratin type II cytoskeleton 75, which is a protein important for pH value of the skin. Among these, the essential amino acids Leu, His, and Trp are considered to be more strongly involved in protein production. These results suggest that the above-mentioned specific amino acids promote the production of keratin type II cytoskeleton 75, and are therefore effective in improving abnormal pH value of the skin (especially abnormal pH value of the skin in older people).

From the results of Table 9-14, it was shown that specific amino acids (i.e., Val, Thr, Ile, His, Ser, and Orn) are correlated with protein-glutamine γ-glutamyltransferase K, which is a protein important for pH value of the skin. Among these, the essential amino acids Val, Thr, Ile, and His are considered to be more strongly involved in protein production. These results suggest that the above-mentioned specific amino acids promote the production of protein-glutamine γ-glutamyltransferase K, and are therefore effective in improving abnormal pH value of the skin (especially abnormal pH value of the skin in older people).

From the results of Table 9-15, it was shown that specific amino acids (i.e., Val, Leu, Asn, and Gln) are correlated with serpin B3, which is a protein important for pH value of the skin. Among these, the essential amino acids Val and Leu are considered to be more strongly involved in protein production. These results suggest that the above-mentioned specific amino acids promote the production of serpin B3, and are therefore effective in improving abnormal pH value of the skin (especially abnormal pH value of the skin in older people).

From the results of Table 9-16, it was shown that specific amino acids (i.e., Thr, Leu, Asn, and Gln) are correlated with triose phosphoric acid isomerase, which is a protein important for pH value of the skin. Among these, the essential amino acids Thr and Leu are considered to be more strongly involved in protein production. These results suggest that the above-mentioned specific amino acids promote the production of triose phosphoric acid isomerase, and are therefore effective in improving abnormal pH value of the skin (especially abnormal pH value of the skin in older people).

In the present invention, it is considered that the production of protein in the epidermis is promoted and the amount of protein in the stratum corneum is promoted, by applying, to the skin, an amino acid that has a positive or negative correlation with the amount of protein in the stratum corneum.

### [Experimental Example 2] Influence of specific amino acid on the production of protein related to skin conditions of epidermal keratinocytes

### (Explanation relating to construction of experimental system)

Culture media (e.g., D-MEM medium) used for cell culture contain amino acids necessary for cells to survive and grow. On the other hand, in epidermal keratinocytes that make up human skin, it is possible that the necessary amount of amino acids does not reach the cells due to aging and external influences such as dryness. In the following Experimental Examples 2-1, 2-2, and 2-3, media with the following compositions (human skin-simulating medium 1, human skin-simulating medium 2), in which the essential amino acids contained in D-MEM medium were reduced to 30% or 20%, were used as media simulating a situation in which the amount of amino acids in human skin was depleted.

Human skin-simulating medium 1: Essential amino acids in D-MEM (Life Technologies 21068-028) were changed to the concentrations shown in Table 10, human skin-simulating medium 1. Furthermore, calcium chloride (amount to achieve concentration 2 mM in the medium), sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), and antibiotics (amount to achieve penicillin concentration 50 units/mL in the medium and streptomycin concentration 50 µg/mL in the medium) were added.

human skin-simulating medium 2: The essential amino acids in D-MEM (Life Technologies 21068-028) were changed to the concentrations shown in Table 10, human skin-simulating medium 2. Furthermore, calcium chloride (amount to achieve concentration 2 mM in the medium), sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), and antibiotics (amounts to achieve concentration 50 units/mL of penicillin in the medium and 50 µg/mL of streptomycin in the medium).

**[Table 10]**

| essential amino acid | concentration (mM) | |
|---|---|---|
| | human skin-simulating medium 1 | human skin-simulating medium 2 |
| His | 0.06 | 0.04 |
| Ile | 0.24 | 0.16 |
| Leu | 0.24 | 0.16 |
| Lys | 0.24 | 0.16 |
| Met | 0.06 | 0.04 |
| Phe | 0.12 | 0.08 |
| Thr | 0.24 | 0.16 |
| Trp | 0.024 | 0.016 |
| Val | 0.24 | 0.16 |

### [Experimental Example 2-1] Influence of specific amino acids (Leu, Met, Phe, Trp) on desmoglein-1 production in epidermal keratinocytes

Among the amino acids shown to be correlated with desmoglein-1 in the results of Experimental Example 1 (Table 6-4, Table 7-1, Table 9-5), the following experiment was conducted to confirm the effect of the essential amino acids Leu, Met, Phe, and Trp in promoting the production of desmoglein-1 in epidermal keratinocytes.

1. Epidermal keratinocytes were cultured in a medium obtained by adding sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), antibiotics (amount to achieve penicillin concentration 50 units/mL in the medium and streptomycin concentration 50 µg/mL in the medium), and calcium chloride (amount to achieve concentration 0.03 mM in the medium) to D-MEM (Life Technologies 21068-028) (hereinafter this medium is referred to as a low calcium medium).
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (1) or (2).
   Evaluation condition (1): Human skin-simulating medium 1 (hereinafter the sample evaluated under evaluation condition (1) is referred to as "Control 1").
   Evaluation condition (2): Medium obtained by adding Leu (final concentration 0.8 mM), Met (final concentration 0.2 mM), Phe (final concentration 0.4 mM), and Trp (final concentration 0.08 mM) to human skin-simulating medium 1 (hereinafter the sample evaluated under evaluation condition (2) is referred to as "Example 1").
   Human skin-simulating medium 1 was set to a high calcium concentration (concentration 2 mM in the medium) in order to produce experimental conditions under which differentiation was induced at the same time as the evaluation began, because desmoglein-1 is a protein that is expressed in association with cell differentiation induced by high calcium concentrations.
4. After culturing the sample of the above-mentioned 3 for 5 days, a protein extract (extract containing membrane and membrane-associated proteins) was prepared using Mem-PER Plus (Thermo Fisher Scientific 89842) and Halt Protease Inhibitor Cocktail (Thermo Fisher Scientific 87785), and desmoglein-1 was quantified using a commercially available ELISA kit (Cloud-Clone Corp SEA729Hu).
5. The protein extract of the above-mentioned 4 was diluted with water, and the protein was quantified using Micro BCA Protein Assay Kit (Thermo Fisher Scientific 23235).
6. The amount of desmoglein-1 determined in the above-mentioned 4 was normalized by the amount of protein determined in the above-mentioned 5.
7. The amount of normalized desmoglein-1 in Example 1 was calculated with the amount of normalized desmoglein-1 in Control 1 as 100%.

The results are shown in Fig. 1.

From the results of Fig. 1, it was shown that addition of Leu, Met, Phe, and Trp to amino acid-depleted cells remarkably increased the amount of desmoglein-1.

### [Experimental Example 2-2] Influence of specific amino acids (Trp, Val) on protein-glutamine γ-glutamyltransferase E production in epidermal keratinocytes

Among the amino acids shown to be correlated with protein-glutamine γ-glutamyltransferase E in the results of Experimental Example 1 (Table 6-9, Table 7-8), the following experiment was conducted to confirm the effect of the essential amino acids Trp and Val in promoting the production of protein-glutamine γ-glutamyltransferase E in epidermal keratinocytes.

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (3) or (4).
   Evaluation condition (3): Human skin-simulating medium 1 (hereinafter the sample evaluated under evaluation condition (3) is referred to as "Control 2").
   Evaluation condition (4): Medium obtained by adding Trp (final concentration 0.08 mM) and Val (final concentration 0.8 mM) to human skin-simulating medium 1 (hereinafter the sample evaluated under evaluation condition (4) is referred to as "Example 2").
   Human skin-simulating medium 1 was set to a high calcium concentration (concentration 2 mM in the medium) in order to produce experimental conditions under which differentiation was induced at the same time as the evaluation began, because protein-glutamine γ-glutamyltransferase E is a protein that is expressed in association with cell differentiation induced by high calcium concentrations.
4. After culturing the sample of the above-mentioned 3 for 6 days, a protein extract (extract containing cytoplasm protein) was prepared using Mem-PER Plus (Thermo Fisher Scientific 89842) and Halt Protease Inhibitor Cocktail (Thermo Fisher Scientific 87785), and protein-glutamine γ-glutamyltransferase E was quantified using a commercially available ELISA kit (Cloud-Clone Corp SEB756Hu).
5. The protein extract of the above-mentioned 4 was diluted with water, and the protein was quantified using Micro BCA Protein Assay Kit (Thermo Fisher Scientific 23235).
6. The amount of protein-glutamine γ-glutamyltransferase E determined in the above-mentioned 4 was normalized by the amount of protein determined in the above-mentioned 5.
7. The amount of normalized protein-glutamine γ-glutamyltransferase E in Example 2 was calculated with the amount of normalized protein-glutamine γ-glutamyltransferase E in Control 2 as 100%.

The results are shown in Fig. 2.

From the results of Fig. 2, it was shown that addition of Trp and Val to amino acid-depleted cells remarkably increased the amount of protein-glutamine γ-glutamyltransferase E.

### [Experimental Example 2-3] Influence of specific amino acids (His, Leu, Val) on caspase-14 in epidermal keratinocytes

Among the amino acids shown to be correlated with caspase-14 in the results of Experimental Example 1 (Table 8-1), the following experiment was conducted to confirm the effect of the essential amino acids His, Leu, and Val in promoting the production of caspase-14 in epidermal keratinocytes.

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (5) - (7).
   Evaluation condition (5): Human skin-simulating medium 2 (hereinafter the sample evaluated under evaluation condition (5) is referred to as "Control 3").
   Evaluation condition (6): Medium obtained by adding His (final concentration 0.2 mM), Leu (final concentration 0.8 mM), Val (final concentration 0.8 mM) to human skin-simulating medium 2 (hereinafter the sample evaluated under evaluation condition (6) is referred to as "Example 3").
   Evaluation condition (7): Medium obtained by adding His (final concentration 0.2 mM) to human skin-simulating medium 2 (hereinafter the sample evaluated under evaluation condition (7) is referred to as "Example 4").
   Human skin-simulating medium 2 was set to a high calcium concentration (concentration 2 mM in the medium) in order to produce experimental conditions under which differentiation was induced at the same time as the evaluation began, because caspase-14 is a protein that is expressed in association with cell differentiation induced by high calcium concentrations.
4. After culturing the sample of the above-mentioned 3 for 4 days, a protein extract (extract containing membrane and membrane-associated protein) was prepared using Mem-PER Plus (Thermo Fisher Scientific 89842) and Halt Protease Inhibitor Cocktail (Thermo Fisher Scientific 87785), and caspase-14 was quantified using a commercially available ELISA kit (Cloud-Clone Corp SEA457Hu).
5. The protein extract of the above-mentioned 4 was diluted with water, and the protein was quantified using Micro BCA Protein Assay Kit (Thermo Fisher Scientific 23235).
6. The amount of caspase-14 determined in the above-mentioned 4 was normalized by the amount of protein determined in the above-mentioned 5.
7. The amount of normalized caspase-14 in Example 3 and Example 4 was calculated with the amount of normalized caspase-14 in Control 3 as 100%.

The results are shown in Fig. 3.

From the results of Fig. 3, it was shown that addition of His, Leu and Val or His to amino acid-depleted cells remarkably increased the amount of caspase-14.

### [Experimental Example 3] Influence of specific amino acid on the production of protein related to skin conditions of epidermal keratinocytes

### (Explanation relating to construction of experimental system)

Culture media (e.g., D-MEM medium) used for cell culture contain amino acids necessary for cells to survive and grow. On the other hand, in epidermal keratinocytes that make up human skin, it is possible that the necessary amount of amino acids does not reach the cells due to aging and external influences such as dryness. In the following Experimental Examples 3-1, 3-2, 3-3, and 3-4, media with the following compositions (human skin-simulating medium 1, human skin-simulating medium 2), in which the essential amino acids contained in D-MEM medium were reduced to 30% or 20%, were used as media simulating a situation in which the amount of amino acids in human skin was depleted.

Human skin-simulating medium 1: Essential amino acids in D-MEM (Life Technologies 21068-028) were changed to the concentrations shown in Table 11, human skin-simulating medium 1. Furthermore, calcium chloride (amount to achieve concentration 2 mM in the medium), sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), and antibiotics (amount to achieve penicillin concentration 50 units/mL in the medium and streptomycin concentration 50 µg/mL in the medium) were added.

human skin-simulating medium 2: The essential amino acids in D-MEM (Life Technologies 21068-028) were changed to the concentrations shown in Table 11, human skin-simulating medium 2. Furthermore, calcium chloride (amount to achieve concentration 2 mM in the medium), sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), and antibiotics (amounts to achieve concentration 50 units/mL of penicillin in the medium and 50 µg/mL of streptomycin in the medium).

**[Table 11]**

| essential amino acid | concentration (mM) | |
|---|---|---|
| | human skin-simulating medium 1 | human skin-simulating medium 2 |
| His | 0.06 | 0.04 |
| Ile | 0.24 | 0.16 |
| Leu | 0.24 | 0.16 |
| Lys | 0.24 | 0.16 |
| Met | 0.06 | 0.04 |
| Phe | 0.12 | 0.08 |
| Thr | 0.24 | 0.16 |
| Trp | 0.024 | 0.016 |
| Val | 0.24 | 0.16 |

### [Experimental Example 3-1] Influence of specific amino acids (Leu, Met, Phe, Trp) on desmoglein-1 production in epidermal keratinocytes

Among the amino acids shown to be correlated with desmoglein-1 in the results of Experimental Example 1 (Table 6-4, Table 7-1, Table 9-5), the following experiment (Experimental Examples 3-1-1 and 3-1-2) was conducted to confirm the effect of the essential amino acids Leu, Met, Phe, and Trp in promoting the production of desmoglein-1 in epidermal keratinocytes.

### (Experimental Example 3-1-1)

1. Epidermal keratinocytes were cultured in a medium obtained by adding sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), antibiotics (amount to achieve penicillin concentration 50 units/mL in the medium and streptomycin concentration 50 µg/mL in the medium), and calcium chloride (amount to achieve concentration 0.03 mM in the medium) to D-MEM (Life Technologies 21068-028) (hereinafter this medium is referred to as a low calcium medium).
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (8) - (10).
   Evaluation condition (8): Human skin-simulating medium 1 (hereinafter the sample evaluated under evaluation condition (8) is referred to as "Control 4").
   Evaluation condition (9): Medium obtained by adding Leu (final concentration 0.80 mM), Met (final concentration 0.20 mM), Phe (final concentration 0.40 mM), Trp (final concentration 0.08 mM) to human skin-simulating medium 1 (hereinafter the sample evaluated under evaluation condition (9) is referred to as "Example 5").
   Evaluation condition (10): Medium obtained by adding Phe (final concentration 0.40 mM) to human skin-simulating medium 1 (hereinafter the sample evaluated under evaluation condition (10) is referred to as "Example 6").
   Human skin-simulating medium 1 was set to a high calcium concentration (concentration 2 mM in the medium) in order to produce experimental conditions under which differentiation was induced at the same time as the evaluation began, because desmoglein-1 is a protein that is expressed in association with cell differentiation induced by high calcium concentrations.
4. After culturing the sample of the above-mentioned 3 for 5 days, a protein extract (extract containing membrane and membrane-associated proteins) was prepared using Mem-PER Plus (Thermo Fisher Scientific 89842) and Halt Protease Inhibitor Cocktail (Thermo Fisher Scientific 87785), and desmoglein-1 was quantified using a commercially available ELISA kit (Cloud-Clone Corp SEA729Hu).
5. The protein extract of the above-mentioned 4 was diluted with water, and the protein was quantified using Micro BCA Protein Assay Kit (Thermo Fisher Scientific 23235).
6. The amount of desmoglein-1 determined in the above-mentioned 4 was normalized by the amount of protein determined in the above-mentioned 5.
7. The amounts of normalized desmoglein-1 in Examples 5, 6 (Example 5: n=5, mean, Example 6: n=4, mean) were calculated with the amount of normalized desmoglein-1 (n=5, mean) in Control 4 as 100%.

The results of Experimental Example 3-1-1 are shown in Fig. 4-1.

From the results of Fig. 4-1, it was shown that addition of Phe alone, or Leu, Met, Phe, and Trp to amino acid-depleted cells remarkably increased the amount of desmoglein-1.

### (Experimental Example 3-1-2)

1. Epidermal keratinocytes were cultured in a medium obtained by adding sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), antibiotics (amount to achieve penicillin concentration 50 units/mL in the medium and streptomycin concentration 50 µg/mL in the medium), and calcium chloride (amount to achieve concentration 0.03 mM in the medium) to D-MEM (Life Technologies 21068-028) (hereinafter this medium is referred to as a low calcium medium).
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (11) - (14).
   Evaluation condition (11): Human skin-simulating medium 1 (hereinafter the sample evaluated under evaluation condition (11) is referred to as "Control 5").
   Evaluation condition (12): Medium obtained by adding Leu (final concentration 0.74 mM), Met (final concentration 0.29 mM), Phe (final concentration 0.38 mM), Trp (final concentration 0.076 mM) to human skin-simulating medium 1 (hereinafter the sample evaluated under evaluation condition (12) is referred to as "Example 7").
   Evaluation condition (13): Medium obtained by adding Leu (final concentration 0.50 mM), Met (final concentration 0.32 mM), Phe (final concentration 0.38 mM), Trp (final concentration 0.28 mM) to human skin-simulating medium 1 (hereinafter the sample evaluated under evaluation condition (13) is referred to as "Example 8").
   Evaluation condition (14): Medium obtained by adding Leu (final concentration 0.29 mM), Met (final concentration 0.32 mM), Phe (final concentration 0.35 mM), Trp (final concentration 0.52 mM) to human skin-simulating medium 1 (hereinafter the sample evaluated under evaluation condition (14) is referred to as "Example 9").
   Human skin-simulating medium 1 was set to a high calcium concentration (concentration 2 mM in the medium) in order to produce experimental conditions under which differentiation was induced at the same time as the evaluation began, because desmoglein-1 is a protein that is expressed in association with cell differentiation induced by high calcium concentrations.
4. After culturing the sample of the above-mentioned 3 for 6 days, a protein extract (extract containing membrane and membrane-associated proteins) was prepared using Mem-PER Plus (Thermo Fisher Scientific 89842) and Halt Protease Inhibitor Cocktail (Thermo Fisher Scientific 87785), and desmoglein-1 was quantified using a commercially available ELISA kit (Cloud-Clone Corp SEA729Hu).
5. The protein extract of the above-mentioned 4 was diluted with water, and the protein was quantified using Micro BCA Protein Assay Kit (Thermo Fisher Scientific 23235).
6. The amount of desmoglein-1 determined in the above-mentioned 4 was normalized by the amount of protein determined in the above-mentioned 5.
7. The amounts of normalized desmoglein-1 in Examples 7-9 (Example 7: n=3, mean, Example 8: n=3, mean, Example 9: n=3, mean) were calculated with the amount of normalized desmoglein-1 (n=3, mean) in Control 5 as 100%.

The results of Experimental Example 3-1-2 are shown in Fig. 4-2.

From the results of Fig. 4-2, it was shown that addition of Leu, Met, Phe, and Trp to amino acid-depleted cells remarkably increased the amount of desmoglein-1.

From the results of Experimental Examples 3-1-1 and 3-1-2 (Fig. 4-1, Fig. 4-2), it was found that the ratio of Phe, Trp, Met, and Leu to be added influences an increase in the amount of desmoglein-1.

In Examples 5, 7 - 9, the amount ((A) - (B)) obtained by subtracting the concentrations (indicated as (B)) of Phe, Trp, Met, and Leu in human skin-simulating medium 1 (Control 4 or 5) from the final concentrations (indicated as (A)) of Phe, Trp, Met, and Leu in each Example (i.e., the amounts of Phe, Trp, Met, and Leu added to human skin-simulating medium 1 (Control 4 or 5)) can be said to be the amounts of Phe, Trp, Met, and Leu that contributed to the increase in the amount of desmoglein-1.

In Examples 5, 7 - 9, the (A)-(B) and the molar ratios thereof (rounded to the nearest third decimal place) were calculated and are shown in Tables 12 to 15.

**[Table 12]**

| | concentration in Example 5 (mM) (A) | concentration in Control 4 (mM) (B) | (A) - (B) | molar ratio of Phe, Trp, Met, Leu | weight ratio of Phe, Trp, Met, Leu |
|---|---|---|---|---|---|
| Phe | 0.40 | 0.12 | 0.28 | 0.27 | 0.30 |
| Trp | 0.08 | 0.024 | 0.056 | 0.05 | 0.07 |
| Met | 0.20 | 0.06 | 0.14 | 0.14 | 0.14 |
| Leu | 0.80 | 0.24 | 0.56 | 0.54 | 0.48 |

**[Table 13]**

| | concentration in Example 7 (mM) (A) | concentration in Control 5 (mM) (B) | (A) - (B) | molar ratio of Phe, Trp, Met, Leu | weight ratio of Phe, Trp, Met, Leu |
|---|---|---|---|---|---|
| Phe | 0.38 | 0.12 | 0.26 | 0.25 | 0.28 |
| Trp | 0.076 | 0.024 | 0.052 | 0.05 | 0.07 |
| Met | 0.29 | 0.06 | 0.23 | 0.22 | 0.22 |
| Leu | 0.74 | 0.24 | 0.50 | 0.48 | 0.43 |

**[Table 14]**

| | concentration in Example 8 (mM) (A) | concentration in Control 5 (mM) (B) | (A) - (B) | molar ratio of Phe, Trp, Met, Leu | weight ratio of Phe, Trp, Met, Leu |
|---|---|---|---|---|---|
| Phe | 0.38 | 0.12 | 0.26 | 0.25 | 0.25 |
| Trp | 0.28 | 0.024 | 0.256 | 0.25 | 0.31 |
| Met | 0.32 | 0.06 | 0.26 | 0.25 | 0.23 |
| Leu | 0.50 | 0.24 | 0.26 | 0.25 | 0.20 |

**[Table 15]**

| | concentration in Example 9 (mM) (A) | concentration in Control 5 (mM) (B) | (A) - (B) | molar ratio of Phe, Trp, Met, Leu | weight ratio of Phe, Trp, Met, Leu |
|---|---|---|---|---|---|
| Phe | 0.35 | 0.12 | 0.23 | 0.22 | 0.20 |
| Trp | 0.52 | 0.024 | 0.496 | 0.48 | 0.55 |
| Met | 0.32 | 0.06 | 0.26 | 0.25 | 0.21 |
| Leu | 0.29 | 0.24 | 0.05 | 0.05 | 0.04 |

### [Experimental Example 3-2] Influence of specific amino acids (Trp, Val) on protein-glutamine γ-glutamyltransferase E production in epidermal keratinocytes

Among the amino acids shown to be correlated with protein-glutamine γ-glutamyltransferase E in the results of Experimental Example 1 (Table 6-9, Table 7-8), the following experiment was conducted to confirm the effect of the essential amino acids Trp and Val in promoting the production of protein-glutamine γ-glutamyltransferase E in epidermal keratinocytes.

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (15) - (18).
   Evaluation condition (15): Human skin-simulating medium 1 (hereinafter the sample evaluated under evaluation condition (15) is referred to as "Control 6").
   Evaluation condition (16): Medium obtained by adding Trp (final concentration 0.160 mM), Val (final concentration 0.72 mM) to human skin-simulating medium 1 (hereinafter the sample evaluated under evaluation condition (16) is referred to as "Example 10").
   Evaluation condition (17): Medium obtained by adding Trp (final concentration 0.332 mM), Val (final concentration 0.55 mM) to human skin-simulating medium 1 (hereinafter the sample evaluated under evaluation condition (17) is referred to as "Example 11").
   Evaluation condition (18): Medium obtained by adding Trp (final concentration 0.504 mM), Val (final concentration 0.38 mM) to human skin-simulating medium 1 (hereinafter the sample evaluated under evaluation condition (18) is referred to as "Example 12").
   Human skin-simulating medium 1 was set to a high calcium concentration (concentration 2 mM in the medium) in order to produce experimental conditions under which differentiation was induced at the same time as the evaluation began, because protein-glutamine γ-glutamyltransferase E is a protein that is expressed in association with cell differentiation induced by high calcium concentrations.
4. After culturing the sample of the above-mentioned 3 for 6 days, a protein extract (extract containing cytoplasm protein) was prepared using Mem-PER Plus (Thermo Fisher Scientific 89842) and Halt Protease Inhibitor Cocktail (Thermo Fisher Scientific 87785), and protein-glutamine γ-glutamyltransferase E was quantified using a commercially available ELISA kit (Cloud-Clone Corp SEB756Hu).
5. The protein extract of the above-mentioned 4 was diluted with water, and the protein was quantified using Micro BCA Protein Assay Kit (Thermo Fisher Scientific 23235).
6. The amount of protein-glutamine γ-glutamyltransferase determined in the above-mentioned 4 was normalized by the amount of protein determined in the above-mentioned 5.
7. The amounts of normalized protein-glutamine γ-glutamyltransferase E in Examples 10 - 12 (Example 10: n=3, mean, Example 11: n=3, mean, Example 12: n=3, mean) were calculated with the amount of normalized protein-glutamine γ-glutamyltransferase E (n=3, mean) in Control 6 as 100%.

The results are shown in Fig. 5.

From the results of Fig. 5, it was shown that addition of Trp and Val to amino acid-depleted cells remarkably increased the amount of protein-glutamine γ-glutamyltransferase E.

From the results of Experimental Example 3-2 (Fig. 5), it was found that the ratio of Val and Trp to be added influences an increase in the amount of protein-glutamine γ-glutamyltransferase E.

In Examples 10 - 12, the amount ((A) - (B)) obtained by subtracting the concentrations (indicated as (B)) of Val and Trp in human skin-simulating medium 1 (Control 6) from the final concentrations (indicated as (A)) of Val and Trp in each Example (i.e., the amounts of Val and Trp added to human skin-simulating medium 1 (Control 6)) can be said to be the amounts of Val and Trp that contributed to the increase in the amount of protein-glutamine γ-glutamyltransferase E.

In Examples 10 - 12, the (A)-(B) and the molar ratios thereof (rounded to the nearest third decimal place) were calculated and are shown in Tables 16 to 18.

**[Table 16]**

| | concentration in Example 10 (mM) (A) | concentration in Control 6 (mM) (B) | (A) - (B) | molar ratio of Val and Trp | weight ratio of Val and Trp |
|---|---|---|---|---|---|
| Val | 0.72 | 0.24 | 0.48 | 0.78 | 0.67 |
| Trp | 0.160 | 0.024 | 0.136 | 0.22 | 0.33 |

**[Table 17]**

| | concentration in Example 11 (mM) (A) | concentration in Control 6 (mM) (B) | (A) - (B) | molar ratio of Val and Trp | weight ratio of Val and Trp |
|---|---|---|---|---|---|
| Val | 0.55 | 0.24 | 0.31 | 0.50 | 0.36 |
| Trp | 0.332 | 0.024 | 0.308 | 0.50 | 0.64 |

**[Table 18]**

| | concentration in Example 12 (mM) (A) | concentration in Control 6 (mM) (B) | (A) - (B) | molar ratio of Val and Trp | weight ratio of Val and Trp |
|---|---|---|---|---|---|
| Val | 0.38 | 0.24 | 0.14 | 0.23 | 0.15 |
| Trp | 0.504 | 0.024 | 0.48 | 0.77 | 0.85 |

### [Experimental Example 3-3] Influence of specific amino acids (His, Leu, Val) on caspase-14 in epidermal keratinocytes

Among the amino acids shown to be correlated with caspase-14 in the results of Experimental Example 1 (Table 8-1), the following experiments (Experimental Examples 3-3-1, 3-3-2, and 3-3-3) were conducted to confirm the effect of the essential amino acids His, Leu, and Val in promoting the production of caspase-14 in epidermal keratinocytes.

### (Experimental Example 3-3-1)

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (19) - (22).
   Evaluation condition (19): Human skin-simulating medium 2 (hereinafter the sample evaluated under evaluation condition (19) is referred to as "Control 7").
   Evaluation condition (20): Medium obtained by adding His (final concentration 0.2 mM), Leu (final concentration 0.8 mM), Val (final concentration 0.8 mM) to human skin-simulating medium 2 (hereinafter the sample evaluated under evaluation condition (20) is referred to as "Example 13").
   Evaluation condition (21): Medium obtained by adding Val (final concentration 0.8 mM) to human skin-simulating medium 2 (hereinafter the sample evaluated under evaluation condition (21) is referred to as "Example 14").
   Evaluation condition (22): Medium obtained by adding His (final concentration 0.2 mM) to human skin-simulating medium 2 (hereinafter the sample evaluated under evaluation condition (22) is referred to as "Example 15").
   Human skin-simulating medium 2 was set to a high calcium concentration (concentration 2 mM in the medium) in order to produce experimental conditions under which differentiation was induced at the same time as the evaluation began, because caspase-14 is a protein that is expressed in association with cell differentiation induced by high calcium concentrations.
4. After culturing the sample of the above-mentioned 3 for 4 days, a protein extract (extract containing membrane and membrane-associated protein) was prepared using Mem-PER Plus (Thermo Fisher Scientific 89842) and Halt Protease Inhibitor Cocktail (Thermo Fisher Scientific 87785), and caspase-14 was quantified using a commercially available ELISA kit (Cloud-Clone Corp SEA457Hu).
5. The protein extract of the above-mentioned 4 was diluted with water, and the protein was quantified using Micro BCA Protein Assay Kit (Thermo Fisher Scientific 23235).
6. The amount of caspase-14 determined in the above-mentioned 4 was normalized by the amount of protein determined in the above-mentioned 5.
7. The amounts of normalized caspase-14 in Examples 13 - 15 (Example 13: n=6, mean, Example 14: n=4, mean, Example 15: n=4, mean) were calculated with the amount of normalized caspase-14 (n=6, mean) in Control 7 as 100%.

The results of Experimental Example 3-3-1 are shown in Fig. 6-1.

From the results of Fig. 6-1, it was shown that addition of Val alone, His alone, or His, Leu, and Val in combination to amino acid-depleted cells remarkably increased the amount of caspase-14.

### (Experimental Example 3-3-2)

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (23) - (26).
   Evaluation condition (23): Human skin-simulating medium 2 (hereinafter the sample evaluated under evaluation condition (23) is referred to as "Control 8").
   Evaluation condition (24): Medium obtained by adding His (final concentration 0.21 mM), Leu (final concentration 0.92 mM), Val (final concentration 0.66 mM) to human skin-simulating medium 2 (hereinafter the sample evaluated under evaluation condition (24) is referred to as "Example 16").
   Evaluation condition (25): Medium obtained by adding His (final concentration 0.52 mM), Leu (final concentration 0.64 mM), Val (final concentration 0.64 mM) to human skin-simulating medium 2 (hereinafter the sample evaluated under evaluation condition (25) is referred to as "Example 17").
   Evaluation condition (26): Medium obtained by adding His (final concentration 0.80 mM), Leu (final concentration 0.33 mM), Val (final concentration 0.66 mM) to human skin-simulating medium 2 (hereinafter the sample evaluated under evaluation condition (26) is referred to as "Example 18").
   Human skin-simulating medium 2 was set to a high calcium concentration (concentration 2 mM in the medium) in order to produce experimental conditions under which differentiation was induced at the same time as the evaluation began, because caspase-14 is a protein that is expressed in association with cell differentiation induced by high calcium concentrations.
4. After culturing the sample of the above-mentioned 3 for 5 days, a protein extract (extract containing membrane and membrane-associated protein) was prepared using Mem-PER Plus (Thermo Fisher Scientific 89842) and Halt Protease Inhibitor Cocktail (Thermo Fisher Scientific 87785), and caspase-14 was quantified using a commercially available ELISA kit (Cloud-Clone Corp SEA457Hu).
5. The protein extract of the above-mentioned 4 was diluted with water, and the protein was quantified using Micro BCA Protein Assay Kit (Thermo Fisher Scientific 23235).
6. The amount of caspase-14 determined in the above-mentioned 4 was normalized by the amount of protein determined in the above-mentioned 5.
7. The amounts of normalized caspase-14 in Examples 16 - 18 (Example 16: n=3, mean, Example 17: n=3, mean, Example 18: n=3, mean) were calculated with the amount of normalized caspase-14 (n=3, mean) in Control 8 as 100%.

The results of Experimental Example 3-3-2 are shown in Fig. 6-2.

From the results of Fig. 6-2, it was shown that addition of His, Leu and Val to amino acid-depleted cells remarkably increased the amount of caspase-14.

### (Experimental Example 3-3-3)

1. Epidermal keratinocytes were cultured in a low calcium medium.
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (27) - (29).
   Evaluation condition (27): Human skin-simulating medium 2 (hereinafter the sample evaluated under evaluation condition (27) is referred to as "Control 9").
   Evaluation condition (28): Medium obtained by adding His (final concentration 0.79 mM), Leu (final concentration 0.23 mM), Val (final concentration 0.78 mM) to human skin-simulating medium 2 (hereinafter the sample evaluated under evaluation condition (28) is referred to as "Example 19").
   Evaluation condition (29): Medium obtained by adding His (final concentration 0.11 mM), Leu (final concentration 0.91 mM), Val (final concentration 0.78 mM) to human skin-simulating medium 2 (hereinafter the sample evaluated under evaluation condition (29) is referred to as "Example 20").
   Human skin-simulating medium 2 was set to a high calcium concentration (concentration 2 mM in the medium) in order to produce experimental conditions under which differentiation was induced at the same time as the evaluation began, because caspase-14 is a protein that is expressed in association with cell differentiation induced by high calcium concentrations.
4. After culturing the sample of the above-mentioned 3 for 6 days, a protein extract (extract containing membrane and membrane-associated protein) was prepared using Mem-PER Plus (Thermo Fisher Scientific 89842) and Halt Protease Inhibitor Cocktail (Thermo Fisher Scientific 87785), and caspase-14 was quantified using a commercially available ELISA kit (Cloud-Clone Corp SEA457Hu).
5. The protein extract of the above-mentioned 4 was diluted with water, and the protein was quantified using Micro BCA Protein Assay Kit (Thermo Fisher Scientific 23235).
6. The amount of caspase-14 determined in the above-mentioned 4 was normalized by the amount of protein determined in the above-mentioned 5.
7. The amounts of normalized caspase-14 in Examples 19 and 20 (Example 19: n=3, mean, Example 20: n=3, mean) were calculated with the amount of normalized caspase-14 (n=3, mean) in Control 9 as 100%.

The results of Experimental Example 3-3-3 are shown in Fig. 6-3.

From the results of Fig. 6-3, it was shown that addition of His, Leu, and Val in combination to amino acid-depleted cells remarkably increased the amount of caspase-14.

From the results of Experimental Examples 3-3-1, 3-3-2, and 3-3-3 (Fig. 6-1, Fig. 6-2, Fig. 6-3), it was found that the ratio of Val, His, and Leu to be added influences an increase in the amount of caspase-14.

In Examples 13, 16 - 20, the amount ((A) - (B)) obtained by subtracting the concentrations (indicated as (B)) of Val, His, and Leu in human skin-simulating medium 1 (Controls 7 - 9) from the final concentrations (indicated as (A)) of Val, His, and Leu in each Example (i.e., the amounts of Val and Trp added to human skin-simulating medium 2 (Controls 7 - 9)) can be said to be the amounts of Val, His, and Leu that contributed to the increase in the amount of caspase-14.

In Examples 13, 16 - 20, the (A)-(B) and the molar ratios thereof (rounded to the nearest third decimal place) were calculated and are shown in Tables 19 to 24.

**[Table 19]**

| | concentration in Example 13 (mM) (A) | concentration in Control 7 (mM) (B) | (A) - (B) | molar ratio of Val, His, Leu | weight ratio of Val, His, Leu |
|---|---|---|---|---|---|
| Val | 0.8 | 0.16 | 0.64 | 0.44 | 0.41 |
| His | 0.2 | 0.04 | 0.16 | 0.11 | 0.14 |
| Leu | 0.8 | 0.16 | 0.64 | 0.44 | 0.46 |

**[Table 20]**

| | concentration in Example 16 (mM) (A) | concentration in Control 8 (mM) (B) | (A) - (B) | molar ratio of Val, His, Leu | weight ratio of Val, His, Leu |
|---|---|---|---|---|---|
| Val | 0.66 | 0.16 | 0.50 | 0.35 | 0.32 |
| His | 0.21 | 0.04 | 0.17 | 0.12 | 0.14 |
| Leu | 0.92 | 0.16 | 0.76 | 0.53 | 0.54 |

**[Table 21]**

| | concentration in Example 17 (mM) (A) | concentration in Control 8 (mM) (B) | (A) - (B) | molar ratio of Val, His, Leu | weight ratio of Val, His, Leu |
|---|---|---|---|---|---|
| Val | 0.64 | 0.16 | 0.48 | 0.33 | 0.29 |
| His | 0.52 | 0.04 | 0.48 | 0.33 | 0.38 |
| Leu | 0.64 | 0.16 | 0.48 | 0.33 | 0.33 |

**[Table 22]**

| | concentration in Example 18 (mM) (A) | concentration in Control 8 (mM) (B) | (A) - (B) | molar ratio of Val, His, Leu | weight ratio of Val, His, Leu |
|---|---|---|---|---|---|
| Val | 0.66 | 0.16 | 0.50 | 0.35 | 0.30 |
| His | 0.80 | 0.04 | 0.76 | 0.53 | 0.59 |
| Leu | 0.33 | 0.16 | 0.17 | 0.12 | 0.11 |

**[Table 23]**

| | concentration in Example 19 (mM) (A) | concentration in Control 9 (mM) (B) | (A) - (B) | molar ratio of Val, His, Leu | weight ratio of Val, His, Leu |
|---|---|---|---|---|---|
| Val | 0.78 | 0.16 | 0.62 | 0.43 | 0.37 |
| His | 0.79 | 0.04 | 0.75 | 0.52 | 0.59 |
| Leu | 0.23 | 0.16 | 0.07 | 0.05 | 0.05 |

**[Table 24]**

| | concentration in Example 20 (mM) (A) | concentration in Control 9 (mM) (B) | (A) - (B) | molar ratio of Val, His, Leu | weight ratio of Val, His, Leu |
|---|---|---|---|---|---|
| Val | 0.78 | 0.16 | 0.62 | 0.43 | 0.40 |
| His | 0.11 | 0.04 | 0.07 | 0.05 | 0.06 |
| Leu | 0.91 | 0.16 | 0.75 | 0.52 | 0.54 |

### [Experimental Example 3-4] Influence of specific amino acids (Leu, Phe, Trp) on desmoglein-1 production in epidermal keratinocytes

Among the amino acids shown to be correlated with desmoglein-1 in the results of Experimental Example 1 (Table 6-4, Table 7-1, Table 9-5), the following experiment was conducted to confirm the effect of the essential amino acids Leu, Met, Phe, and Trp less Met, namely, Leu, Phe, and Trp, in promoting the production of desmoglein-1 in epidermal keratinocytes. Since Met often emits an odor in cosmetics and the choice of cosmetics is considered to be largely influenced by personal preferences, the development of cosmetics that do not contain Met is desired. For this reason, the effect of promoting desmoglein-1 production by blending amino acids that do not contain Met was confirmed.

1. Epidermal keratinocytes were cultured in a medium obtained by adding sodium pyruvate (amount to achieve concentration 1 mM in the medium), glutamine (amount to achieve concentration 4 mM in the medium), FBS (SIGMA-Aldrich F0392-500ML) (amount to achieve concentration 10% in the medium), antibiotics (amount to achieve penicillin concentration 50 units/mL in the medium and streptomycin concentration 50 µg/mL in the medium), and calcium chloride (amount to achieve concentration 0.03 mM in the medium) to D-MEM (Life Technologies 21068-028) (hereinafter this medium is referred to as a low calcium medium).
2. The epidermal keratinocytes grown in the low calcium medium in the above-mentioned 1 were seeded in a 10 cm petri dish at 12×10⁴ cells/well in a low calcium medium.
3. The next day, the medium was replaced with the medium of evaluation condition (30) - (33).
   Evaluation condition (30): Human skin-simulating medium 1 (hereinafter the sample evaluated under evaluation condition (30) is referred to as "Control 10").
   Evaluation condition (31): Medium obtained by adding Leu (final concentration 0.67 mM), Phe (final concentration 0.55 mM), Trp (final concentration 0.06 mM) to human skin-simulating medium 1 (hereinafter the sample evaluated under evaluation condition (31) is referred to as "Example 21").
   Evaluation condition (32): Medium obtained by adding Leu (final concentration 0.54 mM), Phe (final concentration 0.42 mM), Trp (final concentration 0.32 mM) to human skin-simulating medium 1 (hereinafter the sample evaluated under evaluation condition (32) is referred to as "Example 22").
   Evaluation condition (33): Medium obtained by adding Leu (final concentration 0.28 mM), Phe (final concentration 0.55 mM), Trp (final concentration 0.45 mM) to human skin-simulating medium 1 (hereinafter the sample evaluated under evaluation condition (33) is referred to as "Example 23").
   Human skin-simulating medium 1 was set to a high calcium concentration (concentration 2 mM in the medium) in order to produce experimental conditions under which differentiation was induced at the same time as the evaluation began, because desmoglein-1 is a protein that is expressed in association with cell differentiation induced by high calcium concentrations.
4. After culturing the sample of the above-mentioned 3 for 6 days, a protein extract (extract containing membrane and membrane-associated proteins) was prepared using Mem-PER Plus (Thermo Fisher Scientific 89842) and Halt Protease Inhibitor Cocktail (Thermo Fisher Scientific 87785), and desmoglein-1 was quantified using a commercially available ELISA kit (Cloud-Clone Corp SEA729Hu).
5. The protein extract of the above-mentioned 4 was diluted with water, and the protein was quantified using Micro BCA Protein Assay Kit (Thermo Fisher Scientific 23235).
6. The amount of desmoglein-1 determined in the above-mentioned 4 was normalized by the amount of protein determined in the above-mentioned 5.
7. The amounts of normalized desmoglein-1 in Examples 21 - 23 (Example 21: n=3, mean, Example 22: n=3, mean, Example 23: n=3, mean) were calculated with the amount of normalized desmoglein-1 (n=3, mean) in Control 10 as 100%.

The results of Experimental Example 3-4 are shown in Fig. 6-4.

From the results of Fig. 6-4, it was shown that addition of Leu, Phe, and Trp in combination to amino acid-depleted cells remarkably increased the amount of desmoglein-1.

From the results of Experimental Examples 3-4 (Fig. 6-4), it was found that the ratio of Phe, Trp, and Leu to be added influences an increase in the amount of desmoglein-1.

In Examples 21 - 23, the amount ((A) - (B)) obtained by subtracting the concentrations (indicated as (B)) of Phe, Trp, and Leu in human skin-simulating medium 1 (Control 10) from the final concentrations (indicated as (A)) of Phe, Trp, and Leu in each Example (i.e., the amounts of Phe, Trp, and Leu added to human skin-simulating medium 1 (Control 10)) can be said to be the amounts of Phe, Trp, and Leu that contributed to the increase in the amount of desmoglein-1.

In Examples 21 - 23, the (A)-(B) and the molar ratios thereof (rounded to the nearest third decimal place) were calculated and are shown in Tables 25 to 27.

**[Table 25]**

| | concentration in Example 21 (mM) (A) | concentration in Control 10 (mM) (B) | (A) - (B) | molar ratio of Phe, Trp, Leu | weight ratio of Phe, Trp, Leu |
|---|---|---|---|---|---|
| Phe | 0.55 | 0.12 | 0.43 | 0.48 | 0.53 |
| Trp | 0.06 | 0.024 | 0.036 | 0.04 | 0.05 |
| Leu | 0.67 | 0.24 | 0.43 | 0.48 | 0.42 |

**[Table 26]**

| | concentration in Example 22 (mM) (A) | concentration in Control 10 (mM) (B) | (A) - (B) | molar ratio of Phe, Trp, Leu | weight ratio of Phe, Trp, Leu |
|---|---|---|---|---|---|
| Phe | 0.42 | 0.12 | 0.30 | 0.33 | 0.33 |
| Trp | 0.32 | 0.024 | 0.296 | 0.33 | 0.41 |
| Leu | 0.54 | 0.24 | 0.30 | 0.33 | 0.26 |

**[Table 27]**

| | concentration in Example 23 (mM) (A) | concentration in Control 10 (mM) (B) | (A) - (B) | molar ratio of Phe, Trp, Leu | weight ratio of Phe, Trp, Leu |
|---|---|---|---|---|---|
| Phe | 0.55 | 0.12 | 0.43 | 0.48 | 0.43 |
| Trp | 0.45 | 0.024 | 0.426 | 0.48 | 0.54 |
| Leu | 0.28 | 0.24 | 0.04 | 0.04 | 0.03 |

### [Industrial Applicability]

According to the present invention, a composition for improving skin conditions can be provided, which can improve skin functions related to the improvement of skin conditions (decreased moisture content of the stratum corneum, spots, decreased barrier function, pH abnormality), by promoting the production of proteins in the skin that are related to the improvement of skin conditions.

This application is based on patent application No. 2022-209098 filed in Japan, the contents of which are encompassed in full herein.

## Claims

1. A composition for improving skin conditions, comprising at least one member selected from the group consisting of a desmoglein-1 production promoter, a caspase 14 production promoter, and a protein-glutamine γ-glutamyltransferase E production promoter as an active ingredient.

2. The composition according to claim 1, comprising a desmoglein-1 production promoter which is one or more members selected from the group consisting of Phe, Trp, Met, and Leu.

3. The composition according to claim 2, wherein the desmoglein-1 production promoter comprises Phe, Trp, Met, and Leu.

4. The composition according to claim 3, wherein a weight ratio of Phe, Trp, Met, and Leu (Phe:Trp:Met:Leu) is 1:0.2 - 2.8:0.4 - 1.1:0.1 - 1.6.

5. The composition according to claim 2, wherein the desmoglein-1 production promoter comprises Phe, Trp, and Leu.

6. The composition according to claim 5, wherein a weight ratio of Phe, Trp, and Leu (Phe:Trp:Leu) is 1:0.05 - 1.3:0.05 - 0.8.

7. The composition according to any one of claims 2 to 6, which is for the improvement of decreased moisture contents of the stratum corneum, improvement of skin spots, or improvement of abnormal pH value of the skin surface.

8. The composition according to claim 1, comprising a caspase-14 production promoter which is one or more members selected from the group consisting of Val, His, and Leu.

9. The composition according to claim 8, wherein the caspase-14 production promoter comprises Val, His, and Leu.

10. The composition according to claim 9, wherein a weight ratio of Val, His, and Leu (Val:His:Leu) is 1:0.1 - 2.1:0.1 - 1.8.

11. The composition according to any one of claims 8 to 10, which is for the improvement of decreased barrier function of the skin.

12. The composition according to claim 1, comprising a protein-glutamine γ-glutamyltransferase E production promoter which is one or more members selected from the group consisting of Val and Trp.

13. The composition according to claim 12, wherein the protein-glutamine γ-glutamyltransferase E production promoter comprises Val and Trp.

14. The composition according to claim 13, wherein a weight ratio of Val and Trp (Val:Trp) is 1:0.1 - 5.9.

15. The composition according to any one of claims 12 to 14, which is for the improvement of decreased moisture contents of the stratum corneum, or improvement of skin spots.

16. A composition for promoting production of desmoglein-1, comprising one or more members selected from the group consisting of Phe, Trp, Met, and Leu as an active ingredient.

17. The composition according to claim 16, comprising Phe, Trp, Met, and Leu as an active ingredient.

18. The composition according to claim 17, wherein a weight ratio of Phe, Trp, Met, and Leu (Phe:Trp:Met:Leu) is 1:0.2 - 2.8:0.4 - 1.1:0.1 - 1.6.

19. The composition according to claim 16, comprising Phe, Trp, and Leu as an active ingredient.

20. The composition according to claim 19, wherein a weight ratio of Phe, Trp, and Leu (Phe:Trp:Leu) is 1:0.05 - 1.3:0.05 - 0.8.

21. A composition for promoting production of caspase-14, comprising one or more members selected from the group consisting of Val, His, and Leu as an active ingredient.

22. The composition according to claim 21, comprising Val, His, and Leu as an active ingredient.

23. The composition according to claim 22, wherein a weight ratio of Val, His, and Leu (Val:His:Leu) is 1:0.1 - 2.1:0.1 - 1.8.

24. A composition for promoting production of protein-glutamine γ-glutamyltransferase E, comprising one or more members selected from the group consisting of Val and Trp as an active ingredient.

25. The composition according to claim 24, comprising Val and Trp as an active ingredient.

26. The composition according to claim 25, wherein a weight ratio of Val and Trp (Val:Trp) is 1:0.1 - 5.9.

27. A method for improving skin conditions comprising administering at least one member selected from the group consisting of a desmoglein-1 production promoter, a caspase 14 production promoter, and a protein-glutamine γ-glutamyltransferase E production promoter to a subject in need thereof.

28. The method according to claim 27, comprising administering a desmoglein-1 production promoter which is one or more members selected from the group consisting of Phe, Trp, Met, and Leu.

29. The method according to claim 28, wherein the desmoglein-1 production promoter comprises Phe, Trp, Met, and Leu.

30. The method according to claim 29, wherein a weight ratio of Phe, Trp, Met, and Leu (Phe:Trp:Met:Leu) is 1:0.2 - 2.8:0.4 - 1.1:0.1 - 1.6.

31. The method according to claim 28, wherein the desmoglein-1 production promoter comprises Phe, Trp, and Leu.

32. The method according to claim 31, wherein a weight ratio of Phe, Trp, and Leu (Phe:Trp:Leu) is 1:0.05 - 1.3:0.05 - 0.8.

33. The method of any one of claims 28 to 32, wherein the improvement of skin conditions is improvement of decreased moisture contents of the stratum corneum, improvement of skin spots, or improvement of abnormal pH value of the skin surface.

34. The method according to claim 27, comprising administering a caspase-14 production promoter which is one or more members selected from the group consisting of Val, His, and Leu.

35. The method according to claim 34, wherein the caspase-14 production promoter comprises Val, His, and Leu.

36. The method according to claim 35, wherein a weight ratio of Val, His, and Leu (Val:His:Leu) is 1:0.1 - 2.1:0.1 - 1.8.

37. The method according to any one of claims 34 to 36, wherein the improvement of skin conditions is improvement of decreased barrier function of the skin.

38. The method according to claim 27, comprising administering a protein-glutamine γ-glutamyltransferase E production promoter which is one or more members selected from the group consisting of Val and Trp.

39. The method according to claim 38, wherein the protein-glutamine γ-glutamyltransferase E production promoter comprises Val and Trp.

40. The method according to claim 39, wherein a weight ratio of Val and Trp (Val:Trp) is 1:0.1 - 5.9.

41. The method according to any one of claims 38 to 40, wherein the improvement of skin conditions is improvement of decreased moisture contents of the stratum corneum, or improvement of skin spots.

42. A method for promoting production of desmoglein-1 in a subject in need thereof, comprising administering an effective amount of one or more members selected from the group consisting of Phe, Trp, Met, and Leu to the subject.

43. The method according to claim 42, comprising administering Phe, Trp, Met, and Leu.

44. The method according to claim 43, wherein a weight ratio of Phe, Trp, Met, and Leu (Phe:Trp:Met:Leu) is 1:0.2 - 2.8:0.4 - 1.1:0.1 - 1.6.

45. The method according to claim 42, comprising administering Phe, Trp, and Leu.

46. The method according to claim 45, wherein a weight ratio of Phe, Trp, and Leu (Phe:Trp:Leu) is 1:0.05 - 1.3:0.05 - 0.8.

47. A method for promoting production of caspase-14 in a subject in need thereof, comprising administering an effective amount of one or more members selected from the group consisting of Val, His, and Leu to the subject.

48. The method according to claim 47, comprising administering Val, His, and Leu.

49. The method according to claim 48, wherein a weight ratio of Val, His, and Leu (Val:His:Leu) is 1:0.1 - 2.1:0.1 - 1.8.

50. A method for promoting production of protein-glutamine γ-glutamyltransferase E in a subject in need thereof, comprising administering an effective amount of one or more members selected from the group consisting of Val and Trp to the subject.

51. The method according to claim 50, comprising administering Val and Trp.

52. The method according to claim 51, wherein a weight ratio of Val and Trp (Val:Trp) is 1:0.1 - 5.9.

53. A desmoglein-1 production promoter, a caspase 14 production promoter, or a protein-glutamine γ-glutamyltransferase E production promoter, for use in improving skin conditions.

54. The agent according to claim 53, wherein the desmoglein-1 production promoter is one or more members selected from the group consisting of Phe, Trp, Met, and Leu.

55. The agent according to claim 54, wherein the desmoglein-1 production promoter comprises Phe, Trp, Met, and Leu.

56. The agent according to claim 55, wherein a weight ratio of Phe, Trp, Met, and Leu (Phe:Trp:Met:Leu) is 1:0.2 - 2.8:0.4 - 1.1:0.1 - 1.6.

57. The agent according to claim 54, wherein the desmoglein-1 production promoter comprises Phe, Trp, and Leu.

58. The agent according to claim 57, wherein a weight ratio of Phe, Trp, and Leu (Phe:Trp:Leu) is 1:0.05 - 1.3:0.05 - 0.8.

59. The agent according to any one of claims 54 to 58, for use in the improvement of decreased moisture contents of the stratum corneum, improvement of skin spots, or improvement of abnormal pH value of the skin surface.

60. The agent according to claim 53, wherein the caspase-14 production promoter is one or more members selected from the group consisting of Val, His, and Leu.

61. The agent according to claim 60, wherein the caspase-14 production promoter comprises Val, His, and Leu.

62. The agent according to claim 61, wherein a weight ratio of Val, His, and Leu (Val:His:Leu) is 1:0.1 - 2.1:0.1 - 1.8.

63. The agent according to any one of claims 60 to 62, for use in the improvement of decreased barrier function of the skin.

64. The agent according to claim 53, wherein the protein-glutamine γ-glutamyltransferase E production promoter is one or more members selected from the group consisting of Val and Trp.

65. The agent according to claim 64, wherein the protein-glutamine γ-glutamyltransferase E production promoter comprises Val and Trp.

66. The agent according to claim 65, wherein a weight ratio of Val and Trp (Val:Trp) is 1:0.1 - 5.9.

67. The agent according to any one of claims 64 to 66, for use in the improvement of decreased moisture contents of the stratum corneum, or improvement of skin spots.

68. One or more amino acids selected from the group consisting of Phe, Trp, Met, and Leu for use in promoting production of desmoglein-1.

69. The amino acids according to claim 68, which are Phe, Trp, Met, and Leu.

70. The amino acids according to claim 69, wherein a weight ratio of Phe, Trp, Met, and Leu (Phe:Trp:Met:Leu) is 1:0.2 - 2.8:0.4 - 1.1:0.1 - 1.6.

71. The amino acids according to claim 68, which are Phe, Trp, and Leu.

72. The amino acids according to claim 71, wherein a weight ratio of Phe, Trp, and Leu (Phe:Trp:Leu) is 1:0.05 - 1.3:0.05 - 0.8.

73. One or more amino acids selected from the group consisting of Val, His, and Leu, for use in promoting production of caspase-14.

74. The amino acids according to claim 73, which are Val, His, and Leu.

75. The amino acids according to claim 74, wherein a weight ratio of Val, His, and Leu (Val:His:Leu) is 1:0.1 - 2.1:0.1 - 1.8.

76. One or more amino acids selected from the group consisting of Val and Trp, for use in promoting production of protein-glutamine γ-glutamyltransferase E.

77. The amino acids according to claim 76, which are Val and Trp.

78. The amino acids according to claim 77, wherein a weight ratio of Val and Trp (Val:Trp) is 1:0.1 - 5.9.

79. Use of a desmoglein-1 production promoter, a caspase 14 production promoter, or a protein-glutamine γ-glutamyltransferase E production promoter, for producing a composition for improving skin conditions.

80. The use according to claim 79, wherein the desmoglein-1 production promoter is one or more members selected from the group consisting of Phe, Trp, Met, and Leu.

81. The use according to claim 80, wherein the desmoglein-1 production promoter comprises Phe, Trp, Met, and Leu.

82. The use according to claim 81, wherein a weight ratio of Phe, Trp, Met, and Leu (Phe:Trp:Met:Leu) is 1:0.2 - 2.8:0.4 - 1.1:0.1 - 1.6.

83. The use according to claim 80, wherein the desmoglein-1 production promoter comprises Phe, Trp, and Leu.

84. The use according to claim 83, wherein a weight ratio of Phe, Trp, and Leu (Phe:Trp:Leu) is 1:0.05 - 1.3:0.05 - 0.8.

85. The use according to any one of claims 80 to 84, wherein the improvement of skin conditions is improvement of decreased moisture contents of the stratum corneum, improvement of skin spots, or improvement of abnormal pH value of the skin surface.

86. The use according to claim 79, wherein the caspase-14 production promoter is one or more members selected from the group consisting of Val, His, and Leu.

87. The use according to claim 86, wherein the caspase-14 production promoter comprises Val, His, and Leu.

88. The use according to claim 87, wherein a weight ratio of Val, His, and Leu (Val:His:Leu) is 1:0.1 - 2.1:0.1 - 1.8.

89. The use according to any one of claims 86 to 88, wherein the improvement of skin conditions is improvement of decreased barrier function of the skin.

90. The use according to claim 79, wherein the protein-glutamine γ-glutamyltransferase E production promoter is one or more members selected from the group consisting of Val and Trp.

91. The use according to claim 90, wherein protein-glutamine γ-glutamyltransferase E production promoter comprises Val and Trp.

92. The use according to claim 91, wherein a weight ratio of Val and Trp (Val:Trp) is 1:0.1 - 5.9.

93. The use according to any one of claims 90 to 92, wherein the improvement of skin conditions is improvement of decreased moisture contents of the stratum corneum, or improvement of skin spots.

94. Use of one or more amino acids selected from the group consisting of Phe, Trp, Met, and Leu in producing a composition for promoting production of desmoglein-1.

95. The use according to claim 94, wherein the amino acids are Phe, Trp, Met, and Leu.

96. The use according to claim 95, wherein a weight ratio of Phe, Trp, Met, and Leu (Phe:Trp:Met:Leu) is 1:0.2 - 2.8:0.4 - 1.1:0.1 - 1.6.

97. The use according to claim 94, wherein the amino acids are Phe, Trp, and Leu.

98. The use according to claim 97, wherein a weight ratio of Phe, Trp, and Leu (Phe:Trp:Leu) is 1:0.05 - 1.3:0.05 - 0.8.

99. Use of one or more amino acids selected from the group consisting of Val, His, and Leu in producing a composition for promoting production of caspase-14.

100. The use according to claim 99, wherein the amino acids are Val, His, and Leu.

101. The use according to claim 100, wherein a weight ratio of Val, His, and Leu (Val:His:Leu) is 1:0.1 - 2.1:0.1 - 1.8.

102. Use of one or more amino acids selected from the group consisting of Val and Trp in producing a composition for promoting production of protein-glutamine γ-glutamyltransferase E.

103. The use according to claim 102, wherein the amino acids are Val and Trp.

104. The use according to claim 103, wherein a weight ratio of Val and Trp (Val:Trp) is 1:0.1 - 5.9.
